(19) Europäisches Patentamt
European Patent Office
Office européen des brevets

(11) EP 1 682 129 B1

(12) **EUROPEAN PATENT SPECIFICATION**

(45) Date of publication and mention of the grant of the patent:
**21.02.2007 Bulletin 2007/08**

(21) Application number: **04762951.4**

(22) Date of filing: **25.10.2004**

(51) Int Cl.:
***A61K 31/426*** *(2006.01)* ***C07D 277/46*** *(2006.01)*

(86) International application number:
**PCT/DK2004/000733**

(87) International publication number:
**WO 2005/039572 (06.05.2005 Gazette 2005/18)**

# (54) N-THIAZOL-2-YL-BENZAMIDE DERIVATIVES

N-THIAZOL-2-YL-BENZAMID-DERIVATE

DERIVES DE I N /I -THIAZOLE-2-YLE-BENZAMIDE

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **27.10.2003 DK 200301579**
**13.02.2004 DK 200400229**

(43) Date of publication of application:
**26.07.2006 Bulletin 2006/30**

(73) Proprietor: **H. LUNDBECK A/S**
**2500 Copenhagen (DK)**

(72) Inventors:
- **SAMS, Anette, Graven**
**DK- 3500 Vaerlose (DK)**
- **LARSEN, Mogens**
**DK-2765 Smörum (DK)**
- **MIKKELSEN, Gitte**
**DK-2750 Ballerup (DK)**

(74) Representative: **Kjerrumgaard, Lars Bo et al**
**H. Lundbeck A/S,**
**Corporate Patents,**
**9, Ottiliavej**
**2500 Valby-Copenhagen (DK)**

(56) References cited:
**EP-A- 0 934 938** **EP-A- 1 354 880**
**WO-A-00/26202** **WO-A-03/045386**
**WO-A-03/053946** **DE-C- 855 120**

- **JACQUELINE E. VAN MUIJLWIJK-KOEZEN ET AL.: "Thiazole and Thiadiazole Analogues as a Novel class of Adenosine Receptor Antagonists" JOURNAL OF MEDICINAL CHEMISTRY., vol. 44, no. 5, 1 March 2001 (2001-03-01), pages 749-762, XP002318825 USAMERICAN CHEMICAL SOCIETY.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 682 129 B1

Printed by Jouve, 75001 PARIS (FR)

## Description

### Field of the Invention

The compounds of the present invention belong to a novel class of *N*-thiazol-2-yl-benzamide derivatives having affinity for the adenosine 2A ($A_{2A}$) receptor. The compounds are $A_{2A}$-receptor ligands, such as antagonists, agonists, reverse agonists or partial agonists, and are useful in the treatment of neurological and psychiatric disorders where an $A_{2A}$-receptor is implicated. Examples of diseases where an $A_{2A}$-receptor is implicated are Parkinson's Disease (PD), Alzheimer's Disease, Huntington's disease, epilepsia, cerebral ischemia, haemorrhagic stroke, neonatal ischemia and hypoxia, subarachnoid haemorrhage, traumatic brain injury, brain damage following cardiac arrest, and for the treatment of depression and psychosis disorders.

### Background of the invention

Adenosine is present in all cells, including neurons and glia, of mammalian organisms where it modulates a variety of important physiological processes. The action of adenosine is mediated by specific receptors, which belong to the family of G protein-coupled receptors. Four adenosine receptors have been cloned and characterized, $A_1$, $A_{2A}$, $A_{2B}$ and $A_3$ (Fredholm et al, **1994,** *Pharmac. Rev.*, 46, 143-156). The main intracellular signaling pathways involve the formation of cAMP, with $A_1$ and $A_3$ receptors causing inhibition of adenylate cyclase and $A_{2A}$ and $A_{2B}$ receptors activating it (Olah et al, *Pharacol. Ther.*, **2000,** 85, 55-75).

All of the adenosine receptors have been located in the CNS (Impagnatiello et al, **2000,** *Emerg. Ther. Targets*, 4, 635-644; Rosin et al, **1998,** *J. Comp. Neurol.*, 401, 163-186). The receptor of interest here, $A_{2A}$, is predominantly found in dopamine-rich areas, such as the basal ganglia components; the striatum and the globus pallidus, in various mammalians, including humans. The basal ganglia, with the striatum as a central component, are involved in integration of cortical, thalamic and limbic information to produce motor behaviours (for review see Svenningson et al, *Prog. Neurobiol.*, **1999,** 59, 355-396).

In the striatum $A_{2A}$ and dopamine $D_2$ receptors are found closely co-localized on the striatopallidal GABAergic neurons, forming the so-called indirect output pathway from the striatum, which is involved in motor inhibition. $A_{2A}$ receptors contribute to control of motor behaviour by modulating the neurotransmission of GABA, dopamine, acetylcholine and glutamate in various ways. Currently, the interactions between $A_{2A}$ and $D_2$ receptors, and especially the actions of $A_{2A}$ antagonists, is of great interest in the treatment for Parkinson's disease (PD), which involves a decrease in dopamine levels. The $A_{2A}$ receptors interact tonically and antagonistically with the $D_2$ receptors, causing a decrease in affinity of the $D_2$ receptors for dopamin upon stimulation. Thus, $A_{2A}$ antagonists may be capable of enhancing the effect of endogenous dopamine as well as clinically used dopamine agonists and increase the time-period of dopaminergic drug response. (For details and Refs therein see e.g: Richardson et al, **1997,** *Trends Pharmacol. Sci.,* 18, 338-344; Svenningson et al, *Prog. Neurobiol.*, **1999,** 59, 355-396; Fuxe et al, **2001,** *Parkinson's Dis. Adv.*, 86,.345-353).

Selective $A_{2A}$ receptor agonists and antagonists have been widely described in pharmacological, behavioural and neuroprotective experiments in rodents and non-human primates (for reviews see: Richardson et al, **1997,** *Trends Pharmacol. Sci.,* 18, 338-344; Ribeiro et al, **2003,** *Prog. Neurobio*l., 68, 377-392; Ongini et al, **2001,** *Il Farmaco*, 56, 87-90; Wardas, **2003,** *Polish J. Pharmacology*, 54, 313-326).

The close interaction of $D_2$ and $A_{2A}$ receptors can be clearly exemplified in models of catalepsy, where $D_2$ receptor antagonists as well as $A_{2A}$ receptor agonists induce catalepsy, which is counteracted by $A_{2A}$ receptor antagonists and $D_2$ receptor agonists, respectively (see Svenningson et al, *Prog. Neurobiol.*, **1999**, 59, 355-396 and Refs therein).

Promising anti-parkinsonian effects of $A_{2A}$ receptor antagonists have currently been reported by many investigators. For example, both SCH58261 (2-(2-furanyl)-7-(2-phenylethyl)-7*H*-pyrazolo[4,3-*e*][1,2,4]triazolo[1,5-*c*]pyrimidin-5-amine) and KW-6002 (8-[(1E)-2-(3,4-dimethoxyphenyl)ethenyl]-1,3-diethyl-3,7-dihydro-7-methyl-1*H*-purine-2,6-dione), enhance contralateral rotations, elicited by a subtreshold dose of levodopa, in unilateral 6-OHDA (6-hydroxydopamine) lesioned mice and rats (See Ongini et al, **2001,** *Drug Dev. Res.,* 52, 379-386 and refs therein). Furthermore, KW-6002 significantly improves motor impairment induced in non-human primates by MPTP (1-methyl-4-phenyl-1,2,3,6-tetrahydropyridine), without causing dyskinesias, that is commonly described for long-term treatment with the dopamine agonist L-dopa (Kanda et al, **1998,** *Ann. Neurol.*, 43 (4), 507- 513; Grondin et al, **1999,** Neurology, 52 (1), 1673-1677; Kanda et al, **2000,** *Exp. Neurol,* 162, 321-327).

Thus, $A_{2A}$ receptor antagonists show great potential as future drugs for long-term medication of PD patients, since they do not only reverse the motor impairment but also can slow down or stop the progress of the disease by promoting cell survival.

Neuroprotective effects by $A_{2A}$ receptor antagonists have recently been reported in *in vivo* and *in vitro* models of different neurodegenerative diseases (for review see: Wardas J., *Pol J Pharmacol.* **2002,** 54(4), 313-26 and Stone TW. *Adv Exp Med Biol.* **2002,** 513, 249-80). $A_{2A}$ antagonists have been shown to be neuroprotective in different PD

models like in MPTP treated mice and 6-OHDA-lesioned rats. Here, KW-6002 prevented functional loss of dopaminergic nerve terminals in the striatum as well as prevented gliosis normally induced around degenerating neurons (Ikeda et al, **2002**, *J. Neurochem*., 80, 262-270; Hirsch et al, **1999,** *Adv. Neurol*., 80, 9-18; Kanda et al, **2000,** *Ann. Neurology*, 43 (4), 507-513, Lundblad et al. *J Neurochem.* **2003,** 84(6), 1398-410). Similar results have been obtained in experimental models of Huntington's disease (HD). In rat HD models quinolinic acid or kainate induced lesions were reduced after using adenosine $A_{2A}$ receptor antagonists, with a decrease in striatal cell loss and motor changes (Reggio et al, *Brain Res.* 831 (1-2), 12 June **1999,** Pages 315-318; Popoli et al, **2002,** *J. Neurosci*., 22, 1967-1975). In addition, it has been shown that$A_{2A}$ receptor antagonists decrease neuronal cell death after cerebral ischemia in neonatal and adult rats and gerbils (Gao Y, Phillis JW., *Life Sci.* **1994,** 55(3), PL61-5; Monopoli A. et al, *Neuroreport*, **1998,** 9(17), 3955-9). $A_{2A}$ knock out animals have been reported to be protected from neonatal hypoxic ischemia and transient focal ischemia (Bona E. et al, *Neuropharmacology*, **1997,** 36(9), 1327-38; Chen JF. et al, *J Neurosci*, **1999,** 19(21), 9192-200) and from 3NP (3-nitropropionic acid) induced, presynaptic, neurotoxic glutamate release (Blum D. et al, *J. Neurosci*, **2003,** 23 (12), 5361-9). The protective effect of $A_{2A}$ antagonists against neurodegeneration by glutamate release have allready been shown in a rat model of ischemic damage to the cerebral cortex (Simpson RE, *J Neurochem*, **1992,** 58(5), 1683-90 and O'Regan MH. et al, Brain Res, **1992,** 582(1), 22-6).

Protection by $A_{2A}$ antagonists has also been reported in primary astrocytes, in a rat model of bFGF induced astrogliosis, an amyloid beta peptide 25-35 induced neurotoxicity in cerebral granule cells (CGCs) and model of QA induced neuronal cell death in rat organotypic slice cultures (Brambilla R. et al. Glia. 2003 Aug;43(2):190-4; Dall'Igna OP. et al. Br J Pharmacol. 2003 Apr;138(7):1207-9; Tebano MT,. et al. Eur J Pharmacol. 2002 Aug 30;450(3):253-7)

Collectively, $A_{2A}$ receptor antagonists can efficiently protect different neurons from various forms of insult induced neurodegeneration (Abbracchio MP, Cattabeni F, Brain adenosine... Ann NY Acad Sci **1999** 890: 79-92; Ongini E. et al Adenosine A2A receptors and neuroprotection **1997**, 825: 30-48).

Adenosine and its analogues induce "depressant-like" effects in animal models of psychiatric disorders (Minor et al., **1994,** *Behav Neurosci* 108: 265-276; Woodson et al., **1998**, *Behav Neurosci* 112: 399-409). Moreover, these behavioural deficits were found to be reversed by adenosine $A_{2A}$ receptor antagonists (Minor et al., **2001,** *Behav Brain Res* 120: 230-212). Further studies have shown that treatment with adenosine or 2-chloroadenosine increased immobility time in the mouse forced swimming test, another animal model of depression generally considered reliable (Porsolt et al., **1977,** *Arch Int Pharmacodyn Ther* 229: 327-336).

Several compounds with dual affinity for $A_{2A}$ and $A_1$ receptor subtypes, known as the 4-amino[1,2,3]triazolo [4,3-*a*]quinoxalines, have been shown to be active in the rat forced swimming test (Sarges et al., **1990,** *J Med Chem* 33: 2240-2254) indicating antidepressant activity of the substances. Most recently, $A_{2A}$ receptor knockout mice were found to be less sensitive to "depressant" challenges than their wildtype littermates (El Yacoubi et al., **2001,** *Br J Pharmacol* 134: 68-77). Consistent with this data, the $A_{2A}$ receptor antagonists SCH58261 and KW6002 reduced the total immobility time in the mouse tail suspension test (El Yacoubi et al., **2001,** *Br J Pharmacol* 134: 68-77). The antagonists SCH58261 and ZM241385 4-(2-[7-amino-2-(2-furyl)[1,2,4]triazolo[2,3-*a*][1,3,5]triazin-5-ylamino]-ethyl)phenol were also found to reduce immobility when administered to mice previously screened for having high immobility time, while SCH58261 reduced immobility of mice that were selectively bred for their "helplessness" in this model (El Yacoubi et al., **2001,** *Br J Pharmacol* 134: 68-77).

Studies using $A_{2A}$ knockout mice suggest that these animals show a blunted response to psychostimulants such as amphetamine and cocaine, despite the fact that their expression and binding affinities of D1 and D2 receptors are unaffected (Chen et al., **2000,** *Neurosci* 97:195-204). Moreover, inactivation of $A_{2A}$ receptors has been shown to selectively attenuate amphetamine-induced behavioural sensitisation (Chen et al., **2003**, *Neuropsychopharmacol* 28: 1086-1095). In addition, $A_{2A}$ knockout mice show reduced startle and PPI of the acoustic startle (Wang et al., 2003), measures often used to detect antipsychotic activity. Further support is found in studies where pharmacological blockade of $A_{2A}$ receptors with a selective antagonist completely abolished pre-pulse imhibition (PPI) (Nagel et al., **2003,** *Synapse* 49: 279-286). Psychostimulants, such as MK-801 and amphetamine failed to disrupt startle and PPI in $A_{2A}$ KO mice (Wang et al., **2003,** *Behav Brain Res* 143: 201-207).

Thus, the available evidence suggests that adenosine $A_{2A}$ receptor antagonists, by specifically modulating mesostriatal or mesocorticolimbic dopaminergic pathways, may possess antidepressant and/or antipsychotic properties

Certain compounds of formula I in its broadest form, have according to Chemical Abstracts Registry database been disclosed in various chemical catalogs without indication of any pharmaceutical activity.

Certain compounds of formula I wherein $R^1$ is i-propyl are disclosed in WO2000026202 as being useful treating proliferative disorders associated with an altered cell dependent kinase activity.

Two compounds of formula I wherein $R^2$ is hydroxy are disclosed in DE855120 as antituberculosis agents.

Thus, $A_{2A}$ receptor antagonists show great potential as future drugs for long-term medication of PD patients, since they do not only reverse the motor impairment but also can slow down or stop the progress of the disease by promoting cell survival.

Hence, there is a desire for novel $A_{2A}$ receptor antagonists.

## Summary of the Invention

The objective of the present invention is to provide compounds that are antagonists at the $A_{2A}$ receptor.

Accordingly, the present invention relates to the use of compounds of formula I

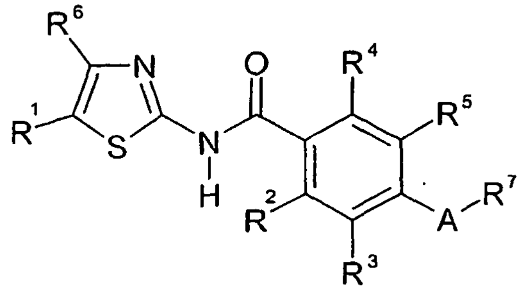

I

wherein $R^1$ and $R^6$ are independently hydrogen, $C_{1-6}$-alkyl or halogen;

$R^2$-$R^5$ are independently selected from the group consisting of hydrogen, halogen, cyano, OH, $NH_2$, nitro, $C_{1-6}$-alkyl, aryl, aryl-$C_{1-6}$-alkyl, heteroaryl-$C_{1-6}$-alkyl, $C_{3-8}$-cycloalkyl, $C_{3-8}$-cycloalkyl-$C_{1-6}$-alkyl, $C_{1-6}$-alkoxy, aryl-$C_{1-6}$-alkoxy, $C_{1-6}$-alkylamino and aryl-$C_{1-6}$-alkylamino wherein each alkyl, alkoxy or aryl may be optionally substituted with one or more halogen, cyano, $C_{1-6}$-alkyl, $C_{1-6}$-alkoxy, or $C_{1-6}$-alkoxy-$C_{1-6}$-alkoxy;

or $R^4$ and $R^5$ together are X-$(CH_2)_n$-Y, wherein X and Y independently are selected from the group consisting of $CH_2$, and NH and O, n is 1, 2 or 3, and $R^2$ and $R^3$ are as defined above;

A is *$NR^8$-CO, *CO-$NR^9$, *$NR^8$-CS or *CS-$NR^9$ in which $R^8$ and $R^9$ are independently selected from the group consisting of hydrogen and $C_{1-6}$-alkyl, or $R^8$ together with $R^3$ are $C_{2-3}$-alkylene or $CH_2CH_2O$ wherein the oxygen is attached to the phenylring, and the * indicates the atom that is attached to the phenyl ring;

and $R^7$ is selected from the group consisting of $C_{1-8}$-alkyl, aryl, heteroaryl, aryl-$C_{1-6}$-alkyl, heteroaryl-$C_{1-6}$-alkyl, $C_{3-8}$-cycloalkyl, $C_{3-8}$-cyloalkyl-$C_{1-6}$-alkyl, $C_{1-6}$-alkoxy, aryl-$C_{1-6}$-alkoxy, heteroaryl-$C_{1-6}$-alkoxy, $C_{1-6}$-alkylamino, aryl-$C_{1-6}$-alkylamino, heteroaryl-$C_{1-6}$-alkylamino, di-($C_{1-6}$-alkyl)-amino, 2,3-dihydrobenzo-[1,4]dioxin-2-yl or adamantan-1-yl-methyl wherein each alkyl and cycloalkyl may be optionally substituted with one or more halogen, cyano, hydroxy, oxo, $C_{1-6}$-alkoxy or $NR^{10}R^{11}$, wherein $R^{10}$ and $R^{11}$ independently are hydrogen or $C_{1-6}$-alkyl, or $R^{10}$ and $R^{11}$ together with the nitrogen form a 5, 6 or 7 membered aliphatic ring which optionally may contain one further heteroatom selected from N and O, and each aryl may be optionally substituted with one or more halogen, cyano, hydroxy, nitro, $C_{1-6}$-alkyl, $C_{1-6}$-alkoxy, $C_{1-6}$-acyl, $C_{1-6}$-acyloxy, $NR^{10}R^{11}$ wherein $R^{10}$ and $R^{11}$ independently are hydrogen or $C_{1-6}$-alkyl or $R^{10}$ and $R^{11}$ together with the nitrogen form a 5, 6 or 7 membered aliphatic ring which optionally may contain one further heteroatom selected from N and O, or a group Z-$(CH_2)_m$-W, wherein Z and W are attached to two adjacent carbon atoms and independently are selected from the group consisting of $CH_2$, NH and O, and m is 1, 2 or 3, provided that when $R^7$ is attached to nitrogen, then $R^7$ is not $C_{1-6}$-alkoxy, aryl-$C_{1-6}$-alkoxy, heteroaryl-$C_{1-6}$-alkoxy, $C_{1-6}$-alkylamino, aryl-$C_{1-6}$-alkylamino, heteroaryl-$C_{1-6}$-alkylamino or di-($C_{1-6}$-alkyl)-amino;

and pharmaceutically acceptable addition salts thereof;

for the manufacture of a medicament for treatment of a disease where an $A_{2A}$-receptor is implicated.

In a second aspect the present invention relates to a pharmaceutical composition comprising compounds of formula I as defined above provided that if A is *$NR^8$-CO, and $R^{1-6}$ and $R^8$ all are hydrogen, then $R^7$ is not thiophen-2-yl; and provided that if A is *$NR^8$-CO, $R^{2-6}$ and $R^8$ all are hydrogen, and $R^1$ is *i*-propyl then $R^7$ is not methyl or benzyl; and provided that if A is *$NR^8$-CO, $R^2$, $R^{4-6}$ and $R^8$ all are hydrogen, $R^3$ is.iodine and $R^1$ is *i*-propyl then $R^7$ is not methyl; and provided that if A is *$NR^8$-CO, $R^1$, $R^{3-6}$ and $R^8$ all are hydrogen and $R^2$ is hydroxy then $R^7$ is not methyl or ethoxy.

In a third aspect the present invention relates to compounds of ,formula I as defined above provided that if A is *$NR^8$-CO, and $R^{1-6}$ and $R^8$ all are hydrogen, then $R^7$ is not selected from the group consisting of $C_{1-4}$-alkyl, pentan-3-yl, trifluoromethyl, pyrimidyl, furan-2yl, thiophen-2-yl, substituted or unsubstituted phenyl or substituted or unsubstituted benzyl;

and provided that if A is *$NR^8$-CO, $R^{2-6}$ and $R^8$ all are hydrogen, and $R^1$ is *i*-propyl then $R^7$ is not methyl or benzyl;

and provided that if A is *$NR^8$-CO, $R^2$, $R^{4-6}$ and $R^8$ all are hydrogen, $R^3$ is iodine and $R^1$ is *i*-propyl then $R^7$ is not methyl;

and provided that if A is *$NR^8$-CO, $R^1$, $R^{3-6}$ and $R^8$ all are hydrogen and $R^2$ is hydroxy then $R^7$ is not methyl or ethoxy;

and provided that if A is *$NR^8$-CO, $R^2$, $R^{4-6}$ and $R^8$ all are hydrogen, $R^2$ is nitro and $R^7$ is methyl then $R^1$ is not hydrogen or methyl.

and provided that if A is *CO-$NR^9$, $R^1$, $R^6$ and $R^9$ all are hydrogen, and $R^7$ is thiazol-2-yl, then $R^{2-5}$ are not all hydrogen or all fluor;

and provided that if A is *CO-$NR^9$, $R^{2-5}$ and $R^9$ all are hydrogen, and $R^6$ is methyl, then $R^1$ may not be hydrogen if $R^7$ is 4-methyl-thiazol-2-yl and $R^1$ may not be methyl if $R^7$ is 4,5-dimethyl-thiazol-2-yl.

The compounds of the invention are $A_{2A}$ receptors antagonists having a human $A_{2A}$ binding affinity ($K_i$) of 5 μM or less, typically of 1 μM or less, preferably of 550 nM or less, more preferred of 200 nM or less, even more preferred of 50 nM or less and most preferred of 10 nM or less.

## Detailed Description of the Invention

In a particular embodiment the present invention relates to use of such compounds for the manufacture of a medicament for the treatment of a disease where an $A_{2A}$-receptor is implicated, is selected from the group consisting of Parkinson's Disease (PD), Alzheimer's Disease, Huntington's disease, epilepsia, cerebral ischemia, haemorrhagic stroke, neonatal ischemia and hypoxia, subarachnoid haemorrhage, traumatic brain injury, brain damage following cardiac arrest, and for the treatment of depression and psychosis disorders.

In a more particular embodiment the present invention relates to use of such compounds for the manufacture of a medicament for the treatment of Parkinson's Disease.

In a particular embodiment the present invention relates to such compounds wherein A is *$NR^8$-CO or *CO-$NR^9$, more particularly *$NR^8$-CO.

In another particular embodiment the present invention relates to such compounds wherein $R^7$ is selected from the group consisting of $C_{1-8}$-alkyl, preferably $C_{3-8}$-alkyl and even more preferred $C_{4-8}$-alkyl which is branched at the β-position, $C_{3-8}$-cycloalkyl-methyl, $C_{3-8}$-cyclalkyl, methylphenyl, methoxybenzyl and thiophen-2-yl-methyl, wherein each alkyl or cycloalkyl may be unsubstituted or substituted with oxo.

In another particular embodiment the present invention relates to such compounds wherein $R^8$ is hydrogen.

In another particular embodiment the present invention relates to such compounds wherein $R^9$ is hydrogen.

In another particular embodiment the present invention relates to such compounds wherein $R^6$ is hydrogen.

In another particular embodiment the present invention relates to such compounds wherein $R^1$ is hydrogen, methyl or chloro, preferably hydrogen.

In yet another particular embodiment the present invention relates to such compounds wherein $R^{2-5}$ are independently selected from the group consisting of hydrogen, halogen, $C_{1-6}$-alkyl, preferably methyl, $C_{1-6}$-alkoxy and $C_{1-6}$-alkoxy-$C_{1-6}$-alkoxy, preferably 2-methoxy-ethoxy.

In a more particular embodiment the present invention relates to such compounds wherein $R^2$ and $R^4$ are independently selected from the group consisting of hydrogen, $C_{1-6}$-alkoxy and $C_{1-6}$-alkoxy-$C_{1-6}$-alkoxy, preferably 2-methoxy-ethoxy.

In another more particular embodiment the present invention relates to such compounds wherein $R^3$ and $R^5$ are independently selected from the group consisting of hydrogen, halogen; $C_{1-6}$-alkyl, preferably methyl, $C_{1-6}$-alkoxy, preferably methoxy, and $C_{1-6}$-alkoxy-$C_{1-6}$-alkoxy, preferably 2-methoxy-ethoxy, trifluoromethyl and trifluoromethoxy.

Particular compounds of the invention are compounds 1-133 as disclosed in the

examples.

The compounds of the general formula I may exist as optical isomers thereof and such optical isomers are also embraced by the invention. Throughout the specification and claims, reference to specific compounds refers to the racemates unless otherwise indicated.

The term $C_{1-6}$-alkyl refers to a branched or unbranched alkyl group having from one to six carbon atoms inclusive, such as methyl, ethyl, 1-propyl, 2-propyl, 1-butyl, 2-butyl, 2-methyl-2-propyl, and 2-methyl-1-propyl. The term $C_{1-8}$-alkyl refers similarly to branched or unbranched alkyl group having from one to eight carbon atoms inclusive.

The term $C_{3-8}$-cycloalkyl designates a monocyclic or bicyclic carbocycle having three to eight C-atoms, such as cyclopropyl, cyclopentyl, cyclohexyl, etc.

Halogen means fluoro, chloro, bromo or iodo.

As used herein, the term acyl refers to a formyl, $C_{1-6}$-alkylcarbonyl, arylcarbonyl, aryl-$C_{1-6}$-alkylcarbonyl, $C_{3-8}$-cycloalkylcarbonyl or a $C_{3-8}$-cycloalkyl-$C_{1-6}$-alkylcarbonyl group.

The terms $C_{1-6}$-alkoxy, $C_{3-8}$-cycloalkyl-$C_{1-6}$-alkyl, aryl-$C_{1-6}$-alkyl, heteroaryl-$C_{1-6}$-alkyl, $C_{1-6}$-alkylamino, $C_{1-6}$-alkylcarbonyl, and the like, designate such groups in which the $C_{1-6}$-alkyl, aryl, heteroaryl and the $C_{3-8}$-cycloalkyl group are as defined above.

The term aryl refers to a carbocyclic aromatic group, such as phenyl or naphthyl, in particular phenyl.

The term heteroaryl refers to 5-membered monocyclic rings such as 1*H*-tetrazolyl, 3*H*-1,2,3-oxathiazolyl, 3*H*-1,2,4-oxathiazolyl, 3*H*-1,2,5-oxathiazolyl, 1,3,2-oxathiazolyl, 1,3,4-oxathiazolyl, 1,4,2-oxathiazolyl, 3*H*-1,2,4-dioxazolyl, 1,3,2-dioxazolyl, 1,4,2-dioxazolyl, 3*H*-1,2,3-dithiazolyl, 3*H*-1,2,4-dithiazolyl, 1,3,2-dithiazolyl, 1,4,2-dithiazolyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl,

1,3,4-thiadiazolyl, 1*H*-1,2,3-triazolyl, 1*H*-1,2,4-triazolyl, isoxazolyl, oxazolyl, isothiazolyl, thiazolyl, 1*H*-imidazolyl, 1*H*-pyrazolyl, 1*H*-pyrrolyl, furanyl, thienyl, 1*H*-pentazole; 6-membered monocyclic rings such as 1,2,3-oxathiazinyl, 1,2,4-oxathiazinyl, 1,2,5-oxathiazinyl, 4*H*-1,3,5-oxathiazinyl, 1,4,2-oxathiazinyl, 1,4,3-oxathiazinyl, 1,2,3-dioxazinyl, 1,2,4-dioxazinyl, 4*H*-1,3,2-dioxazinyl, 4*H*-1,3,5-dioxazinyl, 1,4,2-dioxazinyl, 2*H*-1,5,2-dioxazinyl, 1,2,3-dithiazinyl, 1,2,4-dithiazinyl, 4*H*-1,3,2-dithiazinyl, 4*H*-1,3,5-dithiazinyl, 1,4,2-dithiazinyl, 2*H*-1,5,2-dithiazinyl, 2*H*-1,2,3-oxadiazinyl, 2*H*-1,2,4-oxadiazinyl, 2*H*-1,2,5-oxadiazinyl, 2*H*-1,2,6-oxadiazinyl, 2*H*-1,3,4-oxadiazinyl, 2*H*-1,3,5-oxadiazinyl, 2*H*-1,2,3-thiadiazinyl, 2*H*-1,2,4-thiadiazinyl, 2*H*-1,2,5-thiadiazinyl, 2*H*-1,2,6-thiadiazinyl, 2*H*-1,3,4-thiadiazinyl, 2*H*-1,3,5-thiadiazinyl, 1,2,3-triazinyl, 1,2,4-triazinyl, 1,3,5-triazinyl, 2*H*-1,2-oxazinyl, 2*H*-1,3-oxazinyl, 2*H*-1,4-oxazinyl, 2*H*-1,2-thiazinyl, 2*H*-1,3-thiazinyl, 2*H*-1,4-thiazinyl, pyrazinyl, pyridazinyl, pyrimidyl, pyridyl, 2*H*-pyranyl, 2*H*-thiinyl; and to bicyclic rings such as 3*H*-1,2,3-benzoxathiazolyl, 1,3,2-benzodioxazolyl, 3*H*-1,2,3-benzodithiazolyl, 1,3,2-benzodithiazolyl, benzfurazanyl, 1,2,3-benzoxadiazolyl, 1,2,3-benzothiadiazolyl, 2,1,3-benzothiadiazolyl, 1*H*-benzotriazolyl, 1,2-benzisoxazolyl, 2,1-benzisoxazolyl, benzoxazolyl, 1,2-benzisothiazolyl, 2,1-benzisothiazolyl, benzothiazolyl, 1*H*-benzimidazolyl, 1*H*-indazolyl, 3*H*-1,2-benzoxathiolyl, 1,3-benzoxathiolyl, 3*H*-2,1-benzoxathiolyl, 3*H*-1,2-benzodioxolyl, 1,3-benzodioxolyl 3*H*-1,2-benzodithiolyl, 1,3-benzodithiolyl, 1*H*-indolyl, 2*H*-isoindolyl, benzofuranyl, isobenzofuranyl, 1-benzothienyl, 2-benzothienyl, 1*H*-2,1-benzoxazinyl, 1*H*-2,3-benzoxazinyl, 2*H*-1,2-benzoxazinyl, 2*H*-1,3-benzoxazinyl, 2*H*-1,4-benzoxazinyl, 2*H*-3,1-benzoxazinyl, 1*H*-2,1-benzothiazinyl, 1*H*-2,3-benzothiazinyl, 2*H*-1,2-benzothiazinyl, 2*H*-1,3-benzothiazinyl, 2*H*-1,4-benzothiazinyl, 2*H*-3,1-benzothiazinyl, cinnolinyl, phtalazinyl, quinazolinyl, quinoxalinyl, isoquinolyl, quinolyl, 1*H*-2-benzopyranyl; 2*H*-1-benzopyranyl, 1*H*-2-benzothiopyranyl or 2*H*-1-benzothiopyranyl.

The term *rac* means racemic.

The acid addition salts of the compounds of the invention are pharmaceutically acceptable salts formed with non-toxic acids. Exemplary of such organic salts are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic and theophylline acetic acids, as well as the 8-halotheophyllines, for example 8-bromotheophylline. Exemplary of such inorganic salts are those with hydrochloric, hydrobromic, sulfuric, sulfamic, phosphoric and nitric acids.

The pharmaceutical compositions of this invention, or those which are manufactured in accordance with this invention, may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients or other additives normally used in the art may be used.

Conveniently, the compounds of the invention are administered in unit dosage form containing said compounds in an amount of about 0.01 to 100 mg.

The total daily dose is usually in the range of about 0.05 - 500 mg, and most preferably about 0.1 to 50 mg of the active compound of the invention.

The compounds of the invention are prepared by the following general methods:

a) Coupling of a compound with formula II

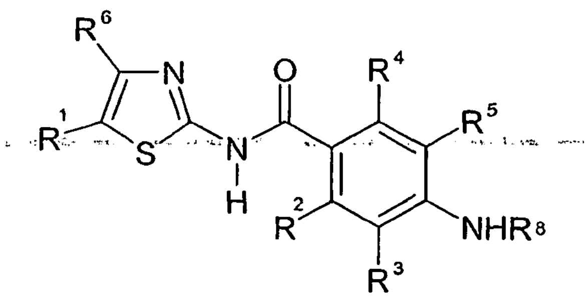

II

wherein $R^1$ - $R^8$ is as described above, with a carboxylic acid $R^7$-COOH or carboxylic acid chloride $R^7$-COCl, wherein $R^7$ is as defined above.

The coupling of compounds of formula II with carboxylic acids $R^7$-COOH is performed by standard procedures knowledgeable to chemists skilled in the art. This includes coupling in the presence of a uronium salt coupling reagent and diisopropyethylamine (DIPEA), at temperatures between 20-80 °C, in a suitable polar or apolar solvent such as NMP or 1,2-dichloroethane, or coupling of starting materials of formula II with carboxylic acid chlorides $R^7$-COCl in the presence of a suitable base such as pyridine at temperatures between 20-60 °C in a suitable solvent such as 1,2-dichloroethane

b) Condensation of a compound with formula II with isocyanates $R^7$-NCO wherein $R^7$ is as defmed above
The coupling of compounds of formula II with isocyanates $R^7$-NCO is performed by standard procedures knowledgeable to chemists skilled in the art. This includes condensation at temperatures between 20-150 °C in a suitable polar or apolar solvent such as NMP or 1,2-dichloroethane.

c) Condensation of a compound with formula II with a chloroformate $R^7$-OCOCl wherein $R^7$ is as defined above.
The coupling of compounds of formula II with chloroformates $R^7$-OCOCl is performed by standard procedures knowledgeable to chemists skilled in the art. This includes condensation at temperatures between 20-80 °C in a suitable polar or apolar solvent such as NMP or 1,2-dichloroethane in the presence of a suitable base, such as triethylamine.

d) Coupling of a compound with formula III

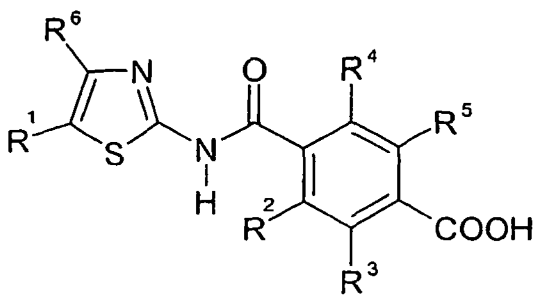

III

wherein $R^1$-$R^6$ is as described above, with an amine $HN(R^9)R^7$, wherein $R^9$ and $R^7$ is as defined above.

The coupling of compounds of formula III with amines $HN(R^9)R^7$ is performed by standard procedures knowledgeable to chemists skilled in the art. This includes coupling in the presence of a uronium salt coupling reagent and diisopropyethylamine (DIPEA), at temperatures between 20-80 °C, in a suitable polar or apolar solvent such as NMP or 1,2-dichloroethane.

Compounds of formula II were prepared according to standard procedures known to chemists skilled in the art as outlined below. Suitably substituted 4-nitro benzoic acid chlorides were either commercially available or prepared by chlorination of the corresponding carboxylic acids with oxalylchloride or sulfonyl chloride, and were coupled with suitably substituted 2-amino thiazoles in a suitable solvent such as 1,2-dichloroethane in the presence of a suitable base such as pyridine, at a suitable temperature between 20-60 °C. The products were then reduced to the corresponding anilines by procedures known to chemists skilled in the art, such as catalytic hydrogenation using hydrogen and a suitable catalyst such as 5% Pd/C in a suitable solvent such as ethanol; or reduction using a suitable metal reagent such as $SnCl_2$ or Zn (s) and a suitable acid such as HCl, at a suitable temperature such as room temperature and in a suitable solvent such as acetic acid or ethanol. Alternatively, starting materials of formula II were prepared by reaction of suitably substituted *N*-protected 4-amino benzoic acids by chlorination of the carboxylic acid, and coupling with suitably substituted 2-amino thiazoles, under the same conditions as described above, followed by deprotection of the amino functionality under suitable conditions, such as acidolysis. Alternatively, suitably substituted 4-amino benzoic acids were coupled with suitably substituted 2-amino thiazoles in the presence of a carbodiimide coupling reagent such as 1-(3-dimethylaminopropyl)-3-ethyl-carbodiimide hydrochloride in the presence of a suitable additive such as 1-hydroxybenzotriazole in a suitable solvent such as 1,2-dichloroethane in the presence of a suitable base such as DIPEA, at a suitable temperature between 20-60 °C.

Compounds of formula III were prepared according to standard procedures known to chemists skilled in the art as outlined below. Suitably substituted methyl 4-chlorocarbonylbenzoates were either commercially available or prepared by chlorination of the corresponding carboxylic acids with oxalylchloride or sulfonyl chloride, and were coupled with 2-amino thiazole in a suitable solvent such as 1,2-dichloroethane in the presence of a suitable base such as pyridine, at a suitable temperature between 20-60 °C. The products were then saponified to the corresponding carboxylates by procedures known to chemists skilled in the art, such as treatment with 2M NaOH (aq.) at a suitable temperature such as room temperature in the presence of a suitable organic co-solvent such as THF, followed by acidification to yield the carboxylic acid products.

## Experimental Section

Analytical LC-MS data were obtained by either of two methods: (method A): on a PE Sciex API 150EX instrument equipped with an IonSpray source and a Shimadzu LC-8A/SLC-10A LC system. Column: 30 X 4.6 mm Waters Symmetry C18 column with 3.5 μm particle size; solventsystem: A = water/trifluoroacetic acid (100:0.05) and B = water/acetonitrile/trifluoroacetic acid (5:95:0.03); method: Linear gradient elution with 90% A to 100% B in 4 min and with a flow rate of 2 ml/min. or (method B): on a Micromass LCT instrument equipped with a 4-way MUX ElectroSpray source, a Micromass Waters MUX-2488 UV-detector, a Sedex 754 4-channels LT-ELS-detector, a CTC Analytics HTS-PAL autosampler equipped with 4 injection valves, and 4 Waters 1525 Binary HPLC pumps. Column: 30 X 4.6 mm Waters Symmetry C18 column with 3.5 μm particle size; solventsystem: A = water/trifluoroacetic acid (100:0.05) and B = water/acetonitrile/trifluoroacetic acid (5:95:0.03); method: Linear gradient elution with 90% A to 100% B in 4 min and with a flow rate of 2 ml/min.

Purity was determined by integration of the UV (254 nm) and ELSD traces. The retention times (RT) are expressed in minutes.

$^1H$ NMR spectra were recorded at 500.13 MHz on a Bruker Avance DRX500 instrument or at 250.13 MHz on a Bruker AC 250 instrument. Deuterated dimethyl sulfoxide (99.8%D) was used as solvent. TMS was used as internal reference standard. Chemical shift values are expressed in ppm. The following abbreviations are used for multiplicity of NMR signals: s = singlet, d = doublet, t = triplet, q = quartet, qui = quintet, h = heptet, dd = double doublet, dt = double triplet, dq = double quartet, tt = triplet of triplets, m = multiplet, br s = broad singlet and br = broad signal.

For column chromatography silica gel of the type Kieselgel 60, 40-60 mesh ASTM was used. Microwave heated experiments were performed with a Personal Chemistry Emrys Synthesiser or a Personal Chemistry Emrys Optimiser.

## Examples

### Preparation of intermediates

4-Amino-*N*-thiazol-2-yl-benzamide:

2-Amino thiazole (100 mmol) was suspended in 1,2-dichloroethane (200 mL) and pyridine (100 mmol) was added. The mixture was added portion wise to a suspension of 4-nitrq benzoic acid chloride (150 mmol) in 1,2-dichloroethane (500 mL) and stirred at 60 °C over night. The reaction mixture was cooled and filtered. The filtrate was washed with 1,2-dichloroethane and dried *in vacuo*.
Yield: 96%
1H NMR ($D_6$-DMSO): 7.33 (d, 1H); 7.60 (d, 1H); 8.26-8.41 (4H); 12.96 (br s, 1H).

4-Nitro-*N*-thiazol-2-yl-benzamide (28 mmol) was suspended in abs. EtOH (400 mL) and ethyl acetate (200 mL) and glacial acetic acid (50 mL) was added followed by 10% Pd/C (0.5 g). The mixture was hydrogenated for 72h at 3 bar $H_2$. The hydrogenation mixture was filtered, and the solvent was removed under reduced pressure. The crude product was added $NaHCO_3$ (sat.) and ethyl acetate, the remaining solid fraction was removed by filtration and dried *in vacuo*. The liquid phases were separated, the organics were washed with brine, dried over $MgSO_4$, filtered and evaporated to yield a solid. The solid fractions were pure product and were combined.
Yield: 83% (80% overall).
1H NMR ($D_6$-DMSO): 5.93 (s, 2H); 6.50 (d, 2H); 7.18 (d, 1H); 7.49 (d, 1H); 7.84 (d, 2H); 12.05 (br s, 1H).

4-Amino-3-methyl-*N*-thiazol-2-yl-benzamide:

4-Nitro-3-methyl-benzoic acid (83 mmol) was suspended in 1,2-dichloroethane (500 mL) and dimethylformamide (DMF) (5 mL) under an argon atmosphere. Oxalylchloride (2M in dichloromethane, 62.3 mL) was added slowly to the stirred suspension. After stirring at room temperature for 1h, the solvent was removed by evaporation under reduced pressure, and the reaction mixture was re-dissolved in 1,2-dichloroethane (400 mL). A suspension of 2-amino thiazole (83 mmol) and pyridine (83 mmol) in 1,2-dichloroethane (100 mL) was added portion wise. The reaction mixture was stirred at 50 °C over night. The solvent was removed under reduced pressure and the solids were re-suspended in ethyl acetate (500 mL) and $NaHCO_3$ (sat.) (500 mL). The solids were removed. by filtration (pure product) and the liquid phases were separated. The organic phase was washed with $NaHCO_3$ (sat.), dried over $MgSO_4$, filtered and evaporated. The crude was re-crystallized from ethyl acetate and the product fractions were combined.
Yield: 76%.
1H NMR ($D_6$-DMSO): 2.58 (s, 3H); 7.33 (d, 1H); 7.60 (d, 1H); 8.10 (d, 2H); 8.20 (d, 2H); 12.92 (br s, 1H).

4-Nitro-3-methyl-*N*-thiazol-2-yl-benzamide (63 mmol) was suspended in abs. EtOH (200 mL) and ethyl acetate (100 mL) and glacial acetic acid (10 mL) was added followed by 10% Pd/C (1 g). The mixture was hydrogenated over

night at 3 bar $H_2$. The hydrogenation mixture was filtered and the solvent was removed under reduced pressure. The crude product was added $NaHCO_3$ (sat.) and ethyl acetate, the remaining solid fraction was removed by filtration and dried *in vacuo*. The liquid phases were separated, the organics were washed with brine, dried over $MgSO_4$, filtered and evaporated to yield a solid. The solid fractions were product and were combined.
Yield: 95% (72% overall).
1H NMR ($D_6$-DMSO): 2.09 (s, 3H); 5.71 (s, 2H); 6.63 (d, 1H); 7.17 (d, 1H); 7.39 (d, 1H); 7.69-7.81 (m, 2H); 11.96 (br s, 1H).
The following compounds were prepared analogously:

4-Amino-2-methoxy-*N*-thiazol-2-yl-benzamide:
Yield: 53%
1H NMR ($D_6$-DMSO): 3.45 (s, 3H); 6.15 (s, 2H); 6.25-6.35 (2H); 7.20 (d, 1H); 7.46 (d, 1H); 7.71 (d, 1H); 10.97 (s, 1H).

4-Amino-3-methoxy-*N*-thiazol-2-yl-benzamide:
Yield: 17%
1H NMR ($D_6$-DMSO): 3.85 (s, 3H); 5.59 (s, 2H); 6.67 (d, 1H); 7.19 (d, 1H); 7.48-7.65 (3H); 12.17 (br s, 1H).

4-Amino-*N*-(5-chloro-thiazol-2-yl)-benzamide:
Yield: 34%
1H NMR ($D_6$-DMSO): 6.02 (s, 2H); 6.59 (d, 2H); 7.53 (s, 1H); 7.83 (d, 2H); 12.29 (br s, 1H).

4-Amino-*N*-(5-methyl-thiazol-2-yl)-benzamide:
Yield: 16%
1H NMR ($D_6$-DMSO): 2.35 (d, 3H); 5.90 (s, 2H); 6.57 (d, 2H); 7.15 (d, 1H); 7.81 (d, 2H); 11.83 (s, 1H).

4-Amino-2-chloro-*N*-thiazol-2-y-benzamide:

4-Nitro-2-chloro-*N*-thiazol-2-yl-benzamide (5 mmol) was dissolved in glacial acetic acid (20 mL) and added to a mixture of $SnCl_2$ in HCl (conc.). The reaction was stirred at room temperature over night, then poured onto ice and neutralized with NaOH. The aqueous phase was extracted with ethyl acetate, the organics were combined, dried over $MgSO_4$, filtered and evaporated. The crude product was purified by flash chromatography on silica using a gradient of ethyl acetate in heptane.
Yield: 46% (overall)
1H NMR ($D_6$-DMSO): 5.95 (s, 2H); 6.52 (m, 1H); 6.65 (d, 1H); 7.23 (d, 1H); 7.36 (d, 1H); 7.50 (d, 1H); 12.18 (s, 1H).

4-Amino-2-methyl-*N*-thiazol-2-yl-benzamide:

4-Acetylamino-2-methyl-benzoic acid (16 mmol) was suspended in 1,2-dichloroethane (100 mL) and DMF (1 mL) under an argon atmosphere. Oxalylchloride (2M in dichloromethane, 12 mL) was added slowly to the stirred suspension. After stirring at room temperature for 1h the solvent was removed by evaporation under reduced pressure, and the reaction mixture was re-dissolved in 1,2-dichloroethane (80 mL). A suspension of 2-amino thiazole (16 mmol) and pyridine (16 mmol) in 1,2-dichloroethane (20 mL) was added portion wise. The reaction mixture was stirred at 50 °C over night. The solvent was removed under reduced pressure and the solids were re-suspended in ethyl acetate (100 mL) and $NaHCO_3$ (sat.) (100 mL). The liquid phases were separated and the aqueous phase was extracted with ethyl acetate. The combined organic phases were washed with water, dried over $MgSO_4$, filtered and evaporated. The crude product was purified by flash chromatography on silica using gradient elution (heptane/ethyl acetate).
Yield: 25%.
4-Acetylamino-2-methyl-*N*-thiazol-2-yl-benzamide (4 mmol) was refluxed over night in HCl (8M) (50 mL). The pH was adjusted to 8 with NaOH (aq., conc.) and the product was removed by filtration, washed with water and dried *in vacuo*.
Yield: 13% (3% overall)
1H NMR ($D_6$-DMSO): 2.35 (s, 3H); 5.66 (br s, 2H); 6.36-6:46 (2H); 7.17 (d, 1H); 7.42 (d, 1H); 7.48 (d, 1H).

4-Amino-3-fluoro-*N*-thiazol-2-yl-benzamide:

4-Nitro-3-fluoro-benzoic acid (535 mmol) was dissolved in toluene (500 mL) and THF (75 mL). $SOCl_2$ (930 mmol) was added and the mixture was heated at 65 °C for 5h. The reaction mixture was cooled and the solvent removed by evaporation. The residue was re-dissolved in 1,2-dichloroethane. This solution was added dropwise to a suspension of 2-amino-thiazole (480 mmol) and DIPEA (370 mmol) in 1,2-dichloroethane (1L) with mechanical stirring, while the

temperature was kept at 45 °C. Upon complete addition the reaction mixture was heated at 60 °C for 1.5h, then allowed to cool to room temperature and stirred over night. The reaction mixture was filtered, the solids were washed with 1,2-dichloroethane and dried *in vacuo*.
Yield: 35%
1H NMR ($D_6$-DMSO): 7.34 (d, 1H); 7.61 (d, 1H); 8.10 (m, 1H); 8.23 (m, 1H); 8.31 (m, 1H); 13.00 (br, 1H).

4-Nitro-3-fluoro-*N*-thiazol-2-yl-benzamide (7.5 mmol) was suspended in EtOH (abs., 60 mL) and ethyl acetate (30 mL), glacial acetic acid (5 mL) and 10 % Pd/C (300 mg) was added, and the mixture was hydrogenated for 12 days under 3 bar $H_2$. The reaction mixture was filtered and evaporated, and re-dissolved in ethyl acetate (100 mL) and $NaHCO_3$ (sat., 60 mL). The aqueous phase was adjusted to basic pH with NaOH (1M) and the phases were separated. The organic phase was washed with brine, dried over $MgSO_4$, filtered and evaporated.
Yield: 85% (30% overall)
1H NMR ($D_6$-DMSO): 6.00 (s, 2H); 6.80 (t, 1H); 7.21 (d, 1H); 7.51 (d, 1H); 7.74 (m, 1H); 7.81 (m, 1H); 12.19 (s, 1H).

The following compounds were prepared analogously:

4-Amino-2-fluoro-*N*-thiazol-2-yl-benzamide:
Yield: 16%
1H NMR ($D_6$-DMSO): 6.19 (s, 2H); 6.35 (m, 1H); 6.43 (m, 1H); 7.21 (d, 1H); 7.48-7.55 (2H); 11.64 (br, 1H).

4-Amino-*N*-(4,5-dimethyl-thiazol-2-yl)-benzamide:

4-*tert*-Butoxycarbonylamino-benzoic acid (6.3 mmol) and 1-hydroxybenzotriazole (6.3 mmol) were combined in a flask and suspended in 1,2-dichloroethane (30 mL). 1-(3-Dimethylaminopropyl)-3-ethyl-carbodiimide hydrochloride (6.3 mmol) and DIPEA (15.4 mmol) was added followed by 4,5-dimenthyl-2-amino-thiazole hydrochloride (9.1 mmol). The resulting solution was stirred at ambient temperature over night, then the reaction mixture was washed extensively with AcOH (aq., pH ~3), dried over $MgSO_4$, filtered and evaporated. During evaporation of the solvent the desired product precipitated and was collected by filtration, washed with 1,2-dichloroethane and dried.
Yield: 20%
1H NMR ($D_6$-DMSO): 1.50 (s, 9H); 2.20 (s, 3H); 2.26 (s, 3H); 7.58 (d, 2H); 8.02 (d, 2H); 9.73 (s, 1H); 12.20 (br s, 1H).

[4-(Thiazol-2-ylcarbamoyl)-phenyl]-carbamic acid *tert*-butyl ester was deprotected prior to use by dissolution in dichoromethane/trifluoroacetic acid (1:1) for 10 min., followed by evaporation of the solvent. The residue was taken up in dichloromethane, and extracted with NaOH (0,1M). The formed precipitate was the product, and was removed by filtration, washed with water and dried.

4-Methylamino-*N*-thiazol-2-yl-benzamide:

4-Amino-benzoic acid ethyl ester (60.5 mmol) was dissolved in 1,2-dichloroethane (100 mL), and a catalytic amount of 4-(*N*, *N*-dimethylamino) pyridine was added followed by acetic acid anhydride (66.6 mmol) in 1,2-dichloroethane (15 mL). The reaction mixture was stirred at room temperature for 24h, then the solvent was evaporated and the residue was re-dissolved in ethyl acetate (200 mL) and extracted with HCl (0.1M) x2, $Na_2CO_3$ (aq., sat.) x2, $H_2O$ and brine. The organic phase was dried over $MgSO_4$, filtered and evaporated.
Yield: 90%
1H NMR ($D_6$-DMSO): 1.31 (t, 3H); 2.09 (s, 3H); 4.28 (q, 2H); 7.72 (d, 2H); 7.90 (d, 2H); 10.28 (s, 1H).

4-Acetylamino-benzoic acid ethyl ester (54.5 mmol) was dissolved in THF (100 mL), and potassium *tert*-butoxide (54.5 mmol) was added followed by methyl iodide (60 mmol). The reaction mixture was stirred at room temperature for 1h, then the solvent was evaporated. The crude product was used in the next reaction without further purification.

Crude 4-(Acetyl-methyl-amino)-benzoic acid ethyl ester was refluxed over night in conc. HCl (100 mL). The mixture was cooled and a small amount of sodium sulfite was added. The pH was adjusted to 4 with NaOH (aq., conc.), this gave a heavy precipitation and the solids were removed by filtration, washed with water and dried *in vacuo*.
Yield: 74%
1H NMR ($D_6$-DMSO): 2.72 (d, 3H); 6.45 (q, 1H); 6.53 (d, 2H); 7.68 (d, 2H).

4-Methylamino-benzoic acid (13.2 mmol) was dissolved in DMF (4 mL) and 1,2-dichloroethane (25 mL). DIPEA (13.2 mmol), 1-(3-dimethylaminopropyl)-3-ethyl-carbodiimide hydrochloride (13.2 mmol), 1-hydroxybenzotriazole (13.2 mmol) and 2-aminothiazole (13.2 mmol) was added, and the reaction mixture was stirred at 40 °C for 72h. HCl (2M) (13.2 mmol) was added followed by water (20 mL), upon which a solid precipitated. This was removed by filtration, washed with water and dried in vacuo.
Yield: 32% (22% overall)
1H NMR ($D_6$-DMSO): 2.75 (s, 3H); 3.93 (br, 1H); 6.59 (d, 2H); 7.19 (d, 1H); 7.51 (d, 1H); 7.92 (d, 2H); 12.12 (br s, 1H).

4-Propylamino-*N*-thiazol-2-yl-benzamide:

4-Amino-*N*-thiazol-2-yl-benzamide (2.28 mmol) was suspended in THF (10 mL) and propanal (3.42 mmol) was added followed by glacial acetic acid (4.2 mmol) and $NaBH(OAc)_3$ (4.56 mmol). The mixture was stirred at room temperature over night. More $NaBH(OAc)_3$ (2.28 mmol) was added and stirring continued for 3.5h. The solvent was removed by evaporation under reduced pressure and the residue was re-dissolved in ethyl acetate (100 mL) and $NaHCO_3$ (sat.) (40 mL) (pH of the aqueous phase was adjusted to 11). The phases were separated and the aqueous phase was extracted with ethyl acetate. The combined organic extracts were washed with brine, dried over $MgSO_4$, filtered and evaporated. The crude product was re-crystallized from EtOH.
Yield: 36%
1H NMR ($D_6$-DMSO): 0.94 (t, 3H); 1.57 (m, 2H); 3.05 (m, 2H); 6.49 (t, 1H); 6.60 (d, 2H); 7.18 (d, 1H); 7.50 (d, 1H); 7.89 (d, 2H); 12.07 (s, 1H).

4-Amino-2-propoxy-*N*-thiazol-2-yl-benzamide:

NaH (60 % in oil suspension) (30 mmol) was weighed into a flask, and DMF (30 mL) was added followed by drop wise addition of 1-propanol. 2-Fluoro-4-nitro-*N*-thiazol-2-yl-benzamide (7.5 mmol) was added portion wise. The mixture was stirred over night at 80 °C and then poured into water (90 mL). HCl (22 mmol) was added and the aqueous phase was extracted with ethyl acetate. The organic fractions were washed with brine, dried over $MgSO_4$, filtered and evaporated. The crude product was purified by flash chromatography on silica using gradient elution (heptane/ethyl acetate).
Yield: 11 %
1H NMR ($D_6$-DMSO): 0.98 (t, 3H); 1.78 (m, 2H); 4.21 (t, 2H); 7.32 (d, 1H); 7.55 (d, 1H); 7.86-7.93 (3H).

4-Nitro-2-propoxy-*N*-thiazol-2-yl-benzamide (0.8 mmol) was suspended in EtOH (30 mL) and ethyl acetate (6 mL), glacial acetic acid (2.5 mL) and 10 % Pd/C (40 mg) was added, and the mixture was hydrogenated under 3 bar $H_2$ for 3 days. The reaction mixture was filtered and evaporated, re-dissolved in ethyl acetate, and extracted with $NaHCO_3$ (sat.). The organics were dried over $MgSO_4$, filtered and evaporated to yield the product.
Yield: 96% (10% overall)
1H NMR ($D_6$-DMSO): 0.98 (t, 3H); 1.77 (m, 2H); 4.21 (t, 2H); 7.32 (d, 1H); 7.55 (d, 1H); 7.86-7.96 (3H); 12.25 (br s, 1H).

The following compounds were prepared analogously:

4-Amino-2-(2-methoxy-ethoxy)-*N*-thiazol-2-yl-benzamide:
Yield: 16%
1H NMR ($D_6$-DMSO): 3.32 (s, 3H); 3.74 (t, 2H); 4.44 (t, 2H); 7.33 (d, 1H); 7.56 (d, 1H); 7.92-8.01 (3H); 12.14 (s, 1H).

4-Amino-3-(2-methoxy-ethoxy)-*N*-thiazol-2-yl-benzamide:
Yield: 20%
1H NMR ($D_6$-DMSO): 3.35 (s, 3H); 3.72 (t, 2H); 4.18 (t, 2H); 5.53 (s, 2H); 6.69 (d, 1H); 7.19 (d, 1H); 7.51 (d, 1H); 7.57 (dd, 1H); 7.64 (d, 1H); 12.14 (s, 1H).

4-Amino-3-propoxy-N-thiazol-2-yl-benzamide:
Yield: 11%
$^1$H-NMR ($D_6$-DMSO): 1.05 (t, 3H); 1.7 (m, 2H); 4.0 (t, 2H); 5.5 (br, 2H), 6.7 (d, 1H); 7.2 (d, 1H); 7.5 (d, 1H); 7.55 (d, 1H); 7.6 (s, 1H), 12.15 (br, 1H).

4-Amino-3-chloro-*N*-thiazol-2-yl-benzamide:

4-Amino-3-chloro-benzoic acid methyl ester (21.6 mmol) was saponified in EtOH (25 ml) and NaOH (1M, 25 ml) at reflux for 2h. The organic solvent was evaporated and pH adjusted to 4. The product was removed by filtration, washed with water and dried *in vacuo*.
Yield: 92%
1H NMR ($D_6$-DMSO): 6.15 (s, 2H); 6.79 (d, 1H); 7.59 (dd, 1H); 7.71 (d, 1H); 12.37 (br s, 1H).

4-Amino-3-chloro-benzoic acid (19.8 mmol) was dissolved in DMF (10 mL) and 1,2-dichloroethane (80 mL). DIPEA (19.8 mmol), 1-(3-dimethylaminopropyl)-3-ethyl-carbodiimide hydrochloride (19.8 mmol), 1-hydroxybenzotriazole (19.8 mmol) and 2-aminothiazole (19.8 mmol) was added, and the reaction mixture was stirred at 60 °C over night. The volume was reduced *in vacuo,* and water (60 mL) was added. The mixture was extracted with ethyl acetate, the organic phase was washed with $NH_4Cl$ (aq., sat.), dried over $MgSO_4$, filtered and evaporated. The crude product was purified by flash chromatography on silica using gradient elution (heptane/ethyl acetate).

Yield: 42% (39% overall)
1H NMR ($D_6$-DMSO): 6.19 (s, 2H); 6.83 (d, 1H); 7.21 (d, 1H); 7.52 (d, 1H); 7.83 (dd, 1H); 8.07 (d, 1H), 12.24 (br s, 1H).

4-Amino-3-bromo-*N*-thiazol-2-yl-benzamide:

4-Amino-benzoic acid (100 mmol) was dissolved in DMF (50 mL) and *N*-bromosuccinimide (100 mmol) was added. Stirred at ambient temperature for 18h, the reaction mixture was then poured into water (100 mL). The product was removed by filtration, washed with water and dried *in vacuo*.
Yield: 70%
1H NMR ($D_6$-DMSO): 6.10 (s, 2H); 6.78 (d, 1H); 7.63 (dd, 1H); 7.89 (d, 1H); 12.39 (br s, 1H).

4-Amino-3-bromo-benzoic acid (18.5 mmol) was dissolved in DMF (10 mL) and 1,2-dichloroethane (80 mL). DIPEA (18.5 mmol), 1-(3-dimethylaminopropyl)-3-ethyl-carbodiimide hydrochloride (18.5 mmol), 1-hydroxybenzotriazole (18.5 mmol) and 2-aminothiazole (18.5 mmol) was added and the reaction mixture was stirred at 60 °C over night. The volume was reduced *in vacuo*, and water (60 mL) was added. The mixture was extracted with ethyl acetate, the organic phase was washed with $NH_4Cl$ (aq., sat.), dried over $MgSO_4$, filtered and evaporated. The crude product was purified by flash chromatography on silica using gradient elution (heptane/ethyl acetate).
Yield: 33% (23% overall)
1H NMR ($D_6$-DMSO): 6.14 (s, 2H); 6.82 (d, 1H); 7.21 (d, 1H); 7.51 (d, 1H); 7.86 (dd, 1H); 8.22 (d, 1H); 12.24 (br s, 1H).

4-Amino-5-chloro-2-methoxy-*N*-thiazol-2-yl-benzamide:

4-Amino-5-chloro-2-methoxy-benzoic acid (19.8 mmol)) was dissolved in DMF (10 mL) and 1,2-dichloroethane (80 mL). DIPEA (19.8 mmol), 1-(3-dimethylaminopropyl)-3-ethyl-carbodiimide hydrochloride (19.8 mmol), 1-hydroxybenzotriazole (19.8 mmol) and 2-amino thiazole (19.8 mmol) was added and the reaction mixture was stirred at 60°C over night. The volume was reduced *in vacuo,* and water (60 mL) was added. The mixture was extracted with ethyl acetate, the organic phase was washed with $NH_4Cl$ (aq., sat.), dried over $MgSO_4$, filtered and evaporated. The crude product was re-crystallized from ethyl acetate.
Yield: 32%
1H NMR ($D_6$-DMSO): 3.94 (s, 3H); 6.30 (s, 2H); 6.56 (s, 1H); 7.23 (d, 1H); 7.49 (d, 1H); 7.76 (s, 1H); 11.05 (br s, 1H).

The following compounds were prepared analogously:

8-Amino-2,3-dihydro-benzo[1,4]dioxine-5-carboxylic acid thiazol-2-ylamide:
Yield: 33%
1H NMR ($D_6$-DMSO): 4.34 (m, 2H); 4.49 (m, 2H); 5.68 (s, 2H); 5.37 (d, 1H); 7.21 (d, 1H); 7.34 (d, 1H); 7.48 (d, 1H); 10.94 (br s, 1H).

4-Amino-3,5-difluoro-N-thiazol-2-yl-benzamide:
Yield: 27%
LC/MS (m/z) 256 (MH+); RT = 1.9 (method A); purity (UV, ELSD): 94%; 99%.

4-Amino-N-thiazol-2-yl-3-trifluoromethoxy-benzamide:
Yield: 71%
LC/MS (m/z) 304 (MH+); RT = 2.3 (method A); purity (UV, ELSD): 64%; 98%.

4-Amino-3-chloro-5-methyl-N-thiazol-2-yl-benzamide:
Yield: 1.1%
LC/MS (m/z) 268 (MH+); RT = 2.2 (method A); purity (UV, ELSD): 97%; 99%.

4-Amino-3,5-dimethyl-N-thiazol-2-yl-benzamide:
Yield: 69%
LC/MS (m/z) 248 (MH+); RT = 1.8 (method A); purity (UV, ELSD): 85%; 99%.

4-Amino-N-thiazol-2-yl-3-trifluoromethyl-benzamide:

The starting material: 4-amino-3-trifluoromethyl-benzoic acid was prepared according to literature procedures: Krüger et al. Arzneim.Forsch.; 34; 11a; 1984; 1612-1624. Yield: 14%

4-Amino-3-chloro-N-thiazol-2-yl-5-trifluoromethyl-benzamide:

The starting material: 4-amino-3-chloro-5-trifluoromethyl-benzoic acid was prepared according to literature procedures: Krüger et al. Arzneim.Forsch.; 34; 11a; 1984; 1612-1624.
Yield: 20%
LC/MS (m/z) 322 (MH+); RT = 2.6 (method A); purity (UV, ELSD): 97%; 99%.

5-Amino-biphenyl-2-carboxylic acid thiazol-2-ylamide:

2-Bromo-4-nitro-toluene (50 mmol), phenyl boronic acid (55 mmol), $Pd(PPh_3)_2Cl_2$ (2.5 mmol) and $K_2CO_3$ (160 mmol) was combined in a flask and refluxed in a mixture of ethylene glycol dimethyl ether (50 mL) and water (40 mL) under an Argon atmosphere for 18h. The dark mixture was diluted with water (200 mL) and extracted with ethyl acetate. The organic phase was dried over $Na_2CO_3$, filtered and evaporated to yield a brown oil, which was triturated with water. The crude product precipitated and was removed by filtration and re-crystallized from MeOH.
Yield: 96%
1H NMR ($D_6$-DMSO): 2.35 (s, 3H); 7.32-8.20 (8H).

2-Methyl-5-nitro-biphenyl (48 mmol) was suspended in pyridine (100 mL) and water (150 mL), and KMnO4 (239 mmol) was added portion wise over a period of one hour. The mixture was refluxed for 5h. The reaction mixture was cooled, and $MnO_2$ was filtered off. HCl (conc.) was added to the filtrate until the product precipitated, the product was removed by filtration, washed with water and dried *in vacuo*.
Yield: 85%
1H NMR ($D_6$-DMSO): 7.37-8.39 (8H).

5-Nitro-biphenyl-2-carboxylic acid (25 mmol) was suspended in 1,2-dichloroethane (100 mL) and two drops of DMF was added followed by drop wise addition of oxalylchloride (2M solution in dichloromethane) (40 mmol). The mixture turned homogeneous and was stirred at ambient temperature for 1h. Evaporated to dryness, then re-dissolved in 1,2-dichloroethane (20 mL) and added to a suspension of 2-amino thiazole (25 mmol) and pyridine (30 mmol) in 1,2-dichloroethane (50 mL). Stirred over night at ambient temperature, then the mixture was evaporated to dryness and triturated with water. The crude product was filtered off and boiled in MeOH. The white solid was filtered off, and dried *in vacuo*.
Yield: 73%
1H NMR ($D_6$-DMSO): 7.27 (d, 1H); 7.32-7.53 (7H); 7.92 (d, 1H); 8.23 (m, 1H); 8.32 (m, 1H); 12.75 (s, 1H).

5-Nitro-biphenyl-2-carboxylic acid thiazol-2-ylamide (18.1 mmol) was suspended in MeOH (50 mL) and glacial acetic acid (10 mL), and Zn (s) (50 mmol) was added. The mixture turned homogenous after a few minutes, and stirring was continued for 24h. The reaction mixture was filtered and evaporated to dryness, then water (200 mL) was added. The crude product was removed by filtration, washed with water and dried.
Yield: 89% (53% overall)
1H NMR ($D_6$-DMSO): 5.71 (s, 2H); 6.55 (m, 2H); 7.12 (d, 1H); 7.20-7.41 (7H); 11.52 (br s, 1H).

6-Amino-biphenyl-3-carboxylic acid thiazol-2-ylamide:

4-Amino-benzoic acid (100 mmol) was dissolved in DMF (100 mL) and *N*-bromo succinimide (100 mmol) was added portion wise. The orange reaction mixture was stirred over night at ambient temperature, then poured into water. The product was collected by filtration and re-crystallized from MeOH.
Yield: 65%
1H NMR ($D_6$-DMSO): 6.09 (s, 2H); 6.81 (d, 1H); 7.13 (dd, 1H); 7.89 (d, 1H); 12.37 (br, 1H).

4-Amino-3-bromo-benzoic acid (65 mmol), phenyl boronic acid (70 mmol), $Pd(PPh_3)_2Cl_2$ (3.2 mmol) and $K_2CO_3$ (140 mmol) was combined in a flask and refluxed in a mixture of ethylene glycol dimethyl ether (75 mL) and water (75 mL) under an argon atmosphere for 18h.The organic solvent was removed *in vacuo* and pH was adjusted to 4. The crude product was removed by filtration, re-dissolved in ethyl acetate, and passed through a silica plug to remove any Pd-residues. The filtrate was evaporated to dryness and re-crystallized from ethyl acetate/heptane to give off-white crystals.
Yield: 47%
NMR ($D_6$-DMSO): 5.56 (s, 2H); 6.78 (d, 1H); 7.30-7.75 (8H); 12.09 (br, 1H).

6-Amino-biphenyl-3-carboxylic acid (19 mmol) was dissolved in DMF (10 mL) and 1,2-dichloroethane (80 mL). DIPEA (19 mmol), 1-(3-dimethylaminopropyl)-3-ethylcarbodiimide hydrochloride (19 mmol), 1-hydroxybenzothiazole (19 mmol) and 2-amino thiazole (19 mmol) was added, and the reaction mixture was stirred at 50 °C over night. The volume was reduced *in vacuo*, and water (60 mL) was added. The mixture was extracted with ethyl acetate, the organic phase was washed with $NH_4Cl$ (aq., sat.), dried over $MgSO_4$, filtered and evaporated. The crude product was purified by flash

chromatography on silica using gradient elution (ethyl acetate/heptane).
Yield: 26% (8% overall)
NMR ($D_6$-DMSO): 5.64 (s, 2H); 6.82 (d, 1H); 7.19 (d, 1H); 7.34-7.53 (6H); 7.83 (d, 1H); 7.88 (d, 1H); 12.21 (s, 1H).

2,3-Dihydro-1*H*-indole-5-carboxylic acid thiazol-2-ylamide:

2,3-Dihydro-1*H*-indole-5-carboxylic acid (12.4 mmol) was dissolved in DMF (5 mL) and 1,2-dichloroethane (40 mL). DIPEA (12.4 mmol), 1-(3-dimethylaminopropyl)-3-ethyl-carbodiimide hydrochloride (12.4 mmol), 1-hydroxybenzotriazole (12.4 mmol) and 2-aminothiazole (12.4 mmol) was added. Stirred at 50 °C for 48h. HCl (2M) (12.4 mmol) was added to the reaction mixture followed by water (30 mL). This gave a heavy precipitation which was removed by filtration. The solid was washed with water and 1,2-dichloroethane and dried.
Yield: 56%
1H NMR ($D_6$-DMSO): 6.60 (s, 1H); 7.26 (d, 1H); 7.44-7.54 (2H); 7.56 (d, 1H); 7.86 (d, 1H); 8.45 (s, 1H); 11.51 (br s, 1H).
1*H*-Indole-5-carboxylic acid thiazol-2-ylamide (7 mmol) was dissolved in glacial acetic acid (40 mL) and sodium cyanoborohydride (14 mmol) was added portion wise. After stirring at room temperature for 18h the reaction was incomplete and another 14 mmol of sodium cyanoborohydride was added. Stirred at room temperature for 24 h. The reaction mixture was poured into ice water (200 mL). The pH of the mixture was adjusted to 10 with NaOH (conc.), and the aqueous phase was extracted with ethyl acetate. The organic extracts were combined, dried over $MgSO_4$, filtered and evaporated. The crude product was purified by flash chromatography on silica using a gradient of ethyl acetate/heptane (10:90 to 70:30) as eluent.
Yield: 32% (18% overall)
1H NMR ($D_6$-DMSO): 2.98 (t, 2H); 3.57 (t, 2H); 6.35 (br s, 1H); 6.48 (d, 1H); 7.17 (d, 1H); 7.49 (d, 1H); 7.75-7.82 (3H); 12.02 (br s, 1H).

1,2,3,4-Tetrahydro-quinoline-6-carboxylic acid thiazol-2-ylamide:

1,2,3,4-Tetrahydro-quinoline (100 mmol) was dissolved in 1,2-dichloroethane (100 mL) and acetic anhydride (102 mmol) was added. Stirred at room temperature for 4h, then the solvent was removed by evaporation and the residue was dissolved in ethyl acetate and water. The aqueous phase was neutralized with NaOH (2M), the organics were separated, dried over $MgSO_4$, filtered and evaporated.
The crude was used directly in the next reaction
Crude 1-(3,4-dihydro-2*H*-quinolin-1-yl)-ethanone was dissolved in DMF (60 mL), and *N*-bromo succinimide (100 mmol) was added portion wise. Stirred at room temperature for 3h, then the reaction mixture was poured into water (150 mL) and extracted with ethyl acetate. The organic phase was washed with $NH_4Cl$ (sat.), dried over $MgSO_4$, filtered and evaporated.
Yield: 96%
1H NMR ($D_6$-DMSO): 1.85 (m, 2H); 2.16 (s, 3H); 2.70 (m, 2H); 3.66 (m, 2H); 7.25-7.60 (3H).
1-(6-Bromo-3,4-dihydro-2*H*-quinolin-1-yl)-ethanone (96 mmol) was dissolved in DMF (60 mL) and CuCN (200 mmol) was added. The reaction mixture was refluxed for 18h, then cooled and poured into water (400 mL). Aq. $NH_3$ (sat.) (100 mL) was added, and the mixture was stirred vigorously until it had turned blue. The product had precipitated and was removed by filtration, washed with water and dried.
Yield: 53%
1H NMR ($D_6$-DMSO): 1.78 (m, 2H); 2.67 (m, 2H); 3.23 (m, 2H); 6.43 (d, 1H); 7.41-7.47 (2H).
m/z: 201 (MH+)
1-Acetyl-1,2,3,4-tetrahydro-quinoline-6-carbonitrile (50 mmol) was refluxed in HCl (8M) (150 mL) for 18h. The mixture was cooled and pH adjusted to approx. 3 with NaOH. The product precipitated and was removed by filtration, washed with water and dried.
Yield: 61%
1H NMR ($D_6$-DMSO): 1.79 (m, 2H); 2.68 (m, 2H); 3.23 (m, 2H); 5.45 (br, 1H); 6.48 (d, 1H); 7.45-7.50 (2H).
1,2,3,4-Tetrahydro-quinoline-6-carboxylic acid (17 mmol) was dissolved in DMF (5mL) and 1,2-dichloroethane (15 mL) was added, followed by DIPEA (17 mmoL), 1-(3-dimethylaminopropyl)-3-ethylcarbodiimide hydrochloride (17 mmol), 1-hydroxybenzotriazole (17 mmol) and 2-aminothiazole (17 mmol). Stirred at 60 °C for 96h, then HCl (17 mmol) and water (20 mL) was added. The organic solvent was removed by evaporation, and the aqueous phase was extracted with ethyl acetate. The organics were dried over $MgSO_4$, filtered and evaporated. The crude product was purified by flash chromatography on silica with gradient (heptane/ethyl acetate) elution.
Yield: 10% (3% overall)
1H NMR ($D_6$-DMSO): 1.80 (m, 2H); 2.70 (m, 2H); 3.24 (m, 2H); 6.46 (d, 1H); 6.56 (s, 1H); 7.16 (d, 1H); 7.49 (d, 1H); 7.65-7.72 (2H).

3,4-Dihydro-2*H*-benzo[1,4]oxazine-7-carboxylic acid thiazol-2-ylamide:

3-Hydroxy-4-nitro-benzoic acid (100 mmol) was refluxed in HCl (6M) (90 mL) and MeOH (160 mL) over night, the mixture was cooled and poured into water (500 mL). The product was removed by filtration, washed with water and dried.
The crude product was used directly in the next reaction.

Crude 3-hydroxy-4-nitro-benzoic acid methyl ester and triphenylphosphine (100 mmol) was dissolved in dry THF (120 mL) and cooled to 0 °C. Di-ethyl-azodicarboxylate (110 mmol) was added followed by 2-chloroethanol (110 mmol). The reaction mixture was allowed to come to room temperature and was stirred at this temperature over night. The solvent was removed by evaporation and the residue was re-dissolved in MeOH (80 mL), and water (20 mL). The product precipitated and was removed by filtration, washed with water and dried.
Yield : 60%
1H NMR ($D_6$-DMSO): 3.91 (s, 3H); 3.96 (t, 2H); 4.54 (t, 2H); 7.70 (dd, 1H); 7.81 (d, 1H); 8.01 (d, 1H).

3-(2-Chloro-ethoxy)-4-nitro-benzoic acid methyl ester (58 mmol) was suspended in abs. EtOH (200 mL), and ethyl acetate (100 mL), glacial acetic acid (10 mL) and 10% Pd/C (1g) was added. The mixture was hydrogenated under 3 bar $H_2$ for 3 days. The reaction mixture was filtered and evaporated.
The crude product was used directly in the next reaction.

4-Amino-3-(2-chloro-ethoxy)-benzoic acid methyl ester (58 mmol) was dissolved in DMF (150 mL) and $K_2CO_3$ (60 mmol) was added. Stirred at 100°C for 4 days. The reaction mixture was poured into water (500 mL) and extracted with ethyl acetate. The organic phases were washed with $NH_4Cl$ (sat.), dried over $MgSO_4$, filtered and evaporated. The crude product was used directly in the next reaction.

3,4-Dihydro-2*H*-benzo[1,4]oxazine-7-carboxylic acid methyl ester (52 mmol) was dissolved in MeOH (20 mL) and NaOH (2M) (20 mL) and stirred at 60 °C for 48h. The organic solvent was removed by evaporation, and the aqueous phase acidified with HCl (4M). The organic products separated as an oil, and were extracted with ethyl acetate. The organic extract was dried over $MgSO_4$, filtered and evaporated. The crude was purified by flash chromatography on silica with gradient elution (heptane/ethyl acetate).
Yield: 31 %
1H NMR ($D_6$-DMSO): 3.33 (t, 2H); 4.10 (t, 2H); 6.51 (d, 1H); 7.16 (d, 1H); 7.30 (dd, 1H).

3,4-Dihydro-2*H*-benzo[1,4]oxazine-7-carboxylic acid (16 mmol) was dissolved in DMF (5mL) and 1,2-dichloroethane (15 mL) was added, followed by DIPEA (16 mmol), 1-(3-dimethylaminopropyl)-3-ethylcarbodiimide hydrochloride (16 mmol), 1-hydroxybenzotriazole (16 mmol) and 2-aminothiazole (16 mmol). Stirred at 60 °C for 96h, then HCl (16 mmol) and water (20 mL) was added. The organic solvent was removed by evaporation, and the aqueous phase was extracted with ethyl acetate. The product precipitated and was filtered, washed with water and dried. The crude product was re-crystallized from EtOH/water.
Yield: 40% (6% overall)
1H NMR ($D_6$-DMSO): 3.58 (t, 2H); 4.35 (t, 2H); 6.82 (d, 1H); 6.89 (br s, 1H); 7.41 (d, 1H); 7.69 (d, 1H); 7.72 (d, 1H); 7.76 (dd, 1H).

*N*-Thiazol-2-yl-terephthalamic acid:

2-Amino thiazole (21 mmol) and pyridine (21 mmol) were suspended in 1,2-dichloroethane (100 mL) and a suspension of methyl 4-chlorocarbonylbenzoate (25 mmol) in 1,2-dichloroethane (300 mL) was added portion wise. Stirred at 50 °C over night, then the solvent was removed under reduced pressure. The remaining solids were re-suspended in ethyl acetate and $NaHCO_3$ (sat.) and then filtered, washed with ethyl acetate and dried *in vacuo.*
Yield: 79 %
1H NMR ($D_6$-DMSO): 3.90 (s, 3H); 7.31 (d, 1H); 7.59 (d, 1H); 8.09 (d, 2H); 8,20 (d, 2H); 12.83 (br s, 1H).

*N*-Thiazol-2-yl-terephthalamic acid methyl ester (16 mmol) was dissolved in THF (100 mL) and NaOH (2M, aq.) (100 mL) and stirred at room temperature for 4h. The organic solvent was removed by evaporation under reduced pressure and the aqueous phase acidified with HCl (2M, aq.). The precipitated product was removed by filtration, washed with water and dried *in vacuo*.
Yield: 71% (56% overall)
1H NMR ($D_6$-DMSO): 7.33 (d, 1H); 7.59 (d, 1H); 8.07 (d, 2H); 8.19 (d, 2H); 13.08 (br, 1H).

4-Amino-N-(5-chloro-thiazol-2-yl)-benzamide:

2-Amino-5-chlorothiazole hydrochloride (100 mmol) was suspended in 1,2-dichloroethane (60 mL) and pyridine (4.7 mmol) was added. A suspension of 4-nitro benzoic acid chloride (43.8 mmol) in 1,2-dichloroethane (150 mL). The reaction mixture was stirred at 50°C over night. The reaction mixture was cooled to room temperature ant washed with

$NaHCO_3$ (sat) (100 mL), water (100 mL) and NaCl (100 mL). The solvent was removed by evaporation under reduced pressure. The crude product was recrystallised form EtOH.
Yield: 29%
$^1$H-NMR ($D_6$-DMSO): 7.65 (s, 1H); 8.3 (d, 2H); 8.4 (d, 2H); 13.25 (br, 1H).

N-(5-Chloro-thiazol-2-yl)-4-nitro-benzamide (12.9 mmol) was suspended in MeOH (40 mL) and glacial acetic acid (40 mL), and Zn (s) (51.5 mmol) was added. The mixture was stirred at 70°C for 48h. The reaction mixture was evaporated to dryness. Water (200 mL) and concentrated hydrochloric acid (5mL) were added. The mixture was filtered and the solvent was removed by evaporation. The crude product was recrystallised from EtOH/water.
Yield: 54%
$^1$H-NMR ($D_6$-DMSO): 6.04 (s, 2H); 6.6 (d, 2H); 7.5 (s, 1H); 7.85 (d, 2H); 12.4 (br).

4-Amino-3-methoxymethyl-N-thiazol-2-yl-benzamide:

3-Bromomethyl-4-nitro-benzoic acid ethyl ester (3.4 mmol) (Can be prepared according to literature procedures: Damen et al.; Bioorg.Med.Chem.; EN; 10; 1; 2002; 71 - 78) was suspended in MeOH (20 mL). NaOMe (5.4 M in MeOH, 0.77 mL) was added slowly at 0°C. The reaction mixture was stirred 1h at 0°C, then overnight at room temperature. The solvent was removed under reduced pressure. Ethyl acetate (100 mL) was added and the organic phase was washed with water (50 mL) and brine (50 mL). The organic phase was dried over $MgSO_4$ filtered and evaporated.
Yield: 71%
$^1$H-NMR ($D_6$-DMSO): 3.4 (s, 3H); 3.9 (s, 3H); 4.8 (s, 2H); 8.1 (d, 1H); 8.2 (d, 1H); 8.3 (s, 1H).

3-Methoxymethyl-4-nitro-benzoic acid methyl ester (2.6 mmol) was dissolved in MeOH (10 mL) and NaOH (2M, 10 mL) was added. The reaction mixture was stirred at 25°C overnight. pH was adjusted with HCl to pH = 3. 3-Methoxymethyl-4-nitrobenzoic acid was filtered off and washed with water.
Yield: 45%
$^1$H-NMR ($D_6$-DMSO): 3.4 (s, 3H); 4.8 (s, 2H); 8.05 (d, 1H); 8.51 (d, 1H); 8.75 (s, 1H).

3-Methoxymethyl-4-nitro-benzoic acid (1.6 mmol) was suspended in 1,2-dichloroethane (10 mL) and dimethylformamide (DMF) (0.1 mL) under an argon atmosphere. Oxalylchloride (2M in dichloromethane, 1.3 mL) was added slowly to the stirred suspension. After stirring at room temperature for 1h, the solvent was removed by evaporation under reduced pressure, and the reaction mixture was re-dissolved in 1,2-dichloroethane (7 mL). A suspension of 2-amino thiazole (1.3 mmol) and pyridine (0.13 mmol) in 1,2-dichloroethane (5 mL) was added portion wise. The reaction mixture was stirred at 50 °C for 48h. The solvent was removed under reduced pressure. The crude was re-crystallized from EtOH/water. The product contained small amounts of starting material. Used without further purification.
LC/MS (m/z) 294 (MH+); RT = 2.4 (method A); purity (UV, ELSD): 74%; 92%.

3-Methoxymethyl-4-nitro-N-thiazol-2-yl-benzamide (0.88 mmol) was suspended in abs. EtOH (9 mL). Ethyl acetate (4.5 mL) and glacial acetic acid (1.5 mL) was added followed by 10% Pd/C (0.5 g). The mixture was hydrogenated for 72h at 3 bar $H_2$. The hydrogenation mixture was filtered, and the solvent was removed under reduced pressure. The crude product was added NaOH (1M) and extracted with ethyl acetate. The organic phase was washed with water and the solvent was removed under reduced pressure. Purified by preparative HPLC-MS.
Yield: 17%
$^1$H-NMR ($D_6$-DMSO): 3.3 (s, 3H); 4.4 (s, 2H); 6.7 (d, 1H); 7.2 (d, 1H); 7.5 (d, 1H); 7.85 (d, 1H); 7.9 (s, 1H), 12.1 (br, 1H).

**Preparation of the compounds of the invention**

**Examples**

**1**: 4-Butyrylamino-*N*-thiazol-2-yl-benzamide

200 μL of a 0.43 M stock solution of butanoic acid in DMF containing 6 mmol DIPEA per mmol butanoic acid was mixed with 100 μL of a 0.86 M stock solution of *O*-(7-azabenzotriazole-1-yl)-*N*,*N*,*N*',*N*'-tetramethyluronium hexafluorophosphate (HATU) in DMF. The mixture was allowed to react for 10 min. at ambient temperature, then 100 μL of a 0.43 M stock solution of 4-amino-*N*-thiazol-2-yl-benzamide in DMF was added. The resulting mixture was incubated for 18h at ambient temperature. Purification was performed by preparative HPLC-MS.
1H NMR ($D_6$-DMSO): 0.92 (t, 3H); 1.63 (m, 2H); 2.34 (t, 2H); 7.26 (d, 1H); 7.55 (d, 1H); 7.74 (d, 2H); 8.06 (d, 2H); 10.20 (s, 1H); 12.47 (br s, 1H).
LC/MS (m/z) 290 ($MH^+$); RT = 2.12 (method B); purity (UV, ELSD): 98%, 100%.

The following compounds were prepared analogously:

**2:** *rac*-3-Methoxy-4-(3-methyl-4-oxo-pentanoylamino)-*N*-thiazol-2-yl-benzamide

LC/MS (m/z) 362 (MH+); RT = 2,06 (method A); purity (UV, ELSD): 75%; 95%.
**3:** *rac*-4-(3-Methyl-pentanoylamino)-*N*-thiazol-2-yl-benzamide
LC/MS (m/z) 318 (MH+); RT = 2,45 (method A); purity (UV, ELSD): 100%; 94%.
**4**: 4-Hexanoylamino-3-methyl-*N*-thiazol-2-yl-benzamide
LC/MS (m/z) 332 (MH+); RT = 2,56 (method A); purity (UV, ELSD): 100%; 100%.
**5**: 4-(2-Cycloheptyl-acetylamino)-*N*-thiazol-2-yl-benzamide
LC/MS (m/z) 358 (MH+); RT = 2,90 (method A); purity (UV, ELSD): 100%; 93%.
**6**: *rac*-3-Methoxy-4-(3-methyl-pentanoylamino)-*N*-thiazol-2-yl-benzamide
LC/MS (m/z) 348 (MH+); RT = 2,65 (method A); purity (UV, ELSD): 94%; 98%.
**7**: 4-(2-Cycloheptyl-acetylamino)-3-methoxy-*N*-thiazol-2-yl-benzamide
LC/MS (m/z) 389 (MH+); RT = 3,17 (method A); purity (UV, ELSD): 80%; 83%.
**8:** *rac*-4-[2-(2-Oxo-cyclopentyl)-acetylamino]-*N*-thiazol-2-yl-benzamide
LC/MS (m/z) 344 (MH+); RT = 2,08 (method A); purity (UV, ELSD): 84%; 91%.
**9:** 4-Hexanoylamino-3-methoxy-*N*-thiazol-2-yl-benzamide
LC/MS (m/z) 348 (MH+); RT = 2,77 (method A); purity (UV, ELSD): 80%; 100%.
**10:** 3-Methyl-4-(4-phenyl-butyrylamino)-*N*-thiazol-2-yl-benzamide
LC/MS (m/z) 380 (MH+); RT = 2,70 (method A); purity (UV, ELSD): 98%; 100%.
**11:** 4-(2-Cyclohexyl-acetylamino)-*N*-thiazol-2-yl-benzamide
LC/MS (m/z) 344 (MH+); RT = 2,71 (method A); purity (UV, ELSD): 100%; 91%.
**12:** *rac*-4-(2-Bicyclo[2.2.1]hept-2-yl-acetylamino)-*N*-thiazol-2-yl-benzamide
LC/MS (m/z) 356 (MH+); RT = 2,78 (method A); purity (UV, ELSD): 97%; 93%.
**13:** 4-(3,3-Dimethyl-butyrylamino)-*N*-(4,5-dimethyl-thiazol-2-yl)-benzamide
LC/MS (m/z) 346 (MH+); RT = 2,59 (method A); purity (UV, ELSD): 99%; 100%.
**14:** 4-(2-Adamantan-1-yl-acetylamino)-*N*-thiazol-2-yl-benzamide
LC/MS (m/z) 397 (MH+); RT = 3,12 (method A); purity (UV, ELSD): 80%; 96%.
**15:** 4-(3-Benzo[1,3]dioxol-5-yl-propionylamino)-3-methyl-*N*-thiazol-2-yl-benzamide
LC/MS (m/z) 410 (MH+); RT = 2,44 (method A); purity (UV, ELSD): 87%; 98%.
**16:** 4-(3-Hydroxy-3-methyl-butyrylamino)-3-methoxy-*N*-thiazol-2-yl-benzamide
LC/MS (m/z) 350 (MH+); RT = 1,90 (method A); purity (UV, ELSD): 96%; 88%.
**17:** 4-(4-Fluoro-benzoylamino)-3-methoxy-*N*-thiazol-2-yl-benzamide
LC/MS (m/z) 372 (MH+); RT = 2,66 (method A); purity (UV, ELSD): 84%; 95%.
**18:** 4-Benzoylamino-*N*-thiazol-2-yl-benzamide
LC/MS (m/z) 324 (MH+); RT = 2,33 (method A); purity (UV, ELSD): 97%; 99%.
**19**: Thiophene-3-carboxylic acid [4-(thiazol-2-ylcarbamoyl)-phenyl]-amide
LC/MS (m/z) 330 (MH+); RT = 2,33 (method A); purity (UV, ELSD): 100%; 99%.
**20:** *N*-Thiazol-2-yl-4-(2-*o*-tolyl-acetylamino)-benzamide
LC/MS (m/z) 352 (MH+); RT = 2,50 (method A); purity (UV, ELSD): 99%; 91%.
**21:** *N*-Thiazol-2-yl-4-(2-thiophen-3-yl-acetylamino)-benzamide
LC/MS (m/z) 344 (MH+); RT = 2,28 (method A); purity (UV, ELSD): 94%; 85%.
**22:** 4-(2-Cyclopentyl-acetylamino)-3-methyl-*N*-thiazol-2-yl-benzamide
200 μL of a 0.2 M stock solution of 4-amino-3-methyl-*N*-thiazol-2-yl-benzamide in 1,2-dichloroethane/DMF, containing 1.2 mmol pyridine per mmol 4-amino-3-methyl-*N*-thiazol-2-yl-benzamide, was added 0.05 mmol of cyclopentyl-acetyl chloride. The reaction mixture was incubated at ambient temperature for 2h. Purification was performed by preparative HPLC-MS.
1H NMR ($D_6$-DMSO): 1.23 (m, 2H); 1.53 (m, 2H); 1.63 (m, 2H); 1.78 (m, 2H); 2.25 (m, 1H); 2.40 (d, 2H); 2.90 (s, 3H); 7.27 (d, 1H); 7.56 (d, 1H); 7.68 (d, 1H); 7.90 (dd, 1H); 7.98 (d, 1H); 9.38 (s, 1H); 12.49 (br).
LC/MS (m/z) 344 ($MH^+$); RT = 2.66 (method A); purity (UV, ELSD): 95%, 98%.

The following compounds were prepared analogously:

**23:** 4-(3,3-Dimethyl-butyrylamino)-*N*-thiazol-2-yl-benzamide
LC/MS (m/z) 318 (MH+); RT = 2,54 (method A); purity (UV, ELSD): 99%; 99%.

**24:** 4-(3,3-Dimethyl-butyrylamino)-3-methyl-*N*-thiazol-2-yl-benzamide
LC/MS (m/z) 332 (MH+); RT = 2,44 (method A); purity (UV, ELSD): 97%; 100%.

**25:** 4-(3,3-Dimethyl-butyrylamino)-2-methoxy-*N*-thiazol-2-yl-benzamide
LC/MS (m/z) 348 (MH+); RT = 2,63 (method A); purity (UV, ELSD): 98%; 100%.

**26:** 3-Chloro-4-(3-methyl-butyrylamino)-*N*-thiazol-2-yl-benzamide
LC/MS (m/z) 339 (MH+); RT = 2,60 (method A); purity (UV, ELSD): 98%; 100%.

**27:** 3-Bromo-4-(3,3-dimethyl-butyrylamino)-*N*-thiazol-2-yl-benzamide
LC/MS (m/z) 397 (MH+); RT = 2,85 (method A); purity (UV, ELSD): 100%; 100%.

**28**: 4-(3-Methyl-butyrylamino)-*N*-thiazol-2-yl-benzamide
LC/MS (m/z) 304 (MH+); RT = 2,22 (method A); purity (UV, ELSD): 100%; 100%.

**29:** 3-Bromo-4-(3-methyl-butyrylamino)-*N*-thiazol-2-yl-benzamide
LC/MS (m/z) 383 (MH+); RT = 2,64 (method A); purity (UV, ELSD): 88%; 96%.

**30:** 4-(2-Cyclopentyl-acetylamino)-*N*-thiazol-2-yl-benzamide
LC/MS (m/z) 330 (MH+); RT = 2,42 (method A); purity (UV, ELSD): 99%; 100%.

**31:** 3-Methyl-4-(3-methyl-butyrylamino)-*N*-thiazol-2-yl-benzamide
LC/MS (m/z) 318 (MH+); RT = 2,24 (method A); purity (UV, ELSD): 100%; 97%.

**32:** 3-Chloro-4-(cyclopentanecarbonyl-amino)-*N*-thiazol-2-yl-benzamide
LC/MS (m/z) 351 (MH+); RT = 2,73 (method A); purity (UV, ELSD): 100%; 100%.

**33:** 3-Chloro-4-(2-methyl-benzoylamino)-*N*-thiazol-2-yl-benzamide
LC/MS (m/z) 373 (MH+); RT = 2,81 (method A); purity (UV, ELSD): 89%; 100%.

**34:** 3-Bromo-4-(cyclopentanecarbonyl-amino)-*N*-thiazol-2-yl-benzamide
LC/MS (m/z) 395 (MH+); RT = 2,79 (method A); purity (UV, ELSD): 99%; 99%.

**35:** 4-(Cyclopentanecarbonyl-amino)-*N*-thiazol-2-yl-benzamide
LC/MS (m/z) 316 (MH+); RT = 2,32 (method A); purity (UV, ELSD): 97%; 100%.

**36:** 4-(Cyclopentanecarbonyl-amino)-3-methyl-*N*-thiazol-2-yl-benzamide
LC/MS (m/z) 330 (MH+); RT = 2,34 (method A); purity (UV, ELSD): 100%; 97%.

**37:** Cycloheptanecarboxylic acid [2-bromo-4-(thiazol-2-ylcarbamoyl)-phenyl]-amide
LC/MS (m/z) 423 (MH+); RT = 3,20 (method A); purity (UV; ELSD): 90%; 99%.

**38:** 4-Isobutyrylamino-2-methoxy-*N*-thiazol-2-yl-benzamide
LC/MS (m/z) 320 (MH+); RT = 2,20 (method A); purity (UV, ELSD): 100%; 97%.

**39:** 8-(3,3-Dimethyl-butyrylamino)-2,3-dihydro-benzo[1,4]dioxine-5-carboxylic acid thiazol-2-ylamide
LC/MS (m/z) 376 (MH+); RT = 2,68 (method A); purity (UV, ELSD): 99%; 100%.

**40:** 3-Bromo-4-butyrylamino-*N*-thiazol-2-yl-benzamide
LC/MS (m/z) 369 (MH+); RT = 2,43 (method A); purity (UV, ELSD): 89%; 100%.

**41:** 2-Methoxy-4-(3-methyl-butyrylamino)-*N*-thiazol-2-yl-benzamide
LC/MS (m/z) 334 (MH+); RT = 2,44 (method A); purity (UV, ELSD): 100%; 95%.

**42:** Cycloheptanecarboxylic acid [4-(thiazol-2-ylcarbamoyl)-phenyl]-amide
LC/MS (m/z) 344 (MH+); RT = 2,71 (method A); purity (UV, ELSD): 98%; 100%.

**43:** *rac*-2-Methoxy-4-(2-methyl-butyrylamino)-*N*-thiazol-2-yl-benzamide
LC/MS (m/z) 334 (MH+); RT = 2,40 (method A); purity (UV, ELSD): 98%; 98%.

**44:** 4-(Cyclopentanecarbonyl-amino)-2-methoxy-*N*-thiazol-2-yl-benzamide
LC/MS (m/z) 346 (MH+); RT = 2,52 (method A); purity (UV, ELSD): 100%; 95%.

**45:** 3-Bromo-4-(2-methyl-benzoylamino)-*N*-thiazol-2-yl-benzamide

LC/MS (m/z) 417 (MH+); RT = 2,86 (method A); purity (UV, ELSD): 96%; 100%.

**46:** 3-Chloro-4-(3,3-dimethyl-butyrylamino)-*N*-thiazol-2-yl-benzamide
LC/MS (m/z) 353 (MH+); RT = 2,82 (method A); purity (UV, ELSD): 95%; 97%.

**47**: 4-(2-Cyclopentyl-acetylamino)-2-propoxy-*N*-thiazol-2-yl-benzamide
LC/MS (m/z) 389 (MH+); RT = 3,32 (method A); purity (UV, ELSD): 87%; 100%.

**48**: 4-(3,3-Dimethyl-butyrylamino)-2-propoxy-*N*-thiazol-2-yl-benzamide
LC/MS (m/z) 376 (MH+); RT = 3,24 (method A); purity (UV, ELSD): 84%; 99%.

**49**: 4-(2-Cyclopentyl-acetylamino)-3-fluoro-*N*-thiazol-2-yl-benzamide
LC/MS (m/z) 348 (MH+); RT = 2,75 (method A); purity (UV, ELSD): 98%; 100%.

**50**: 4-(3-Methyl-butyrylamino)-2-propoxy-*N*-thiazol-2-yl-benzamide
LC/MS (m/z) 362 (MH+); RT = 3,02 (method A); purity (UV, ELSD): 87%; 100%.

**51:** 3-Fluoro-4-(3-methyl-butyrylamino)-*N*-thiazol-2-yl-benzamide
LC/MS (m/z) 322 (MH+); RT = 2,41 (method A); purity (UV, ELSD): 99%; 100%.

**52:** 4-Butyrylamino-3-fluoro-*N*-thiazol-2-yl-benzamide
LC/MS (m/z) 308 (MH+); RT = 2,19 (method A); purity (UV, ELSD): 73%; 84%.

**53:** 4-Butyrylamino-2-propoxy-*N*-thiazol-2-yl-benzamide
LC/MS (m/z) 348 (MH+); RT = 2,82 (method A); purity (UV, ELSD): 97%; 100%.

**54:** 3-Fluoro-4-(2-methyl-benzoylamino)-*N*-thiazol-2-yl-benzamide
LC/MS (m/z) 356 (MH+); RT = 2,57 (method A); purity (UV, ELSD): 95%; 100%.

**55:** Cycloheptanecarboxylic acid [2-fluoro-4-(thiazol-2-ylcarbamoyl)-phenyl]-amide
LC/MS (m/z) 362 (MH+); RT = 2,90 (method A); purity (UV, ELSD): 88%; 99%.

**56:** 4-(Cyclopentanecarbonyl-amino)-3-fluoro-*N*-thiazol-2-yl-benzamide
LC/MS (m/z) 334 (MH+); RT = 2,53 (method A); purity (UV, ELSD): 100%; 100%.

**57:** 4-(3,3-Dimethyl-butyrylamino)-2-(2-methoxy-ethoxy)-*N*-thiazol-2-yl-benzamide
LC/MS (m/z) 392 (MH+); RT = 2,85 (method A); purity (UV, ELSD): 97%; 100%.

**58:** 3-Fluoro-4-(3-methyl-benzoylamino)-*N*-thiazol-2-yl-benzamide
LC/MS (m/z) 356 (MH+); RT = 2,69 (method A); purity (UV, ELSD): 94%; 100%.

**59**: *rac*-3-Fluoro-*N*-thiazol-2-yl-4-(3,5,5-trimethyl-hexanoylamino)-benzamide
LC/MS (m/z) 378 (MH+); RT = 3,25 (method A); purity (UV, ELSD): 99%; 100%.

**60**: 4-(2-Cyclopentyl-acetylamino)-2-(2-methoxy-ethoxy)-*N*-thiazol-2-yl-benzamide
LC/MS (m/z) 405 (MH+); RT = 2,97 (method A); purity (UV, ELSD): 95%; 100%.

**61:** 4-(2-Methyl-benzoyl)-3,4-dihydro-2H-benzo[1,4]oxazine-7-carboxylic acid thiazol-2-ylamide
LC/MS (m/z) 380 (MH+); RT = 2,65 (method A); purity (UV, ELSD): 96%; 100%.

**62:** 4-(3,3-Dimethyl-butyrylamino)-3-fluoro-*N*-thiazol-2-yl-benzamide
LC/MS (m/z) 336 (MH+); RT = 2,74 (method A); purity (UV, ELSD): 94%; 100%.

**63:** 4-(3,3-Dimethyl-butyrylamino)-2-methyl-*N*-thiazol-2-yl-benzamide
LC/MS (m/z) 332 (MH+); RT = 2,66 (method A); purity (UV, ELSD): 94%; 87%.

**64:** 5-Chloro-4-(3,3-dimethyl-butyrylamino)-2-methoxy-*N*-thiazol-2-yl-benzamide
LC/MS (m/z) 383 (MH+); RT= 3,14 (method A); purity (UV, ELSD): 99%; 100%.

**65:** 4-(3,3-Dimethyl-butyrylamino)-*N*-(5-methyl-thiazol-2-yl)-benzamide
LC/MS (m/z) 332 (MH+); RT = 2,54 (method A); purity (UV, ELSD): 98%; 100%.

**66**: 5-Chloro-2-methoxy-4-(3-methyl-butyrylamino)-*N*-thiazol-2-yl-benzamide
LC/MS (m/z) 369 (MH+); RT = 2,93 (method A); purity (UV, ELSD): 97%; 100%.

**67**: 4-(2-methyl-benzoylamino)-*N*-(5-methyl-thiazol-2-yl)-benzamide
LC/MS (m/z) 352 (MH+); RT = 2,53 (method A); purity (UV, ELSD): 95%; 100%.

**68**: 1-(3,3-Dimethyl-butyryl)-1,2,3,4-tetrahydro-quinoline-6-carboxylic acid thiazol-2-ylamide
LC/MS (m/z) 358 (MH+); RT = 2,80 (method A); purity (UV, ELSD): 97%; 95%.

**69**: 5-Chloro-2-methoxy-4-(2-methyl-benzoylamino)-*N*-thiazol-2-yl-benzamide
LC/MS (m/z) 403 (MH+); RT = 3,15 (method A); purity (UV, ELSD): 86%; 99%.

**70:** 1-(3-Methyl-butyryl)-1,2,3,4-tetrahydro-quinoline-6-carboxylic acid thiazol-2-ylamide
LC/MS (m/z) 344 (MH+); RT = 2,58 (method A); purity (UV, ELSD): 99%; 98%.

**71:** 1-(3,3-Dimethyl-butyryl)-2,3-dihydro-1*H*-indole-5-carboxylic acid thiazol-2-ylamide
LC/MS (m/z) 344 (MH+); RT = 2,79 (method A); purity (UV, ELSD): 99%; 97%.

**72:** 4-[(3,3-Dimethyl-butyryl)-methyl-amino]-*N*-thiazol-2-yl-benzamide
LC/MS (m/z) 332 (MH+); RT = 2,62 (method A); purity (UV, ELSD): 92%; 92%.

**73:** 4-[(2-Cyclopentyl-acetyl)-propyl-amino]-*N*-thiazol-2-yl-benzamide
LC/MS (m/z) 373 (MH+); RT = 2,99 (method B); purity (UV, ELSD): 97%; 100%.

**74:** 2-(2-Methoxy-ethoxy)-4-(3-methyl-butyrylamino)-*N*-thiazol-2-yl-benzamide
LC/MS (m/z) 378 (MH+); RT = 2,66 (method A); purity (UV, ELSD): 81%; 99%.

**75:** *rac*-2-Propoxy-*N*-thiazol-2-yl-4-(3,5,5-trimethyl-hexanoylamino)-benzamide
LC/MS (m/z) 419 (MH+); RT = 3,80 (method A); purity (UV, ELSD): 83%; 98%.

**76:** *rac*-*N*-Thiazol-2-yl-4-(3,5,5-trimethyl-hexanoylamino)-benzamide
LC/MS (m/z) 360 (MH+); RT = 3,04 (method A); purity (UV, ELSD): 98%; 100%.

**77:** 4-(3-Cyclopentyl-propionylamino)-*N*-thiazol-2-yl-benzamide
LC/MS (m/z) 344 (MH+); RT = 2,79 (method A); purity (UV, ELSD): 99%; 100%.

**78:** 4-(2-Cyclopentyl-acetylamino)-3-methoxy-*N*-thiazol-2-yl-benzamide
LC/MS (m/z) 360 (MH+); RT = 2,79 (method A); purity (UV, ELSD): 100%; 100%.

**79:** Cycloheptanecarboxylic acid [2-methyl-4-(thiazol-2-ylcarbamoyl)-phenyl]-amide
LC/MS (m/z) 358 (MH+); RT = 2,72 (method A); purity (UV, ELSD): 100%; 93%.

**80:** 3-Methoxy-4-(3-phenyl-propionylamino)-*N*-thiazol-2-yl-benzamide
LC/MS (m/z) 382 (MH+); RT = 2,72 (method A); purity (UV, ELSD): 100%; 100%.

**81**: Cycloheptanecarboxylic acid [2-chloro-4-(thiazol-2-ylcarbamoyl)-phenyl]-amide
LC/MS (m/z) 379 (MH+); RT = 3,14 (method A); purity (UV, ELSD): 84%; 94%.

**82:** 4-[2-(3-Methoxy-phenyl)-acetylamino]-3-methyl-*N*-thiazol-2-yl-benzamide
LC/MS (m/z) 382 ($MH^+$); RT = 2,39 (method A); purity (UV, ELSD): 94%; 100%.

**83:** 3-Bromo-4-(2-cyclopentyl-acetylamino)-*N*-thiazol-2-yl-benzamide
LC/MS (m/z) 409 (MH+); RT = 2,95 (method A); purity (UV, ELSD): 97%; 100%.

**84:** 4-Butyrylamino-3-chloro-*N*-thiazol-2-yl-benzamide

LC/MS (m/z) 325 (MH+); RT = 2,39 (method A); purity (UV, ELSD): 98%; 100%.

**85:** 5-Chloro-4-(2-cyclopentyl-acetylamino)-2-methoxy-*N*-thiazol-2-yl-benzamide
LC/MS (m/z) 395 (MH+); RT = 3,27 (method A); purity (UV, ELSD): 99%; 100%.

**86:** 5-Chloro-4-(cyclopentanecarbonyl-amino)-2-methoxy-*N*-thiazol-2-yl-benzamide
LC/MS (m/z) 381 (MH+); RT = 3,09 (method A); purity (UV, ELSD): 99%; 100%.

**87:** 4-(Cyclohexanecarbonyl-amino)-2-methoxy-*N*-thiazol-2-yl-benzamide
LC/MS (m/z) 360 (MH+); RT = 2,73 (method A); purity (UV, ELSD): 99%; 100%.

**88:** 2-Methoxy-4-(4-methoxy-benzoylamino)-*N*-thiazol-2-yl-benzamide
LC/MS (m/z) 384 (MH+); RT = 2,55 (method A); purity (UV, ELSD): 99%; 100%.

**89:** 3-Methoxy-4-phenylacetylamino-*N*-thiazol-2-yl-benzamide
LC/MS (m/z) 368 (MH+); RT = 2,58 (method A); purity (UV, ELSD): 87%; 99%.

**90:** 3-Methyl-*N*-thiazol-2-yl-4-(2-thiophen-2-yl-acetylamino)-benzamide
LC/MS (m/z) 358 (MH+); RT = 2,33 (method A); purity (UV, ELSD): 88%; 100%.

**91:** 3-Chloro-4-(2-cyclopentyl-acetylamino)-*N*-thiazol-2-yl-benzamide
LC/MS (m/z) 365 (MH+); RT = 2,92 (method A); purity (UV, ELSD): 98%; 97%.

**92**: 4-(4-Methoxy-benzoylamino)-3-methyl-*N*-thiazol-2-yl-benzamide
LC/MS (m/z) 368 (MH+); RT = 2,35 (method A); purity (UV, ELSD): 98%; 100%.

**93:** 4-Butyrylamino-3-methyl-*N*-thiazol-2-yl-benzamide
LC/MS (m/z) 304 (MH+); RT = 2,02 (method A); purity (UV, ELSD): 100%; 100%.

**94:** 4-(2-Chloro-benzoylamino)-3-methyl-*N*-thiazol-2-yl-benzamide
LC/MS (m/z) 373 (MH+); RT = 2,43 (method A); purity (UV, ELSD): 95%; 100%.

**95:** 4-(2,5 Di-chloro-benzoylamino)-3-methyl-*N*-thiazol-2-yl-benzamide
LC/MS (m/z) 407 (MH+); RT = 2,75 (method A); purity (UV, ELSD): 96%; 100%.

**96:** 4-(2-Chloro-benzoylamino)-2-methoxy-*N*-thiazol-2-yl-benzamide
LC/MS (m/z) 389 (MH+); RT = 2,56 (method A); purity (UV, ELSD): 99%; 100%.

**97:** 4-(2-Ethyl-butyrylamino)-2-methoxy-*N*-thiazol-2-yl-benzamide
LC/MS (m/z) 348 (MH+); RT = 2,58 (method A); purity (UV, ELSD): 99%; 100%.

**98**: 2-Methoxy-4-(2-methyl-benzoylamino)-*N*-thiazol-2-yl-benzamide
LC/MS (m/z) 368 (MH+); RT = 2,52 (method A); purity (UV, ELSD): 83%; 100%.

**99**: 3-Methyl-4-(3-phenyl-propionylamino)-*N*-thiazol-2-yl-benzamide
LC/MS (m/z) 366 (MH+); RT = 2,50 (method A); purity (UV, ELSD): 98%; 100%.

**100**: 4-(3,3-Dimethyl-butyrylamino)-3-methoxy-*N*-thiazol-2-yl-benzamide
LC/MS (m/z) 348 (MH+); RT = 2,68 (method A); purity (UV, ELSD): 99%; 100%.

**101**: *rac*-3-Methyl-*N*-thiazol-2-yl-4-(3,5,5-trimethyl-hexanoylamino)-benzamide
LC/MS (m/z) 375 (MH+); RT = 3,06 (method A); purity (UV, ELSD): 100%; 100%.

**102**: *rac*-2,3-Dihydro-benzo[1,4]dioxine-2-carboxylic acid [2-methoxy-4-(thiazol-2-ylcarbamoyl)-phenyl]-amide
LC/MS (m/z) 412 (MH+); RT = 2,86 (method A); purity (UV, ELSD): 79%; 95%.

**103**: 4-(2,2-Dimethyl-propionylamino)-3-methoxy-*N*-thiazol-2-yl-benzamide
LC/MS (m/z) 334 (MH+); RT = 2,61 (method A); purity (UV, ELSD): 96%; 100%.

**104:** 2-Methoxy-4-(4-methyl-benzoylamino)-*N*-thiazol-2-yl-benzamide
LC/MS (m/z) 368 (MH+); RT = 2,74 (method A); purity (UV, ELSD): 98%; 99%.

**105:** Thiophene-2-carboxylic acid [3-methoxy-4-(thiazol-2-ylcarbamoyl)-phenyl]-amide
LC/MS (m/z) 360 (MH+); RT = 2,46 (method A); purity (UV, ELSD): 99%; 100%.

**106:** 4-(3-Methoxy-benzoylamino)-*N*-thiazol-2-yl-benzamide
LC/MS (m/z) 354 (MH+); RT = 2,40 (method A); purity (UV, ELSD): 98%; 100%.

**107:** 8-(2-Cyclopentyl-acetylamino)-2,3-dihydro-benzo[1,4]dioxine-5-carboxylic acid thiazol-2-ylamide
LC/MS (m/z) 388 (MH+); RT = 2,78 (method A); purity (UV, ELSD): 99%; 100%.

**108**: 6-(2-Cyclopentyl-acetylamino)-biphenyl-3-carboxylic acid thiazol-2-ylamide
LC/MS (m/z) 407 (MH+); RT = 3,08 (method A); purity (UV, ELSD): 94%; 100%.

**109**: 4-(2-Cyclopentyl-acetylamino)-3-(2-methoxy-ethoxy)-*N*-thiazol-2-yl-benzamide
LC/MS (m/z) 405 (MH+); RT = 2,74 (method A); purity (UV, ELSD): 86%; 96%.

**110**: 4-(3,3-Dimethyl-butyrylammo)-2-fluoro-*N*-thiazol-2-yl-benzamide
LC/MS (m/z) 336 (MH+); RT = 2,66 (method A); purity (UV, ELSD): 97%; 98%.

**111**: 2-Chloro-4-(2-methyl-benzoylamino)-*N*-thiazol-2-yl-benzamide
LC/MS (m/z) 373 (MH+); RT = 2,60 (method A); purity (UV, ELSD): 90%; 97%.

**112**: 4-(2-Fluoro-benzoylamino)-*N*-thiazol-2-yl-benzamide
LC/MS (m/z) 342 (MH+); RT = 2,34 (method A); purity (UV, ELSD): 97%; 100%.

**113**: 4-(2-Methoxy-benzoylamino)-*N*-thiazol-2-yl-benzamide
LC/MS (m/z) 354 (MH+); RT = 2,58 (method A); purity (UV, ELSD): 96%; 97%.

**114**: Benzo[*b*]thiophene-2-carboxylic acid [2-methyl-4-(thiazol-2-ylcarbamoyl)-phenyl]-amide
LC/MS (m/z) 394 (MH+); RT = 2,77 (method A); purity (UV, ELSD): 100%; 99%.

**115**: 5-(3,3-Dimethyl-butyrylamino)-biphenyl-2-carboxylic acid thiazol-2-ylamide
LC/MS (m/z) 395 (MH+); RT = 2,87 (method A); purity (UV, ELSD): 98%; 100%.

**116**: N-(5-Chloro-thiazol-2-yl)-4-(3,3-dimethyl-butyrylamino)-benzamide:
LC/MS (m/z) 352 (MH+); RT = 3,1 (method A); purity (UV, ELSD): 100%; 100%.

**117**: N-(5-Chloro-thiazol-2-yl)-4-(3-methyl-butyrylamino)-benzamide:
LC/MS (m/z) 338 (MH+); RT = 2,9 (method A); purity (UV, ELSD): 95%; 98%.

**118**: N-(5-Chloro-thiazol-2-yl)-4-(2-cyclopropyl-acetylamino)-benzamide:
LC/MS (m/z) 336 (MH+); RT = 2,7 (method A); purity (UV, ELSD): 88%; 97%.

**119:** 4-Butyrylamino-N-(5-chloro-thiazol-2-yl)-benzamide:
LC/MS (m/z) 324 (MH+); RT = 2,7 (method A); purity (UV, ELSD): 99%; 99%.

**120:** 4-Benzoylamino-*N*-(5-chloro-thiazol-2-yl)-benzamide:
LC/MS (m/z) 358 (MH+); RT = 2,9 (method A); purity (UV, ELSD): 97%; 99%.

**121:** 3-Fluoro-N-thiazol-2-yl-4-(4,4,4-trifluoro-3-methyl-butyrylamino)-benzamide:
LC/MS (m/z) 376 (MH+); RT = 2,7 (method A); purity (UV, ELSD): 97%; 72%.

**122:** 4-(3,3-Dimethyl-butyrylamino)-N-thiazol-2-yl-3-trifluoromethoxy-benzamide:
LC/MS (m/z) 402 (MH+); RT = 3,1 (method A); purity (UV, ELSD): 98%; 99%.

**123**: 4-(3,3-Dimethyl-butyrylamino)-3-methoxymethyl-N-thiazol-2-yl-benzamide:

LC/MS (m/z) 362 (MH+); RT = 2.6 (method A); purity (UV, ELSD): 86%; 99%.

**124**: 4-(3,3-Dimethyl-butyrylamino)-3-propoxy-N-thiazol-2-yl-benzamide:
LC/MS (m/z) 376 (MH+); RT = 3.3 (method A); purity (UV, ELSD): 99%; 97%.

**125**: 3-Chloro-4-(3,3-dimethyl-butyrylamino)-5-methyl-N-thiazol-2-yl-benzamide: 4-Amino-3-chloro-5-methyl-N-thiazol-2-yl-benzamide (0.06 mmol) was dissolved in 1,2-dichloroethane (0.75 mL). Pyridine (5.8 μL) and 3,3-Dimethyl-butyryl chloride (10 μL) were added. The reaction mixture was heated to 130°C for 2 h in Personal chemistry microwave oven?. The product was filtered off and dried.
LC/MS (m/z) 366.1 (MH+); RT = 2,6 (method A); purity (UV, ELSD): 98%; 99%.

**126:** 4-(3,3-Dimethyl-butyrylamino)-3,5-difluoro-N-thiazol-2-yl-benzamide:
LC/MS (m/z) 354.0 (MH+); RT = 2,5 (method A); purity (UV, ELSD): 91%; 99%.

**127:** 4-(3,3-Dimethyl-butyrylamino)-3,5-dimethyl-N-thiazol-2-yl-benzamide:
LC/MS (m/z) 345.9 (MH+); RT = 2,5 (method A); purity (UV, ELSD): 95%; 99%.

**128:** 4-(3,3-Dimethyl-butyrylamino)-N-thiazol-2-yl-3-trifluoromethyl-benzamide:
LC/MS (m/z) 386.2 (MH+); RT = 2,9 (method A); purity (UV, ELSD): 95%; 99%.

**129:** 3-Chloro-4-(3,3-dimethyl-butyrylamino)-N-thiazol-2-yl-5-trifluoromethyl-benzamide:
LC/MS (m/z) 420.3 (MH+); RT = 2,9 (method A); purity (UV, ELSD): 95%; 98%.

**130**: *N*-(2,2-Dimethyl-propyl)-*N'*-thiazol-2-yl-terephthalamide
*N*-Thiazol-2-yl-terephthalamic acid (2 mmol) was dissolved in 1,2-dichloroethane (10 mL) and DMF (0.5 mL). DIPEA (2 mmol), 1-(3-dimethylaminopropyl)-3-ethylcarbodiimide hydrochloride (2 mmol), 1-hydroxybenzotriazole (2 mmol) and 2,2-dimethyl-propylamine (2.4 mmol) was added. Stirred over night at room temperature, then 2.4 mmol HCl (2M) was added along with water (3 mL). The reaction mixture was filtered, the precipitate re-dissolved in ethyl acetate and extracted with NaOH (2M), dried over $MgSO_4$, filtered and evaporated.
Yield: 16%
1H NMR ($D_6$-DMSO): 0.92 (s, 9H); 3.13 (d, 2H); 7.30 (d, 1H); 7.58 (d, 1H); 7.98 (d, 2H); 8.16 (d, 2H); 8.52 (t, 1H); 12.76 (br s, 1H).

**131:** 3,5-Dichloro-4-(3,3-dimethyl-butyrylamino)-*N*-thiazol-2-yl-benzamide
4-Amino benzoic acid (36 mmol) was suspended in 1,2-dichloroethane. Pyridine (44 mmol) was added followed by drop wise addition of 3,3-dimethyl-butyryl chloride (44 mmol). The mixture was stirred over night at ambient temperature, and then filtered. The solids were washed with 1,2-dichloroethane and dried *in vacuo*.
Yield: 55%
1H NMR ($D_6$-DMSO): 1.02 (t, 9H); 2.22 (s, 2H); 7.71 (d, 2H); 7.88 (d, 2H); 10.09 (s, 1H); 12.68 (br, 1H).
4-(3,3-Dimethyl-butyrylamino)-benzoic acid (2 mmol) was dissolved in DMF (5 mL), *N*-chlorosuccinimide (8.5 mmol) was added portion wise. The reaction mixture was stirred at 40 °C over night. Another 8.5 mmol *N*-chlorosuccinimide was added, the reaction mixture was then stirred over night at 50 °C. Another 4.3 mmol *N*-chlorosuccinimide was added, stirring was continued at 50 °C for 2h. This was repeated 5 times. Water (30 mL) was added, the formed precipitate was filtered off, washed with water and dried *in vacuo*.
Yield: 82%
1H NMR ($D_6$-DMSO): 1.07 (s, 9H); 2.26 (s, 2H); 7.95 (s, 2H); 9.91 (s, 1H).
3,5-Dichloro-4-(3,3-dimethyl-butyrylamino)-benzoic acid (1.7 mmol) was suspended in 1,2-dichloroethane (15 mL) and DMF (150 μL) under an argon atmosphere. Oxalylchloride (2M in dichloromethane, 1.02 mL) was added slowly to the stirred suspension. After Stirring at room temperature for 1h the solvent was removed by evaporation under reduced pressure, and the reaction mixture was re-dissolved in 1,2-dichloroethane (15 mL). A suspension of 2-amino thiazole (1.7 mmol) and pyridine (1.7 mmol) in 1,2-dichloroethane (5 mL) was added portion wise. The reaction mixture was stirred at 50 °C over night. The reaction mixture evaporated and re-dissolved in ethyl acetate, then washed with NaOH (0.1M). The organic phase was dried over $MgSO_4$, filtered and evaporated. The crude was re-crystallized from EtOH.
Yield: 7% (3% overall)
1H NMR ($D_6$-DMSO): 1.08 (s, 9H); 2.27 (s, 2H); 7.31 (d, 1H); 7.58 (d, 1H); 8.20 (s, 1H); 9.92 (s, 1H); 12.87 (br s, 1H).

**132**: 4-(3-*tert*-Butyl-ureido)-*N*-thiazol-2-yl-benzamide

4-Amino-*N*-thiazol-2-yl-benzamide (0.46 mmol) was suspended in 1,2-dichloroethane (5 mL) and 2-isocyanato-2-methyl-propane (2.3 mmol) was added. The reaction mixture was heated at 140 °C by microwave irradiation for 3.5 h. The reaction mixture was evaporated to dryness and purified by preparative HPLC-MS.
Yield: 11 %
LC/MS (m/z) 372 ($MH^+$); RT = 2.21 (method A); purity (UV, ELSD): 95%, 98%.

**133:** [4-(Thiazol-2-ylcarbamoyl)-phenyl]-carbamic acid 2,2-dimethyl-propyl ester
4-Amino-*N*-thiazol-2-yl-benzamide (0.46 mmol) was suspended in 1,2-dichloroethane (5mL) and DIPEA (2.3 mmol) and 2,2-dimethylpropyl chloroformate (0.46 mmol) was added. The reaction mixture was stirred at 50 °C for 24h and 70 °C for 24h. The crude mixture was evaporated to dryness and purified by preparative HPLC-MS.
Yield: 28%
1H NMR ($D_6$-DMSO): 0.96 (s, 9H); 3.84 (s, 2H); 7.26 (d, 1H); 7.55 (d, 1H); 7.62 (d, 2H); 8.06 (d, 2H); 10.00 (s, 1H); 12.44 (br, 1H).

**Pharmacological Testing**

The compounds of the invention were tested according to the following methods:

**$A_{2A}$ efficacy assays**

Cloning of the human cDNA encoding the $A_{2a}$ receptor.
cDNA was obtained by random primed reverse transcription of human fetal brain RNA (Clonetech). A subsequent polymerase chain reaction (PCR) was performed using the cDNA as template and the oligonucleotides TTTACGCGTGGCCATGCCCATCATGGGCTCCTC and TTTCTAGAATCAGCACACTCCTGCTCCATC as primers for the amplification.

The amplification was performed using Pfu polymerase (Stratagene, in accordance with the manufactures recommendation) with an annealing temperature of 54°C. The reaction mixture was analyzed by an agarose gel electrophoresis and a band of 1.2 kb was excised and the DNA eluded. The eluded DNA was digested with the restriction enzymes MluI and XbaI and ligated into a vector, pCIneo, cut with the same enzymes. DNA was isolated and sequenced. CHO cells was transfected with the pCIneo clone expressing the $A_{2a}$ receptor and cells with stable integration of the plasmids were isolated after 2-3 weeks growth in the presence of either 5 mg/ml or 10mg/ml G418.

CHO cells transfected with $A_{2A}$ receptors as described above were grown in F12 nutrient mixture (kaighs modification, Life technologies) with 10% FCS, 1% glutamin and 1% penicillin/streptomycin and 1 mg/mL G418.

24 h prior to assay performance, 10000 cells/well were seeded in costar 96-well plates in media without G418 to 60-80% confluence. The cells were stimulated with NECA (00-9498, final concentration 75 nM) corresponding to about 80% agonist efficacy.

The cell media was removed and the cells washed 3 times in 37 °C pre-equilibrated PBS and incubated (on shaker) with 10 μL of a suspension of acceptor beads and 10μL of a solution of test compound or standard compound (0-10 μM) in darkness for 30 min at 25 °C before addition of 30 μl of a suspension of donor beads and further incubation 60-120 min in darkness. The plates were analysed according to manufacturers instruction (Alpha screen, Perkin Elmer (Pachard Biosciense)).

The acceptor beads were suspended in a stimulation buffer (5 mM HEPES, 0.1 % BSA in Hanks balanced salt pH 7.4 w/o phenol red (Gibco). The donor beads were suspended in a lysis buffer (the stimulation buffer with 0,3% Tween 20 and biotinylated cAMP) according to manufacturers instruction (Alpha screen, Perkin Elmer (Pachard Biosciense)).

The data were fitted with non-linear regression, and $IC_{50}$ and $K_i$ values were calculated from the equations:

$$IC_{50} = ( [ I ]/ (100/(100\text{-}\%INH))/(1+([ag]/EC_{50})$$

and

$$K_i = IC_{50}/(1\text{-}[ag]/EC_{50}),$$

where [ I ] is the inhibitor concentration, [ag] is the assay agonist concentration and $EC_{50}$ is the agonist concentration

required for half maximal effect.

**$A_{2A}$ binding assay:**

Membrane preparations for $A_{2A}$ binding analysis:

Expression in insect cells

The human $A_{2a}$ encoding DNA were excised from the pCIneo constructs by MluI and XbaI and subcloned into the pFASTBAC2 vector cut with XbaI and BssHII. The inserts were recombined into the baculo vector using the Bac-to-Bac® system (Invitrogen). The generation and isolation of baculo virus was performed as described by the distributor (Invitrogen). High Five cells (Invitrogen) was grown at 27°C in suspension to a density of $1*10^6$ and infected with a MOI of 0.5. The cells are harvested 72 h post infection and membranes prepared.

High five cells expressing $A_{2A}$ receptors were homogenized in 50 mM tris-buffer pH 7.4 in an ultra Turrax homogenisator. The membranes were diluted to a concentration of 0.6 mg/ml and 2U Adenosine deaminase (Roche) /ml membrane suspension was added. The solution was preincubated 30 min at 37 °C before use.

$A_{2A}$ binding analysis:

Binding assay was performed in 96 well flat bottom plate and initiated by mixing 10.6 μg protein/well with solutions of standard compounds or test compounds (final concentrations 0-10 μM) and 1 nM final concentration of $^3$H-ZM241385 (R1036 from Tocris). All test compounds were diluted in 50 nM trisbuffer from DMSO-stocks (2 mM or 10 mM). The reactions (final volume = 200 μL) were incubated for 30 min at 25 °C and washed on Unifilter-GF/B with water. The filters were dried 20 min (37 °C) before addition of 35 μl Microscient-0 or Optiphase supermix and counting in a Trilux counter for 1 min.

The data were fitted with non-linear regression, and $IC_{50}$ and $K_i$ values were calculated from the equations :

$$IC_{50} = ( [ I ] / (100/(100\text{-}\%INH))/(1+([L]/K_D)$$

and

$$K_i = IC_{50}/(1-[L]/K_D),$$

where [ I ] is the inhibitor concentration, and [L] and $K_D$ are concentration and dissociation equilibrium constant of the radiotracer, respectively.

The exemplified compounds 1-119 of the invention are $A_{2A}$ receptors antagonists having a human $A_{2A}$ binding affinity ($K_i$) of 530 nM or less.

**Formulation Examples**

The pharmaceutical formulations of the invention may be prepared by conventional methods in the art.

For example: Tablets may be prepared by mixing the active ingredient with ordinary adjuvants and/or diluents and subsequently compressing the mixture in a conventional tabletting machine. Examples of adjuvants or diluents comprise: Corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.

Solutions for injections may be prepared by dissolving the active ingredient and possible additives in a part of the solvent for injection, preferably sterile water, adjusting the solution to the desired volume, sterilising the solution and filling it in suitable ampoules or vials. Any suitable additive conventionally used in the art may be added, such as tonicity agents, preservatives, antioxidants, etc.

Typical examples of recipes for the formulation of the invention are as follows:

1) Tablets containing 5.0 mg of a compound of the invention calculated as the free base:

| | |
|---|---|
| Compound 1 | 5.0 mg |
| Lactose | 60 mg |
| Maize starch | 30 mg |
| Hydroxypropylcellulose | 2.4 mg |
| Microcrystalline cellulose | 19.2 mg |
| Croscarmellose Sodium Type A | 2.4 mg |
| Magnesium stearate | 0.84 mg |

2) Tablets containing 0.5 mg of a compound of the invention calculated as the free base:

| | |
|---|---|
| Compound 1 | 0.5 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Compound 1 | 25 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 mL |
| Flavour | 0.05 mg |
| Saccharin sodium | 0.5 mg |
| Water | ad 1 mL |

4) Solution for injection containing per millilitre:

| | |
|---|---|
| Compound 1 | 0.5 mg |
| Sorbitol | 5.1 mg |
| Acetic Acid | 0.05 mg |
| Saccharin sodium | 0.5 mg |
| Water | ad 1 mL |

**Claims**

**1.** Use of a compound of formula I

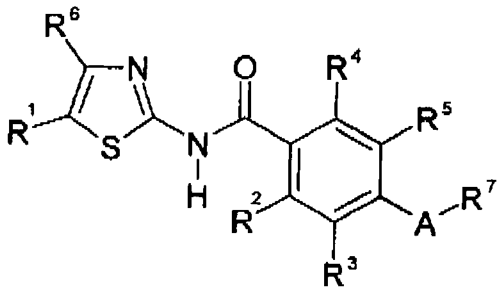

I

wherein $R^1$ and $R^6$ are independently hydrogen, $C_{1-6}$-alkyl or halogen;
$R^2$-$R^5$ are independently selected from the group consisting of hydrogen, halogen, cyano, OH, $NH_2$, nitro, $C_{1-6}$-alkyl, aryl, aryl-$C_{1-6}$-alkyl, heteroaryl-$C_{1-6}$-alkyl, $C_{3-8}$-cycloalkyl, $C_{3-8}$-cycloalkyl-$C_{1-6}$-alkyl, $C_{1-6}$-alkoxy, aryl-$C_{1-6}$-alkoxy, $C_{1-6}$-alkylamino and aryl-$C_{1-6}$-alkylamino wherein each alkyl, alkoxy or aryl may be optionally substituted with one or more halogen, cyano, $C_{1-6}$-alkyl, $C_{1-6}$-alkoxy, or $C_{1-6}$-alkoxy-$C_{1-6}$-alkoxy;
or $R^4$ and $R^5$ together are X-$(CH_2)_n$-Y, wherein X and Y independently are selected from the group consisting of $CH_2$, and NH and O, n is 1, 2 or 3, and $R^2$ and $R^3$ are as defined above;
A is *$NR^8$-CO, *CO-$NR^9$, *$NR^8$-CS or *CS-$NR^9$ in which $R^8$ and $R^9$ are independently selected from the group consisting of hydrogen and $C_{1-6}$-alkyl, or $R^8$ together with $R^3$ are $C_{2-3}$-alkylene or $CH_2CH_2O$ wherein the oxygen is attached to the phenylring, and the * indicates the atom that is attached to the phenyl ring;
and $R^7$ is selected from the group consisting of $C_{1-8}$-alkyl, aryl, heteroaryl, aryl-$C_{1-6}$-alkyl, heteroaryl-$C_{1-6}$-alkyl, $C_{3-8}$-cycloalkyl, $C_{3-8}$-cycloalkyl-$C_{1-6}$-alkyl, $C_{1-6}$-alkoxy, aryl-$C_{1-6}$-alkoxy, heteroaryl-$C_{1-6}$-alkoxy, $C_{1-6}$-alkylamino, aryl-$C_{1-6}$-alkylamino, heteroaryl-$C_{1-6}$-alkylamino, di-($C_{1-6}$-alkyl)-amino, 2,3-dihydrobenzo-[1,4]dioxin-2-yl or adamantan-1-yl-methyl wherein each alkyl and cycloalkyl may be optionally substituted with one or more halogen, cyano, hydroxy, oxo, $C_{1-6}$-alkoxy or $NR^{10}R^{11}$, wherein $R^{10}$ and $R^{11}$ independently are hydrogen or $C_{1-6}$-alkyl, or $R^{10}$ and $R^{11}$ together with the nitrogen form a 5, 6 or 7 membered aliphatic ring which optionally may contain one further heteroatom selected from N and O, and each aryl may be optionally substituted with one or more halogen, cyano, hydroxy, nitro, $C_{1-6}$-alkyl, $C_{1-6}$-alkoxy, $C_{1-6}$-acyl, $C_{1-6}$-acyloxy, $NR^{10}R^{11}$ wherein $R^{10}$ and $R^{11}$ independently are hydrogen or $C_{1-6}$-alkyl or $R^{10}$ and $R^{11}$ together with the nitrogen form a 5, 6 or 7 membered aliphatic ring which optionally may contain one further heteroatom selected from N and O, or a group Z-$(CH_2)_m$-W, Wherein Z and W are attached to two adjacent carbon atoms and independently are selected from the group consisting of $CH_2$, NH and O, and m is 1, 2 or 3, provided that when $R^7$ is attached to nitrogen, then $R^7$ is not $C_{1-6}$-alkoxy, aryl-$C_{1-6}$-alkoxy, heteroaryl-$C_{1-6}$-alkoxy, $C_{1-6}$-alkylamino, aryl-$C_{1-6}$-alkylamino, heteroaryl-$C_{1-6}$-alkylamino or di-($C_{1-6}$-alkyl)-amino;
and pharmaceutically acceptable addition salts thereof;
for the manufacture of a medicament for treatment of a disease where an $A_{2A}$-receptor is implicated.

2. Use of a compound according to claim 1, wherein the disease is selected from the group consisting of Parkinson's Disease , Alzheimer's Disease, Huntington's disease, epilepsia, cerebral ischemia, haemorrhagic stroke, neonatal ischemia and hypoxia, subarachnoid haemorrhage, traumatic brain injury, brain damage following cardiac arrest, and for the treatment of depression and psychosis disorders.

3. Use of a compound according to claim 2, wherein the disease is Parkinson's disease.

4. Use of a compound according to any of claims 1-3, wherein A is *$NR^8$-CO or *CO-$NR^9$.

5. Use of a compound according to claim 4, wherein A is *$NR^8$-CO.

6. Use of a compound according to any of claims 1-5, wherein $R^7$ is selected from the group consisting of $C_{1-8}$-alkyl, preferably $C_{3-8}$-alkyl and even more preferred $C_{4-8}$-alkyl which is branched at the β-position, $C_{3-8}$-cycloalkyl-methyl, $C_{3-8}$-cycloalkyl, methylphenyl, methoxybenzyl and thiophen-2-yl-methyl, wherein each alkyl or cycloalkyl may be unsubstituted or substituted with oxo.

7. Use of a compound according to any of claims 1-6, wherein $R^8$ is hydrogen.

8. Use of a compound according to any of claims 1-7, wherein $R^9$ is hydrogen.

**9.** Use of a compound according to any of claims 1-8, wherein $R^6$ is hydrogen.

**10.** Use of a compound according to any of claims 1-9, wherein $R^1$ is hydrogen, methyl or chloro, preferably hydrogen.

**11.** Use of a compound according to any of claims 1-10, wherein $R^{2-5}$ are independently selected from the group consisting of hydrogen, halogen, $C_{1-6}$-alkyl, preferably methyl, $C_{1-6}$-alkoxy and $C_{1-6}$-alkoxy-$C_{1-6}$-alkoxy, preferably 2-methoxy-ethoxy.

**12.** Use of a compound according to claim 11, wherein $R^2$ and $R^4$ are independently selected from the group consisting of hydrogen, $C_{1-6}$-alkoxy and $C_{1-6}$-alkoxy-$C_{1-6}$-alkoxy, preferably 2-methoxy-ethoxy.

**13.** Use of a compound according to claim 11 or 12, wherein $R^3$ and $R^5$ are independently selected from the group consisting of hydrogen, halogen, $C_{1-6}$-alkyl, preferably methyl, $C_{1-6}$-alkoxy, preferably methoxy, $C_{1-6}$-alkoxy-$C_{1-6}$-alkoxy, preferably 2-methoxy-ethoxy, trifluoromethyl and trifluoromethoxy.

**14.** Use of a compound according to claim 1, wherein said compound is selected from the group consisting of:

4-Butyrylamino-*N*-thiazol-2-yl-benzamide,
*rac*-3-methoxy-4-(3-methyl-4-oxo-pentanoylamino)-*N*-thiazol-2-yl-benzamide,
*rac*-4-(3-methyl-pentanoylamino)-*N*-thiazol-2-yl-benzamide,
4-hexanoylamino-3-methyl-*N*-thiazol-2-yl-benzamide,
4-(2-cycloheptyl-acetylamino)-*N*-thiazol-2-yl-benzamide,
*rac*-3-methoxy-4-(3-methyl-pentanoylamino)-*N*-thiazol-2-yl-benzamide,
4-(2-cycloheptyl-acetylamino)-3-methoxy-*N*-thiazol-2-yl-benzamide,
*rac*-4-[2-(2-oxo-cyclopentyl)-acetylamino]-*N*-thiazol-2-yl-benzamide,
4-hexanoylamino-3-methoxy-*N*-thiazol-2-yl-benzamide,
3-methyl-4-(4-phenyl-butyrylamino)-*N*-thiazol-2-yl-benzamide,
4-(2-cyclohexyl-acetylamino)-*N*-thiazol-2-yl-benzamide,
*rac*-4-(2-bicyclo[2.2.1]hept-2-yl-acetylamino)-*N*-thiazol-2-yl-benzamide,
4-(3,3-dimethyl-butyrylamino)-*N*-(4,5-dimethyl-thiazol-2-yl)-benzamide,
4-(2-adamantan-1-yl-acetylamino)-*N*-thiazol-2-yl-benzamide,
4-(3-benzo[1,3]dioxol-5-yl-propionylamino)-3-methyl-*N*-thiazol-2-yl-benzamide,
4-(3-hydroxy-3-methyl-butyrylamino)-3-methoxy-*N*-thiazol-2-yl-benzamide,
4-(4-fluoro-benzoylamino)-3-methoxy-*N*-thiazol-2-yl-benzamide,
4-benzoylamino-*N*-thiazol-2-yl-benzamide,
thiophene-3-carboxylic acid [4-(thiazol-2-ylcarbamoyl)-phenyl]-amide,
*N*-thiazol-2-yl-4-(2-*o*-tolyl-acetylamino)-benzamide,
*N*-thiazol-2-yl-4-(2-thiophen-3-yl-acetylamino)-benzamide,
4-(2-cyclopentyl-acetylamino)-3-methyl-*N*-thiazol-2-yl-benzamide,
4-(3,3-dimethyl-butyrylamino)-*N*-thiazol-2-yl-benzamide,
4-(3,3-dimethyl-butyrylamino)-3-methyl-*N*-thiazol-2-yl-benzamide,
4-(3,3-dimethyl-butyrylamino)-2-methoxy-*N*-thiazol-2-yl-benzamide,
3-chloro-4-(3-methyl-butyrylamino)-*N*-thiazol-2-yl-benzamide,
3-bromo-4-(3,3-dimethyl-butyrylamino)-*N*-thiazol-2-yl-benzamide,
4-(3-methyl-butyrylamino)-*N*-thiazol-2-yl-benzamide,
3-bromo-4-(3-methyl-butyrylamino)-*N*-thiazol-2-yl-benzamide,
4-(2-cyclopentyl-acetylamino)-*N*-thiazol-2-yl-benzamide,
3-methyl-4-(3-methyl-butyrylamino)-*N*-thiazol-2-yl-benzamide,
3-chloro-4-(cyclopentanecarbonyl-amino)-*N*-thiazol-2-yl-benzamide,
3-chloro-4-(2-methyl-benzoylamino)-*N*-thiazol-2-yl-benzamide,
3-bromo-4-(cyclopentanecarbonyl-amino)-*N*-thiazol-2-yl-benzamide,
4-(cyclopentanecarbonyl-amino)-*N*-thiazol-2-yl-benzamide,
4-(cyclopentanecarbonyl-amino)-3-methyl-*N*-thiazol-2-yl-benzamide,
cycloheptanecarboxylic acid [2-bromo-4-(thiazol-2-ylcarbamoyl)-phenyl]-amide,
4-isobutyrylamino-2-methoxy-*N*-thiazol-2-yl-benzamide,
8-(3,3-dimethyl-butyrylamino)-2,3-dihydro-benzo[1,4]dioxine-5-carboxylic acid thiazol-2-ylamide,
3-bromo-4-butyrylamino-*N*-thiazol-2-yl-benzamide,
2-methoxy-4-(3-methyl-butyrylamino)-*N*-thiazol-2-yl-benzamide,

cycloheptanecarboxylic acid [4-(thiazol-2-ylcarbamoyl)-phenyl]-amide,
*rac*-2-methoxy-4-(2-methyl-butyrylamino)-*N*-thiazol-2-yl-benzamide,
4-(cyclopentanecarbonyl-amino)-2-methoxy-*N*-thiazol-2-yl-benzamide,
3-bromo-4-(2-methyl-benzoylamino)-*N*-thiazol-2-yl-benzamide,
3-chloro-4-(3,3-dimethyl-butyrylamino)-*N*-thiazol-2-yl-benzamide,
4-(2-cyclopentyl-acetylamino)-2-propoxy-*N*-thiazol-2-yl-benzamide,
4-(3,3-dimethyl-butyrylamino)-2-propoxy-*N*-thiazol-2-yl-benzamide,
4-(2-cyclopentyl-acetylamino)-3-fluoro-*N*-thiazol-2-yl-benzamide,
4-(3-methyl-butyrylamino)-2-propoxy-*N*-thiazol-2-yl-benzamide,
3-fluoro-4-(3-methyl-butyrylamino)-*N*-thiazol-2-yl-benzamide,
4-butyrylamino-3-fluoro-*N*-thiazol-2-yl-benzamide,
4-butyrylamino-2-propoxy-*N*-thiazol-2-yl-benzamide,
3-fluoro-4-(2-methyl-benzoylamino)-*N*-thiazol-2-yl-benzamide,
cycloheptanecarboxylic acid [2-fluoro-4-(thiazol-2-ylcarbamoyl)-phenyl]-amide,
4-(cyclopentanecarbonyl-amino)-3-fluoro-*N*-thiazol-2-yl-benzamide,
4-(3,3-dimethyl-butyrylamino)-2-(2-methoxy-ethoxy)-*N*-thiazol-2-yl-benzamide,
3-fluoro-4-(3-methyl-benzoylamino)-*N*-thiazol-2-yl-benzamide,
*rac*-3-fluoro-*N*-thiazol-2-yl-4-(3,5,5-trimethyl-hexanoylamino)-benzamide,
4-(2-cyclopentyl-acetylamino)-2-(2-methoxy-ethoxy)-*N*-thiazol-2-yl-benzamide,
4-(2-methyl-benzoyl)-3,4-dihydro-2H-benzo[1,4]oxazine-7-carboxylic acid thiazol-2-ylamide,
4-(3,3-dimethyl-butyrylamino)-3-fluoro-*N*-thiazol-2-yl-benzamide,
4-(3,3-dimethyl-butyrylamino)-2-methyl-*N*-thiazol-2-yl-benzamide,
5-chloro-4-(3,3-dimethyl-butyrylamino)-2-methoxy-*N*-thiazol-2-yl-benzamide,
4-(3,3-dimethyl-butyrylamino)-*N*-(5-methyl-thiazol-2-yl)-benzamide,
5-chloro-2-methoxy-4-(3-methyl-butyrylamino)-*N*-thiazol-2-yl-benzamide,
4-(2-methyl-benzoylamino)-*N*-(5-methyl-thiazol-2-yl)-benzamide,
1-(3,3-dimethyl-butyryl)-1,2,3,4-tetrahydro-quinoline-6-carboxylic acid thiazol-2-yl-amide,
5-chloro-2-methoxy-4-(2-methyl-benzoylamino)-*N*-thiazol-2-yl-benzamide,
1-(3-methyl-butyryl)-1,2,3,4-tetrahydro-quinoline-6-carboxylic acid thiazol-2-yl-amide,
1-(3,3-dimethyl-butyryl)-2,3-dihydro-1*H*-indole-5-carboxylic acid thiazol-2-ylamide,
4-[(3,3-dimethyl-butyryl)-methyl-amino]-*N*-thiazol-2-yl-benzamide,
4-[(2-cyclopentyl-acetyl)-propyl-amino]-*N*-thiazol-2-yl-benzamide,
2-(2-methoxy-ethoxy)-4-(3-methyl-butyrylamino)-*N*-thiazol-2-yl-benzamide,
*rac*-2-propoxy-*N*-thiazol-2-yl-4-(3,5,5-trimethyl-hexanoylamino)-benzamide,
*rac*-*N*-thiazol-2-yl-4-(3,5,5-trimethyl-hexanoylamino)-benzamide,
4-(3-cyclopentyl-propionylamino)-*N*-thiazol-2-yl-benzamide,
4-(2-cyclopentyl-acetylamino)-3-methoxy-*N*-thiazol-2-yl-benzamide,
cycloheptanecarboxylic acid [2-methyl-4-(thiazol-2-ylcarbamoyl)-phenyl]-amide,
3-methoxy-4-(3-phenyl-propionylamino)-*N*-thiazol-2-yl-benzamide,
cycloheptanecarboxylic acid [2-chloro-4-(thiazol-2-ylcarbamoyl)-phenyl]-amide,
4-[2-(3-methoxy-phenyl)-acetylamino]-3-methyl-*N*-thiazol-2-yl-benzamide,
3-bromo-4-(2-cyclopentyl-acetylamino)-*N*-thiazol-2-yl-benzamide,
4-butyrylamino-3-chloro-*N*-thiazol-2-yl-benzamide,
5-chloro-4-(2-cyclopentyl-acetylamino)-2-methoxy-*N*-thiazol-2-yl-benzamide,
5-chloro-4-(cyclopentanecarbonyl-amino)-2-methoxy-*N*-thiazol-2-yl-benzamide,
4-(cyclohexanecarbonyl-amino)-2-methoxy-*N*-thiazol-2-yl-benzamide,
2-methoxy-4-(4-methoxy-benzoylamino)-*N*-thiazol-2-yl-benzamide,
3-methoxy-4-phenylacetylamino-*N*-thiazol-2-yl-benzamide,
3-methyl-*N*-thiazol-2-yl-4-(2-thiophen-2-yl-acetylamino)-benzamide,
3-chloro-4-(2-cyclopentyl-acetylamino)-*N*-thiazol-2-yl-benzamide,
4-(4-methoxy-benzoylamino)-3-methyl-*N*-thiazol-2-yl-benzamide,
4-butyrylamino-3-methyl-*N*-thiazol-2-yl-benzamide,
4-(2-chloro-benzoylamino)-3-methyl-*N*-thiazol-2-yl-benzamide,
4-(2,5 di-chloro-benzoylamino)-3-methyl-*N*-thiazol-2-yl-benzamide,
4-(2-chloro-benzoylamino)-2-methoxy-*N*-thiazol-2-yl-benzamide,
4-(2-ethyl-butyrylamino)-2-methoxy-*N*-thiazol-2-yl-benzamide,
2-methoxy-4-(2-methyl-benzoylamino)-*N*-thiazol-2-yl-benzamide,
3-methyl-4-(3-phenyl-propionylamino)-*N*-thiazol-2-yl-benzamide,

4-(3,3-dimethyl-butyrylamino)-3-methoxy-*N*-thiazol-2-yl-benzamide,
*rac*-3-methyl-*N*-thiazol-2-yl-4-(3,5,5-trimethyl-hexanoylamino)-benzamide,
*rac*-2,3-dihydro-benzo[1,4]dioxine-2-carboxylic acid [2-methoxy-4-(thiazol-2-yl-carbamoyl)-phenyl]-amide,
4-(2,2-dimethyl-propionylamino)-3-methoxy-*N*-thiazol-2-yl-benzamide,
2-methoxy-4-(4-methyl-benzoylamino)-*N*-thiazol-2-yl-benzamide,
thiophene-2-carboxylic acid [3-methoxy-4-(thiazol-2-ylcarbamoyl)-phenyl]-amide,
4-(3-methoxy-benzoylamino)-*N*-thiazol-2-yl-benzamide,
8-(2-cyclopentyl-acetylamino)-2,3-dihydro-benzo[1,4]dioxine-5-carboxylic acid thiazol-2-ylamide,
6-(2-cyclopentyl-acetylamino)-biphenyl-3-carboxylic acid thiazol-2-ylamide,
4-(2-cyclopentyl-acetylamino)-3-(2-methoxy-ethoxy)-*N*-thiazol-2-yl-benzamide
4-(3,3-dimethyl-butyrylamino)-2-fluoro-*N*-thiazol-2-yl-benzamide,
2-chloro-4-(2-methyl-benzoylamino)-*N*-thiazol-2-yl-benzamide,
4-(2-fluoro-benzoylamino)-*N*-thiazol-2-yl-benzamide,
4-(2-methoxy-benzoylamino)-*N*-thiazol-2-yl-benzamide,
benzo[*b*]thiophene-2-carboxylic acid [2-methyl-4-(thiazol-2-ylcarbamoyl)-phenyl]-amide,
5-(3,3-dimethyl-butyrylamino)-biphenyl-2-carboxylic acid thiazol-2-ylamide,
N-(5-chloro-thiazol-2-yl)-4-(3,3-dimethyl-butyrylamino)-benzamide,
N-(5-chloro-thiazol-2-yl)-4-(3-methyl-butyrylamino)-benzamide,
N-(5-chloro-thiazol-2-yl)-4-(2-cyclopropyl-acetylamino)-benzamide,
4-butyrylamino-N-(5-chloro-thiazol-2-yl)-benzamide,
4-benzoylamino-*N*-(5-chloro-thiazol-2-yl)-benzamide,
3-fluoro-N-thiazol-2-yl-4-(4,4,4-trifluoro-3-methyl-butyrylamino)-benzamide,
4-(3,3-dimethyl-butyrylamino)-N-thiazol-2-yl-3-trifluoromethoxy-benzamide,
4-(3,3-dimethyl-butyrylamino)-3-methoxymethyl-N-thiazol-2-yl-benzamide,
4-(3,3-dimethyl-butyrylamino)-3-propoxy-N-thiazol-2-yl-benzamide,
3-chloro-4-(3,3-dimethyl-butyrylamino)-5-methyl-N-thiazol-2-yl-benzamide,
4-(3,3-dimethyl-butyrylamino)-3,5-difluoro-N-thiazol-2-yl-benzamide,
4-(3,3-dimethyl-butyrylamino)-3,5-dimethyl-N-thiazol-2-yl-benzamide,
4-(3,3-dimethyl-butyrylamino)-N-thiazol-2-yl-3-trifluoromethyl-benzamide,
3-chloro-4-(3,3-dimethyl-butyrylamino)-N-thiazol-2-yl-5-trifluoromethyl-benzamide,
*N*-(2,2-dimethyl-propyl)-*N'*-thiazol-2-yl-terephthalamide,
3,5-dichloro-4-(3,3-dimethyl-butyrylamino)-*N*-thiazol-2-yl-benzamide,
4-(3-*tert*-butyl-ureido)-*N*-thiazol-2-yl-benzamide, and [4-(thiazol-2-ylcarbamoyl)-phenyl]-carbamic acid 2,2-dimethyl-propyl ester.

**15.** A pharmaceutical composition comprising a compound of formula I according to any of claims 1-14 provided that if A is *NR$^8$-CO, and R$^{1-6}$ and R$^8$ all are hydrogen, then R$^7$ is not thiophen-2-yl;
and provided that if A is *NR$^8$-CO, R$^{2-6}$ and R$^8$ all are hydrogen, and R$^1$ is *i*-propyl then R$^7$ is not methyl or benzyl;
and provided that if A is *NR$^8$-CO, R$^2$, R$^{4-6}$ and R$^8$ all are hydrogen, R$^3$ is iodine and R$^1$ is i-propyl then R$^7$ is not methyl;
and provided that if A is *NR$^8$-CO, R$^1$, R$^{3-6}$ and R$^8$ all are hydrogen and R$^2$ is hydroxy then R$^7$ is not methyl or ethoxy.

**16.** The composition according to claim 15, wherein said compound is an $A_{2A}$ receptor antagonist having a human $A_{2A}$ binding affinity ($K_i$) of 200 nM or less.

**17.** A compound of formula I according to any of claims 1-14 provided that if A is *NR$^8$-CO, and R$^{1-6}$ and R$^8$ all are hydrogen, then R$^7$ is not selected from the group consisting of $C_{1-4}$-alkyl, pentan-3-yl, trifluoromethyl, pyrimidyl, furan-2-yl, thiophen-2-yl, substituted or unsubstituted phenyl or substituted or unsubstituted benzyl;
and provided that if A is *NR$^8$-CO, R$^{2-6}$ and R$^8$ all are hydrogen, and R$^1$ is *i*-propyl then R$^7$ is not methyl or benzyl;
and provided that if A is *NR$^8$-CO, R$^2$, R$^{4-6}$ and R$^8$ all are hydrogen, R$^3$ is iodine and R$^1$ is *i*-propyl then R$^7$ is not methyl;
and provided that if A is *NR$^8$-CO, R$^1$, R$^{3-6}$ and R$^8$ all are hydrogen and R$^2$ is hydroxy then R$^7$ is not methyl or ethoxy;
and provided that if A is *NR$^8$-CO, R$^2$, R$^{4-6}$ and R$^8$ all are hydrogen, R$^2$ is nitro and R$^7$ is methyl then R$^1$ is not hydrogen or methyl.
and provided that if A is *CO-NR$^9$, R$^1$, R$^6$ and R$^9$ all are hydrogen, and R$^7$ is thiazol-2-yl, then R$^{2-5}$ are not all hydrogen or all fluor;
and provided that if A is *CO-NR$^9$, R$^{2-5}$ and R$^9$ all are hydrogen, and R$^6$ is methyl, then R$^1$ may not be hydrogen if R$^7$ is 4-methyl-thiazol-2-yl and R$^1$ may not be methyl if R$^7$ is 4,5-dimethyl-thiazol-2-yl.

**18.** The compound according to claim 17, wherein said compound is in the form of a salt.

**19.** The compound according to claim 17 or 18, wherein said compound is an $A_{2A}$ receptor antagonist having a human $A_{2A}$ binding affinity ($K_i$) of 200 nM or less.

**20.** A compound selected from the group consisting of:

4-Butyrylamino-*N*-thiazol-2-yl-benzamide,
*rac*-3-methoxy-4-(3-methyl-4-oxo-pentanoylamino)-*N*-thiazol-2-yl-benzamide,
*rac*-4-(3-methyl-pentanoylamino)-*N*-thiazol-2-yl-benzamide,
4-hexanoylamino-3-methyl-*N*-thiazol-2-yl-benzamide,
4-(2-cycloheptyl-acetylamino)-*N*-thiazol-2-yl-benzamide,
*rac*-3-methoxy-4-(3-methyl-pentanoylamino)-*N*-thiazol-2-yl-benzamide,
4-(2-cycloheptyl-acetylamino)-3-methoxy-*N*-thiazol-2-yl-benzamide,
*rac*-4-[2-(2-oxo-cyclopentyl)-acetylamino]-*N*-thiazol-2-yl-benzamide,
4-hexanoylamino-3-methoxy-*N*-thiazol-2-yl-benzamide,
3-methyl-4-(4-phenyl-butyrylamino)-*N*-thiazol-2-yl-benzamide,
4-(2-cyclohexyl-acetylamino)-*N*-thiazol-2-yl-benzamide,
*rac*-4-(2-bicyclo[2.2.1]hept-2-yl-acetylamino)-*N*-thiazol-2-yl-benzamide,
4-(3,3-dimethyl-butyrylamino)-*N*-(4,5-dimethyl-thiazol-2-yl)-benzamide,
4-(2-adamantan-1-yl-acetylamino)-*N*-thiazol-2-yl-benzamide,
4-(3-benzo[1,3]dioxol-5-yl-propionylamino)-3-methyl-*N*-thiazol-2-yl-benzamide,
4-(3-hydroxy-3-methyl-butyrylamino)-3-methoxy-*N*-thiazol-2-yl-benzamide,
4-(4-fluoro-benzoylamino)-3-methoxy-*N*-thiazol-2-yl-benzamide,
4-benzoylamino-*N*-thiazol-2-yl-benzamide,
thiophene-3-carboxylic acid [4-(thiazol-2-ylcarbamoyl)-phenyl]-amide,
*N*-thiazol-2-yl-4-(2-*o*-tolyl-acetylamino)-benzamide,
*N*-thiazol-2-yl-4-(2-thiophen-3-yl-acetylamino)-benzamide,
4-(2-cyclopentyl-acetylamino)-3-methyl-*N*-thiazol-2-yl-benzamide,
4-(3,3-dimethyl-butyrylamino)-*N*-thiazol-2-yl-benzamide,
4-(3,3-dimethyl-butyrylamino)-3-methyl-*N*-thiazol-2-yl-benzamide,
4-(3,3-dimethyl-butyrylamino)-2-methoxy-*N*-thiazol-2-yl-benzamide,
3-chloro-4-(3-methyl-butyrylamino)-*N*-thiazol-2-yl-benzamide,
3-bromo-4-(3,3-dimethyl-butyrylamino)-*N*-thiazol-2-yl-benzamide,
4-(3-methyl-butyrylamino)-*N*-thiazol-2-yl-benzamide,
3-bromo-4-(3-methyl-butyrylamino)-*N*-thiazol-2-yl-benzamide,
4-(2-cyclopentyl-acetylamino)-*N*-thiazol-2-yl-benzamide,
3-methyl-4-(3-methyl-butyrylamino)-*N*-thiazol-2-yl-benzamide,
3-chloro-4-(cyclopentanecarbonyl-amino)-*N*-thiazol-2-yl-benzamide,
3-chloro-4-(2-methyl-benzoylamino)-*N*-thiazol-2-yl-benzamide,
3-bromo-4-(cyclopentanecarbonyl-amino)-*N*-thiazol-2-yl-benzamide,
4-(cyclopentanecarbonyl-amino)-*N*-thiazol-2-yl-benzamide,
4-(cyclopentanecarbonyl-amino)-3-methyl-*N*-thiazol-2-yl-benzamide,
cycloheptanecarboxylic acid [2-bromo-4-(thiazol-2-ylcarbamoyl)-phenyl]-amide,
4-isobutyrylamino-2-methoxy-*N*-thiazol-2-yl-benzamide,
8-(3,3-dimethyl-butyrylamino)-2,3-dihydro-benzo[1,4]dioxine-5-carboxylic acid thiazol-2-ylamide,
3-bromo-4-butyrylamino-*N*-thiazol-2-yl-benzamide,
2-methoxy-4-(3-methyl-butyrylamino)-*N*-thiazol-2-yl-benzamide,
cycloheptanecarboxylic acid [4-(thiazol-2-ylcarbamoyl)-phenyl]-amide,
*rac*-2-methoxy-4-(2-methyl-butyrylamino)-*N*-thiazol-2-yl-benzamide,
4-(cyclopentanecarbonyl-amino)-2-methoxy-*N*-thiazol-2-yl-benzamide,
3-bromo-4-(2-methyl-benzoylamino)-*N*-thiazol-2-yl-benzamide,
3-chloro-4-(3,3-dimethyl-butyrylamino)-*N*-thiazol-2-yl-benzamide,
4-(2-cyclopentyl-acetylamino)-2-propoxy-*N*-thiazol-2-yl-benzamide,
4-(3,3-dimethyl-butyrylamino)-2-propoxy-*N*-thiazol-2-yl-benzamide,
4-(2-cyclopentyl-acetylamino)-3-fluoro-*N*-thiazol-2-yl-benzamide,
4-(3-methyl-butyrylamino)-2-propoxy-*N*-thiazol-2-yl-benzamide,
3-fluoro-4-(3-methyl-butyrylamino)-*N*-thiazol-2-yl-benzamide,
4-butyrylamino-3-fluoro-*N*-thiazol-2-yl-benzamide,
4-butyrylamino-2-propoxy-*N*-thiazol-2-yl-benzamide,

3-fluoro-4-(2-methyl-benzoylamino)-*N*-thiazol-2-yl-benzamide,
cycloheptanecarboxylic acid [2-fluoro-4-(thiazol-2-ylcarbamoyl)-phenyl]-amide,
4-(cyclopentanecarbonyl-amino)-3-fluoro-*N*-thiazol-2-yl-benzamide,
4-(3,3-dimethyl-butyrylamino)-2-(2-methoxy-ethoxy)-*N*-thiazol-2-yl-benzamide,
3-fluoro-4-(3-methyl-benzoylamino)-*N*-thiazol-2-yl-benzamide,
*rac*-3-fluoro-*N*-thiazol-2-yl-4-(3,5,5-trimethyl-hexanoylamino)-benzamide,
4-(2-cyclopentyl-acetylamino)-2-(2-methoxy-ethoxy)-*N*-thiazol-2-yl-benzamide,
4-(2-methyl-benzoyl)-3,4-dihydro-2H-benzo[1,4]oxazine-7-carboxylic acid thiazol-2-ylamide,
4-(3,3-dimethyl-butyrylamino)-3-fluoro-*N*-thiazol-2-yl-benzamide,
4-(3,3-dimethyl-butyrylamino)-2-methyl-*N*-thiazol-2-yl-benzamide,
5-chloro-4-(3,3-dimethyl-butyrylamino)-2-methoxy-*N*-thiazol-2-yl-benzamide,
4-(3,3-dimethyl-butyrylamino)-*N*-(5-methyl-thiazol-2-yl)-benzamide,
5-chloro-2-methoxy-4-(3-methyl-butyrylamino)-*N*-thiazol-2-yl-benzamide,
4-(2-methyl-benzoylamino)-*N*-(5-methyl-thiazol-2-yl)-benzamide,
1-(3,3-dimethyl-butyryl)-1,2,3,4-tetrahydro-quinoline-6-carboxylic acid thiazol-2-yl-amide,
5-chloro-2-methoxy-4-(2-methyl-benzoylamino)-*N*-thiazol-2-yl-benzamide,
1-(3-methyl-butyryl)-1,2,3,4-tetrahydro-quinoline-6-carboxylic acid thiazol-2-yl-amide,
1-(3,3-dimethyl-butyryl)-2,3-dihydro-1*H*-indole-5-carboxylic acid thiazol-2-ylamide,
4-[(3,3-dimethyl-butyryl)-methyl-amino]-*N*-thiazol-2-yl-benzamide,
4-[(2-cyclopentyl-acetyl)-propyl-amino]-*N*-thiazol-2-yl-benzamide,
2-(2-methoxy-ethoxy)-4-(3-methyl-butyrylamino)-*N*-thiazol-2-yl-benzamide,
*rac*-2-propoxy-*N*-thiazol-2-yl-4-(3,5,5-trimethyl-hexanoylamino)-benzamide,
*rac*-*N*-thiazol-2-yl-4-(3,5,5-dimethyl-hexanoylamino)-benzamide,
4-(3-cyclopentyl-propionylamino)-*N*-thiazol-2-yl-benzamide,
4-(2-cyclopentyl-acetylamino)-3-methoxy-*N*-thiazol-2-yl-benzamide,
cycloheptanecarboxylic acid [2-methyl-4-(thiazol-2-ylcarbamoyl)-phenyl]-amide,
3-methoxy-4-(3-phenyl-propionylamino)-*N*-thiazol-2-yl-benzamide,
cycloheptanecarboxylic acid [2-chloro-4-(thiazol-2-ylcarbamoyl)-phenyl]-amide,
4-[2-(3-methoxy-phenyl)-acetylamino]-3-methyl-*N*-thiazol-2-yl-benzamide,
3-bromo-4-(2-cyclopentyl-acetylamino)-*N*-thiazol-2-yl-benzamide,
4-butyrylamino-3-chloro-*N*-thiazol-2-yl-benzamide,
5-chloro-4-(2-cyclopentyl-acetylamino)-2-methoxy-*N*-thiazol-2-yl-benzamide,
5-chloro-4-(cyclopentanecarbonyl-amino)-2-methoxy-*N*-thiazol-2-yl-benzamide,
4-(cyclohexanecarbonyl-amino)-2-methoxy-*N*-thiazol-2-yl-benzamide,
2-methoxy-4-(4-methoxy-benzoylamino)-*N*-thiazol-2-yl-benzamide,
3-methoxy-4-phenylacetylamino-*N*-thiazol-2-yl-benzamide,
3-methyl-*N*-thiazol-2-yl-4-(2-thiophen-2-yl-acetylamino)-benzamide,
3-chloro-4-(2-cyclopentyl-acetylamino)-*N*-thiazol-2-yl-benzamide,
4-(4-methoxy-benzoylamino)-3-methyl-*N*-thiazol-2-yl-benzamide,
4-butyrylamino-3-methyl-*N*-thiazol-2-yl-benzamide,
4-(2-chloro-benzoylamino)-3-methyl-*N*-thiazol-2-yl-benzamide,
4-(2,5 di-chloro-benzoylamino)-3-methyl-*N*-thiazol-2-yl-benzamide,
4-(2-chloro-benzoylamino)-2-methoxy-*N*-thiazol-2-yl-benzamide,
4-(2-ethyl-butyrylamino)-2-methoxy-*N*-thiazol-2-yl-benzamide,
2-methoxy-4-(2-methyl-benzoylamino)-*N*-thiazol-2-yl-benzamide,
3-methyl-4-(3-phenyl-propionylamino)-*N*-thiazol-2-yl-benzamide,
4-(3,3-dimethyl-butyrylamino)-3-methoxy-*N*-thiazol-2-yl-benzamide,
*rac*-3-methyl-*N*-thiazol-2-yl-4-(3,5,5-trimethyl-hexanoylamino)-benzamide,
*rac*-2,3-dihydro-benzo[1,4]dioxine-2-carboxylic acid [2-methoxy-4-(thiazol-2-yl-carbamoyl)-phenyl]-amide,
4-(2,2-dimethyl-propionylamino)-3-methoxy-*N*-thiazol-2-yl-benzamide,
2-methoxy-4-(4-methyl-benzoylamino)-*N*-thiazol-2-yl-benzamide,
thiophene-2-carboxylic acid [3-methoxy-4-(thiazol-2-ylcarbamoyl)-phenyl]-amide,
4-(3-methoxy-benzoylamino)-*N*-thiazol-2-yl-benzamide,
8-(2-cyclopentyl-acetylamino)-2,3-dihydro-benzo[1,4]dioxine-5-carboxylic acid thiazol-2-ylamide,
6-(2-cyclopentyl-acetylamino)-biphenyl-3-carboxylic acid thiazol-2-ylamide,
4-(2-cyclopentyl-acetylamino)-3-(2-methoxy-ethoxy)-*N*-thiazol-2-yl-benzamide
4-(3,3-dimethyl-butyrylamino)-2-fluoro-*N*-thiazol-2-yl-benzamide,
2-chloro-4-(2-methyl-benzoylamino)-*N*-thiazol-2-yl-benzamide,

4-(2-fluoro-benzoylamino)-*N*-thiazol-2-yl-benzamide,
4-(2-methoxy-benzoylamino)-*N*-thiazol-2-yl-benzamide,
benzo[*b*]thiophene-2-carboxylic acid [2-methyl-4-(thiazol-2-ylcarbamoyl)-phenyl]-amide,
5-(3,3-dimethyl-butyrylamino)-biphenyl-2-carboxylic acid thiazol-2-ylamide,
N-(5-chloro-thiazol-2-yl)-4-(3,3-dimethyl-butyrylamino)-benzamide,
N-(5-chloro-thiazol-2-yl)-4-(3-methyl-butyrylamino)-benzamide,
N-(5-chloro-thiazol-2-yl)-4-(2-cyclopropyl-acetylamino)-benzamide,
4-butyrylamino-N-(5-chloro-thiazol-2-yl)-benzamide,
4-benzoylamino-*N*-(5-chloro-thiazol-2-yl)-benzamide,
3-fluoro-N-thiazol-2-yl-4-(4,4,4-trifluoro-3-methyl-butyrylamino)-benzamide,
4-(3,3-dimethyl-butyrylamino)-N-thiazol-2-yl-3-trifluoromethoxy-benzamide,
4-(3,3-dimethyl-butyrylamino)-3-methoxymethyl-N-thiazol-2-yl-benzamide,
4-(3,3-dimethyl-butyrylamino)-3-propoxy-N-thiazol-2-yl-benzamide,
3-chloro-4-(3,3-dimethyl-butyrylamino)-5-methyl-N-thiazol-2-yl-benzamide,
4-(3,3-dimethyl-butyrylamino)-3,5-difluoro-N-thiazol-2-yl-benzamide,
4-(3,3-dimethyl-butyrylamino)-3,5-dimethyl-N-thiazol-2-yl-benzamide,
4-(3,3-dimethyl-butyrylamino)-N-thiazol-2-yl-3-trifluoromethyl-benzamide,
3-chloro-4-(3,3-dimethyl-butyrylamino)-N-thiazol-2-yl-5-trifluoromethyl-benzamide,
*N*-(2,2-dimethyl-propyl)-*N'*-thiazol-2-yl-terephthalamide,
3,5-dichloro-4-(3,3-dimethyl-butyrylamino)-*N*-thiazol-2-yl-benzamide,
4-(3-*tert*-butyl-ureido)-*N*-thiazol-2-yl-benzamide, and
[4-(thiazol-2-ylcarbamoyl)-phenyl]-carbamic acid 2,2-dimethyl-propyl ester.

**21.** A compound according to any of claims 17-20 for use in medicine.

**22.** Use of a compound according to any of claims 17-20 for the manufacture of a medicament for treatment of a disease selected from the group consisting of: Parkinson's Disease, Alzheimer's Disease, Huntington's disease, epilepsia, cerebral ischemia, haemorrhagic stroke, neonatal ischemia and hypoxia, subarachnoid haemorrhage, traumatic brain injury, brain damage following cardiac arrest, depression and psychosis disorders.

**23.** The use according to claim 22, wherein the disease is Parkinson's Disease.

## Patentansprüche

**1.** Verwendung einer Verbindung der Formel (I):

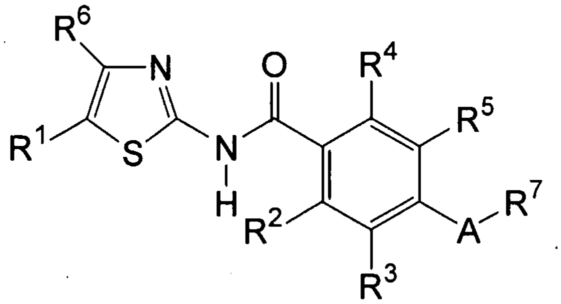

(I)

worin $R^1$ und $R^6$ unabhängig Wasserstoff, $C_{1-6}$-Alkyl oder Halogen sind;
$R^2$-$R^5$ unabhängig aus der Gruppe ausgewählt sind, die aus Wasserstoff, Halogen, Cyano, OH, $NH_2$, Nitro, $C_{1-6}$-Alkyl, Aryl, Aryl-$C_{1-6}$-alkyl, Heteroaryl-$C_{1-6}$-alkyl, $C_{3-8}$-Cycloalkyl, $C_{3-8}$-Cycloalkyl-$C_{1-6}$-alkyl, $C_{1-6}$-Alkoxy, Aryl-$C_{1-6}$-alkoxy, $C_{1-6}$-Alkylamino und Aryl-$C_{1-6}$-alkylamino besteht, worin jedes Alkyl, Alkoxy oder Aryl gegebenenfalls mit einem oder mehreren aus Halogen, Cyano, $C_{1-6}$-Alkyl, $C_{1-6}$-Alkoxy oder $C_{1-6}$-Alkoxy-$C_{1-6}$-alkoxy substituiert sein kann;
oder $R^4$ und $R^5$ zusammen X-$(CH_2)_n$-Y sind, worin X und Y unabhängig aus der Gruppe ausgewählt sind, die aus $CH_2$, NH und O besteht, n 1, 2 oder 3 ist und $R^2$ und $R^3$ wie oben definiert sind;
A *$NR^8$-CO, *CO-$NR^9$, *$NR^8$-CS oder *CS-$NR^9$ ist, worin $R^8$ und $R^9$ unabhängig aus der Gruppe ausgewählt sind, die aus Wasserstoff und $C_{1-6}$-Alkyl besteht, oder $R^8$ zusammen mit $R^3$ $C_{2-3}$-Alkylen oder $CH_2CH_2O$ ist, worin der

Sauerstoff an den Phenylring gebunden ist und das Symbol * anzeigt, daß das Atom an den Phenylring gebunden ist; und $R^7$ aus der Gruppe ausgewählt ist, die aus $C_{1-8}$-Alkyl, Aryl, Heteroaryl, Aryl-$C_{1-6}$-alkyl, Heteroaryl-$C_{1-6}$-alkyl, $C_{3-8}$-Cycloalkyl, $C_{3-8}$-Cycloalkyl-$C_{1-6}$-alkyl, $C_{1-6}$-Alkoxy, Aryl-$C_{1-6}$-alkoxy, Heteroaryl-$C_{1-6}$-alkoxy, $C_{1-6}$-Alkylamino, Aryl-$C_{1-6}$-alkylamino, Heteroaryl-$C_{1-6}$-alkylamino, Di-($C_{1-6}$-alkyl)-amino, 2,3-Dihydrobenzo[1,4]dioxin-2-yl oder Adamantan-1-yl-methyl besteht, worin jedes Alkyl und Cycloalkyl gegebenenfalls mit einem oder mehreren aus Halogen, Cyano, Hydroxy, Oxo, $C_{1-6}$-Alkoxy oder $NR^{10}R^{11}$ substituiert sein kann, worin $R^{10}$ und $R^{11}$ unabhängig Wasserstoff oder $C_{1-6}$-Alkyl sind oder $R^{10}$ und $R^{11}$ zusammen mit dem Stickstoff einen 5-, 6- oder 7-gliedrigen aliphatischen Ring bilden, der gegebenenfalls ein weiteres Heteroatom enthalten kann, das aus N und O ausgewählt ist, und jedes Aryl gegebenenfalls substituiert sein kann mit einem oder mehreren aus Halogen, Cyano, Hydroxy, Nitro, $C_{1-6}$-Alkyl, $C_{1-6}$-Alkoxy, $C_{1-6}$-Acyl, $C_{1-6}$-Acyloxy, $NR^{10}R^{11}$, worin $R^{10}$ und $R^{11}$ unabhängig Wasserstoff oder $C_{1-6}$-Alkyl sind oder $R^{10}$ und $R^{11}$ zusammen mit dem Stickstoff einen 5-, 6- oder 7-gliedrigen aliphatischen Ring bilden, der gegebenenfalls ein weiteres Heteroatom enthalten kann, das aus N und O ausgewählt ist, oder einer Gruppe Z-$(CH_2)_m$-W, worin Z und W an zwei benachbarte Kohlenstoffatome gebunden sind und unabhängig aus der Gruppe ausgewählt sind, die aus $CH_2$, NH und O besteht, und m 1, 2 oder 3 ist, mit der Maßgabe, daß dann, wenn $R^7$ an Stickstoff gebunden ist, $R^7$ nicht $C_{1-6}$-Alkoxy, Aryl-$C_{1-6}$-alkoxy, Heteroaryl-$C_{1-6}$-alkoxy, $C_{1-6}$-Alkylamino, Aryl-$C_{1-6}$-alkylamino, Heteroaryl-$C_{1-6}$-alkylamino oder Di-($C_{1-6}$-alkyl)-amino ist; und von pharmazeutisch akzeptablen Säureadditionssalzen davon;
zur Herstellung eines Medikaments zur Behandlung einer Krankheit, an der ein $A_{2A}$-Rezeptor beteiligt ist.

**2.** Verwendung einer Verbindung gemäß Anspruch 1, worin die Krankheit aus der Gruppe ausgewählt ist, die aus Parkinson-Krankheit, Alzheimer-Krankheit, Huntington-Krankheit, Epilepsie, zerebraler Ischämie, hämorrhagischem Schlag, neonataler Ischämie und Hypoxie, subarachnoidaler Blutung, traumatischer Hirnverletzung, Hirnverletzung nach Herzstillstand besteht, und zur Behandlung von Depression und Psychosestörungen.

**3.** Verwendung einer Verbindung gemäß Anspruch 2, worin die Krankheit Parkinson-Krankheit ist.

**4.** Verwendung einer Verbindung gemäß einem der Ansprüche 1 bis 3, worin A *$NR^8$-CO oder *CO-$NR^9$ ist.

**5.** Verwendung einer Verbindung gemäß Anspruch 4, worin A *$NR^8$-CO ist.

**6.** Verwendung einer Verbindung gemäß einem der Ansprüche 1 bis 5, worin $R^7$ aus der Gruppe ausgewählt ist, die aus $C_{1-8}$-Alkyl, bevorzugt $C_{3-8}$-Alkyl und noch mehr bevorzugt $C_{4-8}$-Alkyl, das in der β-Position verzweigt ist, $C_{3-8}$-Cycloalkyl-methyl, $C_{3-8}$-Cycloalkyl, Methylphenyl, Methoxybenzyl und Thiophen-2-yl-methyl besteht, worin jedes Alkyl oder Cycloalkyl unsubstituiert oder mit Oxo substituiert sein kann.

**7.** Verwendung einer Verbindung gemäß einem der Ansprüche 1 bis 6, worin $R^8$ Wasserstoff ist.

**8.** Verwendung einer Verbindung gemäß einem der Ansprüche 1 bis 7, worin $R^9$ Wasserstoff ist.

**9.** Verwendung einer Verbindung gemäß einem der Ansprüche 1 bis 8, worin $R^6$ Wasserstoff ist.

**10.** Verwendung einer Verbindung gemäß einem der Ansprüche 1 bis 9, worin $R^1$ Wasserstoff, Methyl oder Chlor ist, bevorzugt Wasserstoff.

**11.** Verwendung einer Verbindung gemäß einem der Ansprüche 1 bis 10, worin $R^2$ bis $R^5$ unabhängig aus der Gruppe ausgewählt sind, die aus Wasserstoff, Halogen, $C_{1-6}$-Alkyl, bevorzugt Methyl, $C_{1-6}$-Alkoxy und $C_{1-6}$-Alkoxy-$C_{1-6}$-alkoxy, bevorzugt 2-Methoxy-ethoxy, besteht.

**12.** Verwendung einer Verbindung gemäß Anspruch 11, worin $R^2$ und $R^4$ unabhängig aus der Gruppe ausgewählt sind, die aus Wasserstoff, $C_{1-6}$-Alkoxy und $C_{1-6}$-alkoxy-$C_{1-6}$-alkoxy, bevorzugt 2-Methoxy-ethoxy, besteht.

**13.** Verwendung einer Verbindung gemäß Anspruch 11 oder 12, worin $R^3$ und $R^5$ unabhängig aus der Gruppe ausgewählt sind, die aus Wasserstoff, Halogen, $C_{1-6}$-Alkyl, bevorzugt Methyl, $C_{1-6}$-Alkoxy, bevorzugt Methoxy, $C_{1-6}$-Alkoxy-$C_{1-6}$-alkoxy, bevorzugt 2-Methoxy-ethoxy, Trifluormethyl und Trifluormethoxy besteht.

**14.** Verwendung einer Verbindung gemäß Anspruch 1, worin die Verbindung aus der Gruppe ausgewählt ist, die aus den folgenden besteht:

4-Butyrylamino-N-thiazol-2-yl-benzamid,
rac-3-Methoxy-4-(3-methyl-4-oxo-pentanoylamino)-N-thiazol-2-yl-benzamid,
rac-4-(3-Methyl-pentanoylamino)-N-thiazol-2-yl-benzamid,
4-Hexanoylamino-3-methyl-N-thiazol-2-yl-benzamid,
4-(2-Cycloheptyl-acetylamino)-N-thiazol-2-yl-benzamid,
rac-3-Methoxy-4-(3-methyl-pentanoylamino)-N-thiazol-2-yl-benzamid,
4-(2-Cycloheptyl-acetylamino)-3-methoxy-N-thiazol-2-yl-benzamid,
rac-4-[2-(2-Oxo-cyclopentyl)-acetylamino]-N-thiazol-2-yl-benzamid,
4-Hexanoylamino-3-methoxy-N-thiazol-2-yl-benzamid,
3-Methyl-4-(4-phenyl-butyrylamino)-N-thiazol-2-yl-benzamid,
4-(2-Cyclohexyl-acetylamino)-N-thiazol-2-yl-benzamid,
rac-4-(2-Bicyclo[2.2.1]hept-2-yl-acetylamino)-N-thiazol-2-yl-benzamid,
4-(3,3-Dimethyl-butyrylamino)-N-(4,5-dimethyl-thiazol-2-yl)-benzamid,
4-(2-Adamantan-1-yl-acetylamino)-N-thiazol-2-yl-benzamid,
4-(3-Benzo[1,3]dioxol-5-yl-propionylamino)-3-methyl-N-thiazol-2-yl-benzamid,
4-(3-Hydroxy-3-methyl-butyrylamino)-3-methoxy-N-thiazol-2-yl-benzamid,
4-(4-Fluor-benzoylamino)-3-methoxy-N-thiazol-2-yl-benzamid,
4-Benzoylamino-N-thiazol-2-yl-benzamid,
Thiophen-3-carbonsäure-[4-(thiazol-2-yl-carbamoyl)-phenyl] -amid,
N-Thiazol-2-yl-4-(2-o-tolyl-acetylamino)-benzamid,
N-Thiazol-2-yl-4-(2-thiophen-3-yl-acetylamino)-benzamid,
4-(2-Cyclopentyl-acetylamino)-3-methyl-N-thiazol-2-yl-benzamid,
4-(3,3-Dimethyl-butyrylamino)-N-thiazol-2-yl-benzamid,
4-(3,3-Dimethyl-butyrylamino)-3-methyl-N-thiazol-2-yl-benzamid,
4-(3,3-Dimethyl-butyrylamino)-2-methoxy-N-thiazol-2-yl-benzamid,
3-Chlor-4-(3-methyl-butyrylamino)-N-thiazol-2-yl-benzamid,
3-Brom-4-(3,3-dimethyl-butyrylamino)-N-thiazol-2-yl-benzamid,
4-(3-Methyl-butyrylamino)-N-thiazol-2-yl-benzamid,
3-Brom-4-(3-methyl-butyrylamino)-N-thiazol-2-yl-benzamid,
4-(2-Cyclopentyl-acetylamino)-N-thiazol-2-yl-benzamid,
3-Methyl-4-(3-methyl-butyrylamino)-N-thiazol-2-yl-benzamid,
3-Chlor-4-(cyclopentancarbonyl-amino)-N-thiazol-2-yl-benzamid,
3-Chlor-4-(2-methyl-benzoylamino)-N-thiazol-2-yl-benzamid,
3-Brom-4-(cyclopentancarbonyl-amino)-N-thiazol-2-yl-benzamid,
4-(Cyclopentancarbonyl-amino)-N-thiazol-2-yl-benzamid,
4-(Cyclopentancarbonyl-amino)-3-methyl-N-thiazol-2-yl-benzamid,
Cycloheptancarbonsäure-[2-brom-4-(thiazol-2-yl-carbamoyl)-phenyl]-amid,
4-Isobutyrylamino-2-methoxy-N-thiazol-2-yl-benzamid,
8-(3,3-Dimethyl-butyrylamino)-2,3-dihydro-benzo[1,4]dioxin-5-carbonsäure-thiazol-2-yl-amid,
3-Brom-4-butyrylamino-N-thiazol-2-yl-benzamid,
2-Methoxy-4-(3-methyl-butyrylamino)-N-thiazol-2-yl-benzamid,
Cycloheptancarbonsäure-[4-(thiazol-2-yl-carbamoyl)-phenyl]-amid,
rac-2-Methoxy-4-(2-methyl-butyrylamino)-N-thiazol-2-yl-benzamid,
4-(Cyclopentancarbonyl-amino)-2-methoxy-N-thiazol-2-yl-benzamid,
3-Brom-4-(2-methyl-benzoylamino)-N-thiazol-2-yl-benzamid,
3-Chlor-4-(3,3-dimethyl-butyrylamino)-N-thiazol-2-yl-benzamid,
4-(2-Cyclopentyl-acetylamino)-2-propoxy-N-thiazol-2-yl-benzamid,
4-(3,3-Dimethyl-butyrylamino)-2-propoxy-N-thiazol-2-yl-benzamid,
4-(2-Cyclopentyl-acetylamino)-3-fluor-N-thiazol-2-yl-benzamid,
4-(3-Methyl-butyrylamino)-2-propoxy-N-thiazol-2-yl-benzamid,
3-Fluor-4-(3-methyl-butyrylamino)-N-thiazol-2-yl-benzamid,
4-Butyrylamino-3-fluor-N-thiazol-2-yl-benzamid,
4-Butyrylamino-2-propoxy-N-thiazol-2-yl-benzamid,
3-Fluor-4-(2-methyl-benzoylamino)-N-thiazol-2-yl-benzamid,
Cycloheptancarbonsäure-[2-fluor-4-(thiazol-2-yl-carbamoyl)-phenyl]-amid,
4-(Cyclopentancarbonyl-amino)-3-fluor-N-thiazol-2-yl-benzamid,
4-(3,3-Dimethyl-butyrylamino)-2-(2-methoxy-ethoxy)-N-thiazol-2-yl-benzamid,
3-Fluor-4-(3-methyl-benzoylamino)-N-thiazol-2-yl-benzamid,

rac-3-Fluor-N-thiazol-2-yl-4-(3,5,5-trimethyl-hexanoylamino)-benzamid,
4-(2-Cyclopentyl-acetylamino)-2-(2-methoxy-ethoxy)-N-thiazol-2-yl-benzamid,
4-(2-Methyl-benzoyl)-3,4-dihydro-2H-benzo[1,4]oxazin-7-carbonsäure-thiazol-2-yl-amid,
4-(3,3-Dimethyl-butyrylamino)-3-fluor-N-thiazol-2-yl-benzamid,
4-(3,3-Dimethyl-butyrylamino)-2-methyl-N-thiazol-2-yl-benzamid,
5-Chlor-4-(3,3-dimethyl-butyrylamino)-2-methoxy-N-thiazol-2-yl-benzamid,
4-(3,3-Dimethyl-butyrylamino)-N-(5-methyl-thiazol-2-yl)-benzamid,
5-Chlor-2-methoxy-4-(3-methyl-butyrylamino)-N-thiazol-2-yl-benzamid,
4-(2-Methyl-benzoylamino)-N-(5-methyl-thiazol-2-yl)-benzamid,
1-(3,3-Dimethyl-butyryl)-1,2,3,4-tetrahydro-chinolin-6-carbonsäure-thiazol-2-yl-amid,
5-Chlor-2-methoxy-4-(2-methyl-benzoylamino)-N-thiazol-2-yl-benzamid,
1-(3-Methyl-butyryl)-1,2,3,4-tetrahydro-chinolin-6-carbonsäure-thiazol-2-yl-amid,
1-(3,3-Dimethyl-butyryl)-2,3-dihydro-1H-indol-5-carbonsäure-thiazol-2-yl-amid,
4-[(3,3-Dimethyl-butyryl)-methyl-amino]-N-thiazol-2-yl-, benzamid,
4-[(2-Cyclopentyl-acetyl)-propyl-amino]-N-thiazol-2-yl-benzamid,
2-(2-Methoxy-ethoxy)-4-(3-methyl-butyrylamino)-N-thiazol-2-yl-benzamid,
rac-2-Propoxy-N-thiazol-2-yl-4-(3,5,5-trimethyl-hexanoylamino)-benzamid,
rac-N-thiazol-2-yl-4-(3,5,5-trimethyl-hexanoylamino)-benzamid,
4-(3-Cyclopentyl-propionylamino)-N-thiazol-2-yl-benzamid,
4-(2-Cyclopentyl-acetylamino)-3-methoxy-N-thiazol-2-yl-benzamid,
Cycloheptancarbonsäure-[2-methyl-4-(thiazol-2-yl-carbamoyl)-phenyl]-amid,
3-Methoxy-4-(3-phenyl-propionylamino)-N-thiazol-2-yl-benzamid,
Cycloheptancarbonsäure-[2-chlor-4-(thiazol-2-yl-carbamoyl)-phenyl]-amid,
4-[2-(3-Methoxy-phenyl)-acetylamino]-3-methyl-N-thiazol-2-yl-benzamid,
3-Brom-4-(2-cyclopentyl-acetylamino)-N-thiazol-2-yl-benzamid,
4-Butyrylamino-3-chlor-N-thiazol-2-yl-benzamid,
5-Chlor-4-(2-cyclopentyl-acetylamino)-2-methoxy-N-thiazol-2-yl-benzamid,
5-Chlor-4-(cyclopentancarbonyl-amino)-2-methoxy-N-thiazol-2-yl-benzamid,
4-(Cyclohexancarbonyl-amino)-2-methoxy-N-thiazol-2-yl-benzamid,
2-Methoxy-4-(4-methoxy-benzoylamino)-N-thiazol-2-yl-benzamid,
3-Methoxy-4-phenylacetylamino-N-thiazol-2-yl-benzamid,
3-Methyl-N-thiazol-2-yl-4-(2-thiophen-2-yl-acetylamino)-benzamid,
3-Chlor-4-(2-cyclopentyl-acetylamino)-N-thiazol-2-yl-benzamid,
4-(4-Methoxy-benzoylamino)-3-methyl-N-thiazol-2-yl-benzamid,
4-Butyrylamino-3-methyl-N-thiazol-2-yl-benzamid,
4-(2-Chlor-benzoylamino)-3-methyl-N-thiazol-2-yl-benzamid,
4-(2,5-Dichlor-benzoylamino)-3-methyl-N-thiazol-2-yl-benzamid,
4-(2-Chlor-benzoylamino)-2-methoxy-N-thiazol-2-yl-benzamid,
4-(2-Ethyl-butyrylamino)-2-methoxy-N-thiazol-2-yl-benzamid,
2-Methoxy-4-(2-methyl-benzoylamino)-N-thiazol-2-yl-benzamid,
3-Methyl-4-(3-phenyl-propionylamino)-N-thiazol-2-yl-benzamid,
4-(3,3-Dimethyl-butyrylamino)-3-methoxy-N-thiazol-2-yl-benzamid,
rac-3-Methyl-N-thiazol-2-yl-4-(3,5,5-trimethyl-hexanoylamino)-benzamid,
rac-2,3-Dihydro-benzo[1,4]dioxin-2-carbonsäure-[2-methoxy-4-(thiazol-2-yl-carbamoyl)-phenyl]-amid,
4-(2,2-Dimethyl-propionylamino)-3-methoxy-N-thiazol-2-yl-benzamid,
2-Methoxy-4-(4-methyl-benzoylamino)-N-thiazol-2-yl-benzamid,
Thiophen-2-carbonsäure-[3-methoxy-4-(thiazol-2-yl-carbamoyl)-phenyl]-amid,
4-(3-Methoxy-benzoylamino)-N-thiazol-2-yl-benzamid,
8-(2-Cyclopentyl-acetylamino)-2,3-dihydro-benzo[1,4]dioxin-5-carbonsäure-thiazol-2-yl-amid,
6-(2-Cyclopentyl-acetylamino)-biphenyl-3-carbonsäure-thiazol-2-yl-amid,
4-(2-Cyclopentyl-acetylamino)-3-(2-methoxy-ethoxy)-N-thiazol-2-yl-benzamid,
4-(3,3-Dimethyl-butyrylamino)-2-fluor-N-thiazol-2-yl-benzamid,
2-Chlor-4-(2-methyl-benzoylamino)-N-thiazol-2-yl-benzamid,
4-(2-Fluor-benzoylamino)-N-thiazol-2-yl-benzamid,
4-(2-Methoxy-benzoylamino)-N-thiazol-2-yl-benzamid,
Benzo[b]thiophen-2-carbonsäure-[2-methyl-4-(thiazol-2-yl-carbamoyl)-phenyl]-amid,
5-(3,3-Dimethyl-butyrylamino)-biphenyl-2-carbonsäure-thiazol-2-yl-amid,
N-(5-Chlor-thiazol-2-yl)-4-(3,3-dimethyl-butyrylamino)-benzamid,

N-(5-Chlor-thiazol-2-yl)-4-(3-methyl-butyrylamino)-benzamid,
N-(5-Chlor-thiazol-2-yl)-4-(2-cyclopropyl-acetylamino)-benzamid,
4-Butyrylamino-N-(5-chlor-thiazol-2-yl)-benzamid,
4-Benzoylamino-N-(5-chlor-thiazol-2-yl)-benzamid,
3-Fluor-N-thiazol-2-yl-4-(4,4,4-trifluor-3-methyl-butyrylamino)-benzamid,
4-(3,3-Dimethyl-butyrylamino)-N-thiazol-2-yl-3-trifluormethoxy-benzamid,
4-(3,3-Dimethyl-butyrylamino)-3-methoxymethyl-N-thiazol-2-yl-benzamid,
4-(3,3-Dimethyl-butyrylamino)-3-propoxy-N-thiazol-2-yl-benzamid,
3-Chlor-4-(3,3-dimethyl-butyrylamino)-5-methyl-N-thiazol-2-yl-benzamid,
4-(3,3-Dimethyl-butyrylamino)-3,5-difluor-N-thiazol-2-yl-benzamid,
4-(3,3-Dimethyl-butyrylamino)-3,5-dimethyl-N-thiazol-2-yl-benzamid,
4-(3,3-Dimethyl-butyrylamino)-N-thiazol-2-yl-3-trifluormethyl-benzamid,
3-Chlor-4-(3,3-dimethyl-butyrylamino)-N-thiazol-2-yl-5-trifluormethyl-benzamid,
N-(2,2-Dimethyl-propyl)-N'-thiazol-2-yl-terephthalamid,
3,5-Dichlor-4-(3,3-dimethyl-butyrylamino)-N-thiazol-2-yl-benzamid,
4-(3-tert-Butyl-ureido)-N-thiazol-2-yl-benzamid und
[4-(Thiazol-2-yl-carbamoyl)-phenyl]-carbaminsäure-2,2-dimethyl-propylester.

**15.** Pharmazeutische Zusammensetzung, die eine Verbindung der Formel (I) gemäß einem der Ansprüche 1 bis 14 umfaßt, mit der Maßgabe, daß dann, wenn A *$NR^8$-CO ist und $R^1$-$R^6$ und $R^8$ alle Wasserstoff sind, $R^7$ nicht Thiophen-2-yl ist;
und mit der Maßgabe, daß dann, wenn A *$NR^8$-CO ist, $R^2$-$R^6$ und $R^8$ alle Wasserstoff sind und $R^1$ i-Propyl ist, $R^7$ nicht Methyl oder Benzyl ist;
und mit der Maßgabe, daß dann, wenn A *$NR^8$-CO ist, $R^2$, $R^4$-$R^6$ und $R^8$ alle Wasserstoff sind, $R^3$ Iod ist und $R^1$ i-Propyl ist, $R^7$ nicht Methyl ist;
und mit der Maßgabe, daß dann, wenn A *$NR^8$-CO ist, $R^1$, $R^3$-$R^6$ und $R^8$ alle Wasserstoff sind und $R^2$ Hydroxy ist, $R^7$ nicht Methyl oder Ethoxy ist.

**16.** Zusammensetzung gemäß Anspruch 15, worin die Verbindung ein $A_{2A}$-Rezeptorantagonist mit einer humanen $A_{2A}$-Bindungsaffinität ($K_i$) von 200 nM oder weniger ist.

**17.** Verbindung der Formel (I) gemäß einem der Ansprüche 1 bis 14, mit der Maßgabe, daß dann, wenn A *$NR^8$-CO ist und $R^1$-$R^6$ und $R^8$ alle Wasserstoff sind, $R^7$ nicht aus der Gruppe ausgewählt ist, die aus $C_{1-4}$-Alkyl, Pentan-3-yl, Trifluormethyl, Pyrimidyl, Furan-2-yl, Thiophen-2-yl, substituiertem oder unsubstituiertem Phenyl oder substituiertem oder unsubstituiertem Benzyl besteht;
und mit der Maßgabe, daß dann, wenn A *$NR^8$-CO ist, $R^2$-$R^6$ und $R^8$ alle Wasserstoff sind und $R^1$ i-Propyl ist, $R^7$ nicht Methyl oder Benzyl ist;
und mit der Maßgabe, daß dann, wenn A *$NR^8$-CO ist, $R^2$, $R^4$-$R^6$ und $R^8$ alle Wasserstoff sind, $R^3$ Iod ist und $R^1$ i-Propyl ist, $R^7$ nicht Methyl ist;
und mit der Maßgabe, daß dann, wenn A *$NR^8$-CO ist, $R^1$, $R^3$-$R^6$ und $R^8$ alle Wasserstoff sind und $R^2$ Hydroxy ist, $R^7$ nicht Methyl oder Ethoxy ist;
und mit der Maßgabe, daß dann, wenn A *$NR^8$-CO ist, $R^2$, $R^4$-$R^6$ und $R^8$ alle Wasserstoff sind, $R^2$ Nitro ist und $R^7$ Methyl ist, $R^1$ nicht Wasserstoff oder Methyl ist;
und mit der Maßgabe, daß dann, wenn A *CO-$NR^9$ ist, $R^1$, $R^6$ und $R^9$ alle Wasserstoff sind und $R^7$ Thiazol-2-yl ist, $R^2$-$R^5$ nicht alle Wasserstoff oder alle Fluor sind;
und mit der Maßgabe, daß dann, wenn A *CO-$NR^9$ ist, $R^2$-$R^5$ und $R^9$ alle Wasserstoff sind und $R^6$ Methyl ist, $R^1$ nicht Wasserstoff sein kann, wenn $R^7$ 4-Methyl-thiazol-2-yl ist, und $R^1$ nicht Methyl sein kann, wenn $R^7$ 4,5-Dimethyl-thiazol-2-yl ist.

**18.** Verbindung gemäß Anspruch 17, worin die Verbindung in Form eines Salzes ist.

**19.** Verbindung gemäß Anspruch 17 oder 18, worin die Verbindung ein $A_{2A}$-Rezeptorantagonist mit einer humanen $A_{2A}$-Bindungsaffinität ($K_i$) von 200 nM oder weniger ist.

**20.** Verbindung, die aus der Gruppe ausgewählt ist, die aus:

4-Butyrylamino-N-thiazol-2-yl-benzamid,
rac-3-Methoxy-4-(3-methyl-4-oxo-pentanoylamino)-N-thiazol-2-yl-benzamid,

rac-4-(3-Methyl-pentanoylamino)-N-thiazol-2-yl-benzamid,
4-Hexanoylamino-3-methyl-N-thiazol-2-yl-benzamid,
4-(2-Cycloheptyl-acetylamino)-N-thiazol-2-yl-benzamid,
rac-3-Methoxy-4-(3-methyl-pentanoylamino)-N-thiazol-2-yl-benzamid,
4-(2-Cycloheptyl-acetylamino)-3-methoxy-N-thiazol-2-yl-benzamid,
rac-4-[2-(2-Oxo-cyclopentyl)-acetylamino]-N-thiazol-2-yl-benzamid,
4-Hexanoylamino-3-methoxy-N-thiazol-2-yl-benzamid,
3-Methyl-4-(4-phenyl-butyrylamino)-N-thiazol-2-yl-benzamid,
4-(2-Cyclohexyl-acetylamino)-N-thiazol-2-yl-benzamid,
rac-4-(2-Bicyclo[2.2.1]hept-2-yl-acetylamino)-N-thiazol-2-yl-benzamid,
4-(3,3-Dimethyl-butyrylamino)-N-(4,5-dimethyl-thiazol-2-yl)-benzamid,
4-(2-Adamantan-1-yl-acetylamino)-N-thiazol-2-yl-benzamid,
4-(3-Benzo[1,3]dioxol-5-yl-propionylamino)-3-methyl-N-thiazol-2-yl-benzamid,
4-(3-Hydroxy-3-methyl-butyrylamino)-3-methoxy-N-thiazol-2-yl-benzamid,
4-(4-Fluor-benzoylamino)-3-methoxy-N-thiazol-2-yl-benzamid,
4-Benzoylamino-N-thiazol-2-yl-benzamid,
Thiophen-3-carbonsäure-[4-(thiazol-2-yl-carbamoyl)-phenyl]-amid,
N-Thiazol-2-yl-4-(2-o-tolyl-acetylamino)-benzamid,
N-Thiazol-2-yl-4-(2-thiophen-3-yl-acetylamino)-benzamid,
4-(2-Cyclopentyl-acetylamino)-3-methyl-N-thiazol-2-yl-benzamid,
4-(3,3-Dimethyl-butyrylamino)-N-thiazol-2-yl-benzamid,
4-(3,3-Dimethyl-butyrylamino)-3-methyl-N-thiazol-2-yl-benzamid,
4-(3,3-Dimethyl-butyrylamino)-2-methoxy-N-thiazol-2-yl-benzamid,
3-Chlor-4-(3-methyl-butyrylamino)-N-thiazol-2-yl-benzamid,
3-Brom-4-(3,3-dimethyl-butyrylamino)-N-thiazol-2-yl-benzamid,
4-(3-Methyl-butyrylamino)-N-thiazol-2-yl-benzamid,
3-Brom-4-(3-methyl-butyrylamino)-N-thiazol-2-yl-benzamid,
4-(2-Cyclopentyl-acetylamino)-N-thiazol-2-yl-benzamid,
3-Methyl-4-(3-methyl-butyrylamino)-N-thiazol-2-yl-benzamid,
3-Chlor-4-(cyclopentancarbonyl-amino)-N-thiazol-2-yl-benzamid,
3-Chlor-4-(2-methyl-benzoylamino)-N-thiazol-2-yl-benzamid,
3-Brom-4-(cyclopentancarbonyl-amino)-N-thiazol-2-yl-benzamid,
4-(Cyclopentancarbonyl-amino)-N-thiazol-2-yl-benzamid,
4-(Cyclopentancarbonyl-amino)-3-methyl-N-thiazol-2-yl-benzamid,
Cycloheptancarbonsäure-[2-brom-4-(thiazol-2-yl-carbamoyl)-phenyl]-amid,
4-Isobutyrylamino-2-methoxy-N-thiazol-2-yl-benzamid,
8-(3,3-Dimethyl-butyrylamino)-2,3-dihydro-benzo[1,4]dioxin-5-carbonsäure-thiazol-2-yl-amid,
3-Brom-4-butyrylamino-N-thiazol-2-yl-benzamid,
2-Methoxy-4-(3-methyl-butyrylamino)-N-thiazol-2-yl-benzamid,
Cycloheptancarbonsäure-[4-(thiazol-2-yl-carbamoyl)-phenyl]-amid,
rac-2-Methoxy-4-(2-methyl-butyrylamino)-N-thiazol-2-yl-benzamid,
4-(Cyclopentancarbonyl-amino)-2-methoxy-N-thiazol-2-yl-benzamid,
3-Brom-4-(2-methyl-benzoylamino)-N-thiazol-2-yl-benzamid,
3-Chlor-4-(3,3-dimethyl-butyrylamino)-N-thiazol-2-yl-benzamid,
4-(2-Cyclopentyl-acetylamino)-2-propoxy-N-thiazol-2-yl-benzamid,
4-(3,3-Dimethyl-butyrylamino)-2-propoxy-N-thiazol-2-yl-benzamid,
4-(2-Cyclopentyl-acetylamino)-3-fluor-N-thiazol-2-yl-benzamid,
4-(3-Methyl-butyrylamino)-2-propoxy-N-thiazol-2-yl-benzamid,
3-Fluor-4-(3-methyl-butyrylamino)-N-thiazol-2-yl-benzamid,
4-Butyrylamino-3-fluor-N-thiazol-2-yl-benzamid,
4-Butyrylamino-2-propoxy-N-thiazol-2-yl-benzamid,
3-Fluor-4-(2-methyl-benzoylamino)-N-thiazol-2-yl-benzamid,
Cycloheptancarbonsäure-[2-fluor-4-(thiazol-2-yl-carbamoyl)-phenyl]-amid,
4-(Cyclopentancarbonyl-amino)-3-fluor-N-thiazol-2-yl-benzamid,
4-(3,3-Dimethyl-butyrylamino)-2-(2-methoxy-ethoxy)-N-thiazol-2-yl-benzamid,
3-Fluor-4-(3-methyl-benzoylamino)-N-thiazol-2-yl-benzamid,
rac-3-Fluor-N-thiazol-2-yl-4-(3,5,5-trimethyl-hexanoylamino)-benzamid,
4-(2-Cyclopentyl-acetylamino)-2-(2-methoxy-ethoxy)-N-thiazol-2-yl-benzamid,

4-(2-Methyl-benzoyl)-3,4-dihydro-2H-benzo[1,4]oxazin-7-carbonsäure-thiazol-2-yl-amid,
4-(3,3-Dimethyl-butyrylamino)-3-fluor-N-thiazol-2-yl-benzamid,
4-(3,3-Dimethyl-butyrylamino)-2-methyl-N-thiazol-2-yl-benzamid,
5-Chlor-4-(3,3-dimethyl-butyrylamino)-2-methoxy-N-thiazol-2-yl-benzamid,
4-(3,3-Dimethyl-butyrylamino)-N-(5-methyl-thiazol-2-yl)-benzamid,
5-Chlor-2-methoxy-4-(3-methyl-butyrylamino)-N-thiazol-2-yl-benzamid,
4-(2-Methyl-benzoylamino)-N-(5-methyl-thiazol-2-yl)-benzamid,
1-(3,3-Dimethyl-butyryl)-1,2,3,4-tetrahydro-chinolin-6-carbonsäure-thiazol-2-yl-amid,
5-Chlor-2-methoxy-4-(2-methyl-benzoylamino)-N-thiazol-2-yl-benzamid,
1-(3-Methyl-butyryl)-1,2,3,4-tetrahydro-chinolin-6-carbonsäure-thiazol-2-yl-amid,
1-(3,3-Dimethyl-butyryl)-2,3-dihydro-1H-indol-5-carbonsäure-thiazol-2-yl-amid,
4-[(3,3-Dimethyl-butyryl)-methyl-amino]-N-thiazol-2-yl-benzamid,
4-[(2-Cyclopentyl-acetyl)-propyl-amino]-N-thiazol-2-yl-benzamid,
2-(2-Methoxy-ethoxy)-4-(3-methyl-butyrylamino)-N-thiazol-2-yl-benzamid,
rac-2-Propoxy-N-thiazol-2-yl-4-(3,5,5-trimethyl-hexanoylamino)-benzamid,
rac-N-thiazol-2-yl-4-(3,5,5-trimethyl-hexanoylamino)-benzamid,
4-(3-Cyclopentyl-propionylamino)-N-thiazol-2-yl-benzamid,
4-(2-Cyclopentyl-acetylamino)-3-methoxy-N-thiazol-2-yl-benzamid,
Cycloheptancarbonsäure-[2-methyl-4-(thiazol-2-yl-carbamoyl)-phenyl]-amid,
3-Methoxy-4-(3-phenyl-propionylamino)-N-thiazol-2-yl-benzamid,
Cycloheptancarbonsäure-[2-chlor-4-(thiazol-2-yl-carbamoyl)-phenyl]-amid,
4-[2-(3-Methoxy-phenyl)-acetylamino]-3-methyl-N-thiazol-2-yl-benzamid,
3-Brom-4-(2-cyclopentyl-acetylamino)-N-thiazol-2-yl-benzamid,
4-Butyrylamino-3-chlor-N-thiazol-2-yl-benzamid,
5-Chlor-4-(2-cyclopentyl-acetylamino)-2-methoxy-N-thiazol-2-yl-benzamid,
5-Chlor-4-(cyclopentancarbonyl-amino)-2-methoxy-N-thiazol-2-yl-benzamid,
4-(Cyclohexancarbonyl-amino)-2-methoxy-N-thiazol-2-yl-benzamid,
2-Methoxy-4-(4-methoxy-benzoylamino)-N-thiazol-2-yl-benzamid,
3-Methoxy-4-phenylacetylamino-N-thiazol-2-yl-benzamid,
3-Methyl-N-thiazol-2-yl-4-(2-thiophen-2-yl-acetylamino)-benzamid,
3-Chlor-4-(2-cyclopentyl-acetylamino)-N-thiazol-2-yl-benzamid,
4-(4-Methoxy-benzoylamino)-3-methyl-N-thiazol-2-yl-benzamid,
4-Butyrylamino-3-methyl-N-thiazol-2-yl-benzamid,
4-(2-Chlor-benzoylamino)-3-methyl-N-thiazol-2-yl-benzamid,
4-(2,5-Dichlor-benzoylamino)-3-methyl-N-thiazol-2-yl-benzamid,
4-(2-Chlor-benzoylamino)-2-methoxy-N-thiazol-2-yl-benzamid,
4-(2-Ethyl-butyrylamino)-2-methoxy-N-thiazol-2-yl-benzamid,
2-Methoxy-4-(2-methyl-benzoylamino)-N-thiazol-2-yl-benzamid,
3-Methyl-4-(3-phenyl-propionylamino)-N-thiazol-2-yl-benzamid,
4-(3,3-Dimethyl-butyrylamino)-3-methoxy-N-thiazol-2-yl-benzamid,
rac-3-Methyl-N-thiazol-2-yl-4-(3,5,5-trimethyl-hexanoylamino)-benzamid,
rac-2,3-Dihydro-benzo[1,4]dioxin-2-carbonsäure-[2-methoxy-4-(thiazol-2-yl-carbamoyl)-phenyl]-amid,
4-(2,2-Dimethyl-propionylamino)-3-methoxy-N-thiazol-2-yl-benzamid,
2-Methoxy-4-(4-methyl-benzoylamino)-N-thiazol-2-yl-benzamid,
Thiophen-2-carbonsäure-[3-methoxy-4-(thiazol-2-yl-carbamoyl)-phenyl]-amid,
4-(3-Methoxy-benzoylamino)-N-thiazol-2-yl-benzamid,
8-(2-Cyclopentyl-acetylamino)-2,3-dihydro-benzo[1,4]dioxin-5-carbonsäure-thiazol-2-yl-amid,
6-(2-Cyclopentyl-acetylamino)-biphenyl-3-carbonsäure-thiazol-2-yl-amid,
4-(2-Cyclopentyl-acetylamino)-3-(2-methoxy-ethoxy)-N-thiazol-2-yl-benzamid,
4-(3,3-Dimethyl-butyrylamino)-2-fluor-N-thiazol-2-yl-benzamid,
2-Chlor-4-(2-methyl-benzoylamino)-N-thiazol-2-yl-benzamid,
4-(2-Fluor-benzoylamino)-N-thiazol-2-yl-benzamid,
4-(2-Methoxy-benzoylamino)-N-thiazol-2-yl-benzamid,
Benzo[b]thiophen-2-carbonsäure-[2-methyl-4-(thiazol-2-yl-carbamoyl)-phenyl]-amid,
5-(3,3-Dimethyl-butyrylamino)-biphenyl-2-carbonsäure-thiazol-2-yl-amid,
N-(5-Chlor-thiazol-2-yl)-4-(3,3-dimethyl-butyrylamino)-benzamid,
N-(5-Chlor-thiazol-2-yl)-4-(3-methyl-butyrylamino)-benzamid,
N-(5-Chlor-thiazol-2-yl)-4-(2-cyclopropyl-acetylamino)-benzamid,

4-Butyrylamino-N-(5-chlor-thiazol-2-yl)-benzamid,
4-Benzoylamino-N-(5-chlor-thiazol-2-yl)-benzamid,
3-Fluor-N-thiazol-2-yl-4-(4,4,4-trifluor-3-methyl-butyrylamino)-benzamid,
4-(3,3-Dimethyl-butyrylamino)-N-thiazol-2-yl-3-trifluormethoxy-benzamid,
4-(3,3-Dimethyl-butyrylamino)-3-methoxymethyl-N-thiazol-2-yl-benzamid,
4-(3,3-Dimethyl-butyrylamino)-3-propoxy-N-thiazol-2-yl-benzamid,
3-Chlor-4-(3,3-dimethyl-butyrylamino)-5-methyl-N-thiazol-2-yl-benzamid,
4-(3,3-Dimethyl-butyrylamino)-3,5-difluor-N-thiazol-2-yl-benzamid,
4-(3,3-Dimethyl-butyrylamino)-3,5-dimethyl-N-thiazol-2-yl-benzamid,
4-(3,3-Dimethyl-butyrylamino)-N-thiazol-2-yl-3-trifluormethyl-benzamid,
3-Chlor-4-(3,3-dimethyl-butyrylamino)-N-thiazol-2-yl-5-trifluormethyl-benzamid,
N-(2,2-Dimethyl-propyl)-N'-thiazol-2-yl-terephthalamid,
3,5-Dichlor-4-(3,3-dimethyl-butyrylamino)-N-thiazol-2-yl-benzamid,
4-(3-tert-Butyl-ureido)-N-thiazol-2-yl-benzamid und
[4-(Thiazol-2-yl-carbamoyl)-phenyl]-carbaminsäure-2,2-dimethyl-propylester besteht.

**21.** Verbindung gemäß einem der Ansprüche 17 bis 20 zur Verwendung in der Medizin.

**22.** Verwendung einer Verbindung gemäß einem der Ansprüche 17 bis 20 zur Herstellung eines Medikaments zur Behandlung einer Krankheit, die aus der Gruppe ausgewählt ist, die aus Parkinson-Krankheit, Alzheimer-Krankheit, Huntington-Krankheit, Epilepsie, zerebraler Ischämie, hämorrhagischem Schlag, neonataler Ischämie und Hypoxie, subarachnoidaler Blutung, traumatischer Hirnverletzung, Hirnverletzung nach Herzstillstand, Depression und Psychosestörungen besteht.

**23.** Verwendung gemäß Anspruch 22, worin die Krankheit Parkinson-Krankheit ist.

## Revendications

**1.** Utilisation d'un composé ayant la formule I

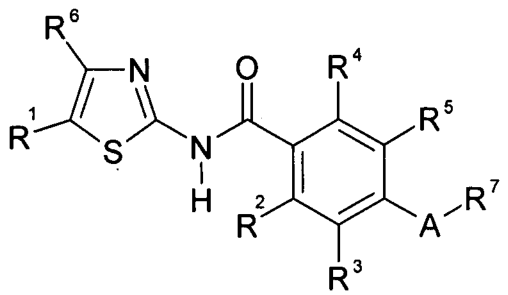

I

dans laquelle $R^1$ et $R^6$ sont indépendamment un atome d'hydrogène, un groupe alkyle en $C_{1\ à\ 6}$ ou un atome d'halogène ;
$R^2$ à $R^5$ sont indépendamment choisis dans le groupe formé par hydrogène, halogène, cyano, OH, $NH_2$, nitro, alkyle en $C_{1\ à\ 6}$, aryle, aryl-alkyle en $C_{1\ à\ 6}$, hétéroaryl-alkyle en $C_{1\ à\ 6}$, cycloalkyle en $C_{3\ à\ 8}$, cycloalkyle en $C_{3\ à\ 8}$-alkyle en $C_{1\ à\ 6}$, alcoxy en $C_{1\ à\ 6}$, aryl-alcoxy en $C_{1\ à\ 6}$, alkyle en $C_{1\ à\ 6}$-amino, et aryl-alkylamino en $C_{1\ à\ 6}$ dans lesquels chaque alkyle, alcoxy ou aryle peut être facultativement substitué par un ou plusieurs groupes halogéno, cyano, alkyle en $C_{1\ à\ 6}$, alcoxy en $C_{1\ à\ 6}$, ou alcoxy en $C_{1\ à\ 6}$-alcoxy en $C_{1\ à\ 6}$ ;
ou $R^4$ et $R^5$ forment ensemble un groupe X-$(CH_2)_n$Y, où X et Y sont indépendamment choisis dans le groupe formé par $CH_2$ et NH et O, n est 1, 2 ou 3, et $R^2$ et $R^3$ sont tels que définis ci-dessus ;
A est *$NR^8$-CO, *CO-$NR^9$, *$NR^8$-CS ou *CS-$NR^9$ où $R^8$ et $R^9$ sont indépendamment choisis dans le groupe formé par hydrogène et alkyle en $C_{1\ à\ 6}$, ou $R^8$ forme avec $R^3$ un groupe alkylène en $C_{2-3}$ ou $CH_2CH_2O$ où l'oxygène est lié au cycle phényle, et * indique l'atome qui est lié au cycle phényle ;
et $R^7$ est choisi dans le groupe formé par alkyle en $C_{1\ à\ 8}$, aryle, hétéroaryle, aryl-alkyle en $C_{1\ à\ 6}$, hétéroaryl-alkyle en $C_{1\ à\ 6}$, cycloalkyle en $C_{3\ à\ 8}$, cycloalkyle en $C_{3\ à\ 8}$-alkyle en $C_{1\ à\ 6}$, alcoxy en $C_{1\ à\ 6}$, aryl-alcoxy en $C_{1\ à\ 6}$, hétéroaryl-alcoxy en $C_{1\ à\ 6}$, alkylamino en $C_{1\ à\ 6}$, aryle-alkylamino en $C_{1\ à\ 6}$, hétéroaryle-alkylamino en $C_{1\ à\ 6}$, di-(alkyle en

$C_{1\ à\ 6}$)amino, 2,3-dihydrobenzo-[1,4]dioxin-2-yle ou adamantan-1-yl-méthyle où chaque alkyle et chaque cycloalkyle peut être substitué par un ou plusieurs groupes halogéno, cyano, hydroxy, oxo, alcoxy en $C_{1\ à\ 6}$ ou $NR^{10}R^{11}$, où $R^{10}$ et $R^{11}$ sont indépendamment un atome d'hydrogène ou un groupe alkyle en $C_{1\ à\ 6}$, ou $R^{10}$ et $R^{11}$ forment avec l'azote un cycle aliphatique à 5, 6 ou 7 chaînons qui peut contenir un autre hétéroatome choisi parmi N et O, et chaque aryle peut être facultativement substitué par un ou plusieurs groupes halogéno, cyano, hydroxy, nitro, alkyle en $C_{1\ à\ 6}$, alcoxy en $C_{1\ à\ 6}$, acyle en $C_{1\ à\ 6}$, acyloxy en $C_{1\ à\ 6}$, $NR^{10}R^{11}$ où $R^{10}$ et $R^{11}$ sont indépendamment un atome d'hydrogène ou un groupe alkyle en $C_{1\ à\ 6}$ ou $R^{10}$ et $R^{11}$ forment avec l'azote un cycle aliphatique à 5, 6 ou 7 chaînons pouvant contenir un autre hétéroatome choisi parmi N et O, ou un groupe Z-$(CH_2)_m$-W, dans lequel Z et W sont liés à deux atomes de carbone adjacents et sont indépendamment choisis dans le groupe formé par $CH_2$, NH et O, et m est 1, 2 ou 3 ;
avec la réserve que, lorsque $R^7$ est lié à l'azote, alors $R^7$ n'est pas un alcoxy en $C_{1\ à\ 6}$, un aryl-alcoxy en $C_{1\ à\ 6}$, un hétéroaryl-alcoxy en $C_{1\ à\ 6}$, un alkylamino en $C_{1\ à\ 6}$, un aryle-alkylamino en $C_{1\ à\ 6}$, un hétéroaryl-alkylamino en $C_{1\ à\ 6}$ ou un di-(alkyle en $C_{1\ à\ 6}$)-amino ;
et les sels d'addition pharmaceutiquement acceptables de ceux-ci ;
pour la fabrication d'un médicament destiné au traitement d'une maladie dans laquelle un récepteur $A_{2A}$-est impliqué.

**2.** Utilisation d'un composé selon la revendication 1 dans laquelle la maladie est choisie dans le groupe formé par la maladie de Parkinson, la maladie d'Alzheimer, la maladie de Huntington, l'épilepsie, l'ischémie cérébrale, l'accident cérébro-vasculaire hémorragique, l'ischémie et l'hypoxie néonatales, l'hémorragie méningée, les lésions traumatiques du cerveau, les lésions du cerveau à la suite d'un arrêt cardiaque, et pour le traitement de la dépression et des troubles psychotiques.

**3.** Utilisation d'un composé selon la revendication 2 dans laquelle la maladie est la maladie de Parkinson.

**4.** Utilisation d'un composé selon l'une quelconque des revendications 1 à 3 dans laquelle A est *$NR^8$-CO ou *CO-$NR^9$.

**5.** Utilisation d'un composé selon la revendication 4 dans laquelle A est *$NR^8$-CO.

**6.** Utilisation d'un composé selon l'une quelconque des revendications 1 à 5 dans laquelle $R^7$ est choisi dans le groupe formé par alkyle en $C_{1\ à\ 8}$, de préférence alkyle en $C_{3\ à\ 8}$ et de manière davantage préférée alkyle en $C_{4\ à\ 8}$ qui est ramifié en position β, cycloalkyle en $C_{3\ à\ 8}$-méthyle, cycloalkyle en $C_{3\ à\ 8}$, méthylphényle, méthoxybenzyle et thiophén-2-yl-méthyle, où chaque alkyle ou cycloalkyle peut être non substitué ou substitué par l'oxo.

**7.** Utilisation d'un composé selon l'une quelconque des revendications 1 à 6 dans laquelle $R^8$ est un atome d'hydrogène.

**8.** Utilisation d'un composé selon l'une quelconque des revendications 1 à 7 dans laquelle $R^9$ est un atome d'hydrogène,

**9.** Utilisation d'un composé selon l'une quelconque des revendications 1 à 8 dans laquelle $R^6$ est un atome d'hydrogène.

**10.** Utilisation d'un composé selon l'une quelconque des revendications 1 à 9 dans laquelle $R^1$ est un atome d'hydrogène, un méthyle ou un chloro, de préférence un atome d'hydrogène,

**11.** Utilisation d'un composé selon l'une quelconque des revendications 1 à 10 dans laquelle $R^{2\ à\ 5}$ sont indépendamment choisis dans le groupe formé par un atome d'hydrogène, un atome d'halogène, alkyle en $C_{1\ à\ 6}$, de préférence méthyle, alcoxy en $C_{1\ à\ 6}$ et alcoxy en $C_{1\ à\ 6}$-alcoxy en $C_{1\ à\ 6}$, de préférence 2-méthoxy-éthoxy.

**12.** Utilisation d'un composé selon la revendication 11 dans laquelle $R^2$ et $R^4$ sont indépendamment choisis dans le groupe formé par hydrogène, alcoxy en $C_{1\ à\ 6}$ et alcoxy en $C_{1\ à\ 6}$-alcoxy en $C_{1\ à\ 6}$, de préférence 2-méthoxy-éthoxy.

**13.** Utilisation d'un composé selon la revendication 11 ou 12 dans laquelle $R^3$ et $R^5$ sont indépendamment choisis dans le groupe formé par hydrogène, halogène, alkyle en $C_{1\ à\ 6}$, de préférence méthyle, alcoxy en $C_{1\ à\ 6}$, de préférence méthoxy, alcoxy en $C_{1\ à\ 6}$-alcoxy en $C_{1\ à\ 6}$, de préférence 2-méthoxy-éthoxy, trifluorométhyle et trifluorométhoxy.

**14.** Utilisation d'un composé selon la revendication 1 dans laquelle ledit composé est choisi dans le groupe formé par :

4-butyrylamino-*N*-thiazol-2-yl-benzamide,
*rac*-3-méthoxy-4-(3-méthyl-4-oxo-pentanoylamino)-*N*-thiazol-2-yl-benzamide,
*rac*-4-(3-méthyl-pentanoylamino)-*N*-thiazol-2-yl-benzamide,

4-hexanoylamino-3-méthyl-*N*-thiazol-2-yl-benzamide,
4-(2-cycloheptyl-acétylamino)-*N*-thiazol-2-yl-benzamide,
*rac*-3-méthoxy-4-(3-méthyl-pentanoylamino)-*N*-thiazol-2-yl-benzamide,
4-(2-cycloheptyl-acétylamino)- 3-méthoxy-*N*-thiazol-2-yl-benzamide,
*rac*-4-[2-(2-oxo-cyclopentyl)-acétylamino]-*N*-thiazol-2-yl-benzamide,
4-hexanoylamino-3-méthoxy-*N*-thiazol-2-yl-benzamide.
3-méthyl-4-(4-phényl-butyrylamino)-*N*-thiazol-2-yl-benzamide,
4-(2-cyclohexyl-acétylamino)-*N*-thiazol-2-yl-benzamide,
*rac*-4-(2-bicyclo[2.2.1]hept-2-yl-acetylamino)-*N*-thiazol-2-yl-benzamide,
4-(3,3-diméthyl-butyrylamino)-*N*-(4,5-diméthyl-thiazol-2-yl)-benzamide,
4-(2-adamantan-1-yl-acétylamino)-*N*-thiazol-2-yl-benzamide,
4-(3-benzo[1,3]dioxol-5-yl-propionylamino)-3-méthyl-*N*-thiazol-2-yl-benzamide,
4-(3-hydroxy-3-méthyl-butyrylamino)-3-méthoxy-*N*-thiazol-2-yl-benzamide,
4-(4-fluoro-benzoylamino)-3-méthoxy-*N*-thiazol-2-yl-benzamide,
4-benzoylamino-*N*-thiazol-2-yl-benzamide,
[4-(thiazol-2-ylcarbamoyl)-phényl]-amide de l'acide thiophène-3-carboxylique,
*N*-thiazol-2-yl-4-(2-*o*-tolyl-acétylamino)-benzamide,
*N*-thiazol-2-yl-4-(2-thiophen-3-yl-acétylamino)-benzamide,
4-(2-cyclopentyl-acétylamino)-3-méthyl-*N*-thiazol-2-yl-benzamide,
4-(3,3-diméthyl-butyrylamino)-*N*-thiazol-2-yl-benzamide,
4-(3,3-diméthyl-butyrylamino)-3-méthyl-*N*-thiazol-2-yl-benzamide,
4-(3,3-diméthyl-butyrylamino)-2-méthoxy-*N*-thiazol-2-yl-benzamide,
3-chloro-4-(3-méthyl-butyrylamino)-*N*-thiazol-2-yl-benzamide,
3-bromo-4-(3,3-diméthyl-butyrylamino)-*N*-thiazol-2-yl-benzamide,
4-(3-méthyl-butyrylamino)-*N*-thiazol-2-yl-benzamide,
3-bromo-4-(3-méthyl-butyrylamino)-*N*-thiazol-2-yl-benzamide,
4-(2-cyclopentyl-acétylamino)-*N*-thiazol-2-yl-benzamide,
3-méthyl-4-(3-méthyl-butyrylamino)-*N*-thiazol-2-yl-benzamide,
3-chloro-4-(cyclopentanecarbonyl-amino)-*N*-thiazol-2-yl-benzamide,
3-chloro-4-(2-méthyl-benzoylamino)-*N*-thiazol-2-yl-benzamide,
3-bromo-4-(cyclopentanecarbonyl-amino)-*N*-thiazol-2-yl-benzamide,
4-(cyclopentanecarbonyl-amino)-*N*-thiazol-2-yl-benzamide,
4-(cyclopentanecarbonyl-amino)-3-méthyl-*N*-thiazol-2-yl-benzamide,
[2-bromo-4-(thiazol-2-ylcarbamoyl)-phényl]-amide d'acide cycloheptanecarboxylique,
4-isobutyrylamino-2-méthoxy-*N*-thiazol-2-yl-benzamide,
thiazol-2-ylamide de l'acide 8-(3,3-diméthyl-butyrylamino)-2,3-dihydrobenzo[1,4]dioxine-5-carboxylique,
3-bromo-4-butyrylamino-*N*-thiazol-2-yl-benzamide,
2-méthoxy-4-(3-méthyl-butyrylamino)-*N*-thiazol-2-yl-benzamide,
[4-(thiazol-2-ylcarbamoyl)-phényll]-amide d'acide cycloheptanecarboxylique,
*rac*-2-méthoxy-4-(2-méthyl-butyrylamino)-*N*-thiazol-2-yl-benzamide,
4-(cyclopentanecarbonyl-amino)-2-méthoxy-*N*-thiazol-2-yl-benzamide,
3-bromo-4-(2-méthyl-benzoylamino)-*N*-thiazol-2-yl-benzamide,
3-chloro-4-(3,3-diméthyl-butyrylamino)-*N*-thiazol-2-yl-benzamide,
4-(2-cyclopentyl-acétylamino)-2-propoxy-*N*-thiazol- 2-yl-benzamide,
4-(3,3-diméthyl-butyrylamino)-2-propoxy-*N*-thiazol-2-yl-benzamide,
4-(2-cyclopentyl-acétylamino)-3-fluoro-*N*-thiazol-2-yl-benzamide,
4-(3-méthyl-butyrylamino)-2-propoxy-*N*-thiazol-2-yl-benzamide,
3-fluoro-4-(3-méthyl-butyrylamino)-*N*-thiazol-2-yl-benzamide,
4-butyrylamino-3-fluoro-*N*-thiazol-2-yl-benzamide,
4-butyrylamino-2-propoxy-*N*-thiazol-2-yl-benzamide,
3-fluoro-4-(2-méthyl-benzoylamino)-*N*-thiazol-2-yl-benzamide,
[2-fluoro-4-(thiazol-2-ylcarbamoyl)-phényl]-amide d'acide cycloheptanecarboxylique,
4-(cyclopentanecarbonyl-amino)-3-fluoro-*N*-thiazol-2-yl-benzamide,
4-(3,3-diméthyl-butyrylamino)-2-(2-méthoxy-éthoxy)-*N*-thiazol-2-yl-benzamide,
3-fluoro-4-(3-méthyl-benzoylamino)-*N*-thiazol-2-yl-benzamide,
*rac*-3-fluoro-*N*-thiazol-2-yl-4-(3,5,5-triméthyl-hexanoylamino)-benzamide,
4-(2-cyclopentyl-acétylamino)-2-(2-méthoxy-éthoxy)-*N*-thiazol-2-yl-benzamide,
thiazol-2-ylamide de l'acide 4-(2-méthyl-benzoyl)-3,4-dihydro-2H-benzo[1,4]oxazine-7-carboxylique,

4-(3,3-diméthyl-butyrylamino)-3-fluoro-*N*-thiazol-2-yl-benzamide,
4-(3,3-diméthyl-butyrylamino)-2-méthyl-*N*-thiazol-2-yl-benzamide,
5-chloro-4-(3,3-diméthyl-butyrylamino)-2-méthoxy-*N*-thiazol-2-yl-benzamide,
4-(3,3-diméthyl-butyrylamino)-*N*-(5-méthyl-thiazol-2-yl)-benzamide,
5-chloro-2-méthoxy-4-(3-méthyl-butyrylamino)-*N*-thiazol-2-yl-benzamide,
4-(2-méthyl-benzoylamino)-*N*-(5-méthyl-thiazol-2-yl)-benzamide,
thiazol-2-yl-amide de l'acide 1-(3,3-diméthyl-butyryl)-1,2,3,4-tetrahydro-quinoline-6-carboxylique,
5-chloro-2-méthoxy-4-(2-méthyl-benzoylamino)-*N*-thiazol-Z-yl-benzamide,
thiazol-2-yl-amide de l'acide 1-(3-méthyl-butyryl)-1,2,3,4-tetrahydro-quinoline-6-carboxylique,
thiazol-2-yl-amide de l'acide 1-(3,3-diméthyl-butyryl)-2,3-dihydro-1*H*-indole-5-carboxylique,
4-[(3,3-diméthyl-butyryl)-méthyl-amino]- *N*-thiazol-2-yl-benzamide.
4-[(2-cyclopentyl-acétyl)-propyl-amino]-*N*-thiazol-2-yl-benzamide,
2-(2-méthoxy-éthoxy)-4-(3-méthyl-butyrylamino)-*N*-thiazol-2-yl-benzamide,
*rac*-2-propoxy-*N*-thiazol-2-yl-4-(3,5,5-triméthyl-hexanoylamino)-benzamide.
*rac*-*N*-thiazol-2-yl-4-(3,5,5-triméthyl-hexanoylamino)-benzamide,
4-(3-cyclopentyl-propionylamino)-*N*-thiazol-2-yl-benzamide,
4-(2-cyclopentyl-acétylamino)-3-méthoxy-*N*-thiazol-2-yl-benzamide,
[2-méthyl-4-(thiazol-2-ylcarbamoyl)-phényl]-amide d'acide cycloheptanecarboxylique,
3-méthoxy-4-(3-phényl-propionylamino)-*N*-thiazol-2-yl-benzamide,
[2-chloro-4-(thiazol-2-ylcarbamoyl)-phényl]-amide d'acide cycloheptanecarboxylique,
4-[2-(3-méthoxy-phényl)-acétylamino)-3-méthyl-*N*-thiazol-2-yl-benzamide,
3-bromo-4-(2-cyclopentyl-acétylamino)-*N*-thiazol-2-yl-benzamide,
4-butyrylamino-3-chloro-*N*-thiazol-2-yl-benzamide,
5-chloro-4-(2-cyclopentyl-acétylamino)-2-méthoxy-*N*-thiazol-2-yl-benzamide,
5-chloro-4-(cyclopentanecarbonyl-amino)-2-méthoxy-*N*-thiazol-2-yl-benzamide,
4-(cyclohexanecarbonyl-amino)-2-méthoxy-*N*-thiazol-2-yl-benzamide,
2-méthoxy-4-(4-méthoxy-benzoylamino)-*N*-thiazol-2-yl-benzamide,
3-méthoxy-4-phénylacétylamino-N-thiazol-2-yl-benzamide,
3-méthyl-*N*-thiazol-2-yl-4-(2-thiophen-2-yl-acétylamino)-benzamide,
3-chloro-4-(2-cyclopentyl-acétylamino)-*N*-thiazol-2-yl-benzamide,
4-(4-méthoxy-benzoylamino)-3-méthyl-*N*-thiazol-2-yl-benzamide,
4-butyrylamino-3-méthyl-*N*-thiazol-2-yl-benzamide,
4-(2-chloro-benzoylamino)-3-méthyl-*N*-thiazol-2-yl-benzamide,
4-(2,5 di-chloro-benzoylamino)-3-méthyl-*N*-thiazol-2-yl-benzamide,
4-(2-chloro-benzoylamino)-2-méthoxy-*N*-thiazol-2-yl-benzamide,
4-(2-ethyl-butyrylamino)-2-méthoxy-*N*-thiazol-2-yl-benzamide,
2-méthoxy-4-(2-méthyl-benzoylamino)-*N*-thiazol-2-yl-benzamide,
3-méthyl-4-(3-phényl-propionylamino)-*N*-thiazol-2-yl-benzamide,
4-(3,3-diméthyl-butyrylamino)-3-méthoxy-*N*-thiazol-2-yl-benzamide,
*rac*-3-méthyl-*N*-thiazol-2-yl-4-(3,5,5-triméthyl-hexanoylamino)-benzamide,
[2-méthoxy-4-(thiazol-2-yl-carbamoyl)- phényl]-amide de l'acide *rac*- 2,3-dihydrobenzo[1,4]dioxine-2-carboxylique,
4-(2,2-diméthyl-propionylamino)-3-méthoxy-*N*-thiazol-2-yl-benzamide,
2-méthoxy-4-(4-méthyl-benzoylamino)-*N*-thiazol-2-yl-benzamide,
[3-méthoxy-4-(thiazol-2-ylcarbamoyl)-phényl]-amide d'acide thiophène-2-carboxylique,
4-(3-méthoxy-benzoylamino)-*N*-thiazol-2-yl-benzamide,
thiazol-2-ylamide d'acide 8-(2-cyclopentyl-acétylamino)-2,3-dihydrobenzo[1,4]dioxine-5-carboxylique,
thiazol-2-ylamide d'acide 6-(2-cyclopentyl-acétylamino)-biphényl-3-carboxylique,
4-(2-cyclopentyl-acétylamino)-3-(2-méthoxy-éthoxy)-*N*-thiazol-2-yl-benzamide
4-(3,3-diméthyl-butyrylamino)-2-fluoro-*N*-thiazol-2-yl-benzamide,
2-chloro-4-(2-méthyl-benzoylamino)-*N*-thiazol-2-yl-benzamide,
4-(2-fluoro-benzoylamino)-*N*-thiazol-2-yl-benzamide,
4-(2-méthoxy-benzoylamino)-*N*-thiazol-2-yl-benzamide,
[2-méthyl-4-(thiazol-2-ylcarbamoyl)-phényl]-amide d'acide benzo[*b*]thiophène-2-carboxylique,
thiazol-2-ylamide d'acide 5-(3, 3-diméthyl-butyrylamino)-biphényl-2-carboxylique,
N-(5-chloro-thiazol-2-yl)-4-(3,3-diméthyl-butyrylamino)-benzamide,
N-(5-chloro-thiazol-2-yl)-4-(3-méthyl-butyrylamino)-benzamide,
N-(5-chloro-thiazol-2-yl)-4-(2-cyclopropyl-acétylamino)-benzamide.

4-butyrylamino-*N*-(5-chloro-thiazol-2-yl)-benzamide,
4-benzoylamino-*N*-(5-chloro-thiazol-2-yl)-benzamide,
3-fluoro-*N*-thiazol-2-yl-4-(4,4,4-trifluoro-3-méthyl-butyrylamino)-benzamide,
4-(3,3-diméthyl-butyrylamino)-*N*-thiazol-2-yl-3-trifluorométhoxy-benzamide,
4-(3,3-diméthyl-butyrylamino)-3-méthoxyméthyl-*N*-thiazol-2-yl-benzamide,
4-(3,3-diméthyl-butyrylamino)-3-propoxy-*N*-thiazol-2-yl-benzamide,
3-chloro-4-(3,3-diméthyl-butyrylamino)-5-méthyl-*N*-thiazol-2-yl-benzamide,
4-(3,3-diméthyl-butyrylamino)-3,5-difluoro-*N*- thiazol-2-yl-benzamide,
4-(3,3-diméthyl-butyrylamino)-3,5-diméthyl-*N*-thiazol-2-yl-benzamide,
4-(3,3-diméthyl-butyrylamino)-N'-thiazol-2-yl-3-trifluorométhyl-benzamide,
3-chloro-4-(3,3-diméthyl-butyrylamino)-*N*-thiazol-2-yl-5-trifluorométhyl-benzamide,
*N*-(2,2-diméthyl-propyl)-*N*-thiazol-2-yl-téréphtalamide,
3,5-dichloro-4-(3,3-diméthyl-butyrylamino)-*N*-thiazol-2-yl-benzamide,
4-(3-*tert*-butyl-uréido)-*N*-thiazol-2-yl-benzamide, et
[4-(thiazol-2-ylcarbamoyl)-phényl]-carbamate de 2,2-diméthylpropyle.

**15.** Composition pharmaceutique comprenant un composé de formule I selon l'une quelconque des revendications 1 à 14 avec la réserve que, si A est *$NR^8$-CO, et $R^{1 \text{ à } 6}$ et $R^8$ sont tous un atome hydrogène, alors $R^7$ n'est pas un thiophén-2-yle ;
et que, si A est *$NR^8$-CO, $R^{2 \text{ à } 6}$ et $R^8$ sont tous un atome d'hydrogène et $R^1$ est un i-propyle, alors $R^7$ n'est pas un méthyle ou un benzyle ;
et que, si A est *$NR^8$-CO, $R^2$, $R^{4 \text{ à } 6}$ et $R^8$ sont tous un atome d'hydrogène, $R^3$ est un iode et $R^1$ est un *i*-propyle, alors $R^7$ n'est pas un méthyle ;
et que, si A est *$NR^8$-CO, $R^1$, $R^{3 \text{ à } 6}$ et $R^8$ sont tous un hydrogène et $R^2$ est un hydroxy, alors $R^7$ n'est ni un méthyle ni un éthoxy.

**16.** Composition selon la revendication 15, dans laquelle ledit composé est un agoniste de récepteur $A_{2A}$ ayant une affinité de liaison ($K_i$) pour le $A_{2A}$ humain de 200 nM ou moins.

**17.** Composé ayant la formule I selon l'une quelconque des revendications 1 à 14 avec la réserve que, si A est *$NR^8$-CO, et $R^{1 \text{ à } 6}$ et $R^8$ sont tous un atome d'hydrogène, alors $R^7$ n'est pas choisi dans le groupe formé par alkyle en $C_{1 \text{ à } 4}$, pentan-3-yle, trifluorométhyle, pyrimidyle, furan-2-yle, thiophén-2-yle, phényle substitué ou non substitué ou benzyle substitué ou non substitué ;
et que, si A est *$NR^8$-CO, $R^{2 \text{ à } 6}$ et $R^8$ sont tous un atome d'hydrogène, et $R^1$ est un *i*-propyle, alors $R^7$ n'est ni un méthyle ni un benzyle ;
et que, si A est *$NR^8$-CO, $R^2$, $R^{4 \text{ à } 6}$ et $R^8$ sont tous un atome d'hydrogène, $R^3$ est un atome d'iode et $R^1$ est un *i*-propyle, alors $R^7$ n'est pas un méthyle ;
et que, si A est *$NR^8$-CO, $R^1$, $R^{3 \text{ à } 6}$ et $R^8$ sont tous un atome d'hydrogène et $R^2$ est un hydroxy, alors $R^7$ n'est ni un méthyle ni un éthoxy ;
et que, si A est * $NR^8$-CO, $R^2$, $R^{4 \text{ à } 6}$ et $R^8$ sont tous un atome d'hydrogène, $R^2$ est un nitro et $R^7$ est un méthyle, alors $R^1$ n'est ni un hydrogène ni un méthyle ;
et pour autant que si A est *CO-$NR^9$, $R^1$, $R^6$ et $R^9$ sont tous un atome d'hydrogène, et $R^7$ est un thiazol-2-yle, alors $R^{2 \text{ à } 5}$ ne sont pas tous un atome d'hydrogène ni tous un atome de fluor ;
et que, si A est *CO-$NR^9$, $R^{2 \text{ à } 5}$ et $R^9$ sont tous un atome d'hydrogène, et $R^6$ est un méthyle, alors $R^1$ peut ne pas être un atome d'hydrogène si $R^7$ est un 4-méthyl-thiazol-2-yle et $R^1$ peut ne pas être un méthyle si $R^7$ est un 4,5-diméthyl-thiazol-2-yle.

**18.** Composé selon la revendication 17, où ledit composé est sous forme de sel.

**19.** Composé selon la revendication 17 ou 18, où ledit composé est un agoniste de récepteur $A_{2A}$ ayant une affinité de liaison ($K_i$) pour le $A_{2A}$ humain de 200 nM ou moins.

**20.** Composé choisi dans le groupe formé par :

4-butyrylamino-*N*-thiazol-2-yl-benzamide,
*rac*-3-méthoxy-4-(3-méthyl-4-oxo-pentanoylamino)-*N*-thiazol-2-yl-benzamide,
*rac*-4-(3-méthyl-pentanoylamino)-*N*-thiazol-2-yl-benzamide,
4-hexanoylamino-3-méthyl-*N*-thiazol-2-yl-benzamide,

4-(2-cycloheptyl-acétylamino)-*N*-thiazol-2-yl-benzamide,
*rac*-3-méthoxy-4-(3-méthyl-pentanoylamino)-*N*-thiazol-2-yl-benzamide,
4-(2-cycloheptyl-acétylamino)- 3-méthoxy-*N*-thiazol-2-yl-benzamide,
*rac*-4-[2-(2-oxo-cyclopentyl)-acétylamino]-*N*-thiazol-2-yl-benzamide,
4-hexanoylamino-3-méthoxy-*N*-thiazol-2-yl-benzamide.
3-méthyl-4-(4-phényl-butyrylamino)-*N*-thiazol-2-yl-benzamide,
4-(2-cyclohexyl-acétylamino)-*N*-thiazol-2-yl-benzamide,
*rac*-4-(2-bicyclo[2.2.1]hept-2-yl-acetylamino)-*N*-thiazol-2-yl-benzamide,
4-(3,3-diméthyl-butyrylamino)-*N*-(4,5-diméthyl-thiazol-2-yl)-benzamide,
4-(2-adamantan-1-yl-acétylamino)-*N*-thiazol-2-yl-benzamide,
4-(3-benzo[1,3]dioxol-5-yl-propionylamino)-3-méthyl-*N*-thiazol-2-yl-benzamide,
4-(3-hydroxy-3-méthyl-butyrylamino)-3-méthoxy-*N*-thiazol-2-yl-benzamide,
4-(4-fluoro-benzoylamino)-3-méthoxy-*N*-thiazol-2-yl-benzamide,
4-benzoylamino-*N*-thiazol-2-yl-benzamide,
[4-(thiazol-2-ylcarbamoyl)-phényl]-amide de l'acide thiophène-3-carboxylique,
*N*-thiazol-2-yl-4-(2-*o*-tolyl-acétylamino)-benzamide,
*N*-thiazol-2-yl-4-(2-thiophen-3-yl-acétylamino)-benzamide,
4-(2-cyclopentyl-acétylamino)-3-méthyl-*N*-thiazol-2-yl-benzamide,
4-(3,3-diméthyl-butyrylamino)-*N*-thiazol-2-yl-benzamide,
4-(3,3-diméthyl-butyrylamino)-3-méthyl-*N*-thiazol-2-yl-benzamide,
4-(3,3-diméthyl-butyrylamino)-2-méthoxy-*N*-thiazol-2-yl-benzamide,
3-chloro-4-(3-méthyl-butyrylamino)-*N*-thiazol-2-yl-benzamide,
3-bromo-4-(3,3-diméthyl-butyrylamino)-*N*-thiazol-2-yl-benzamide,
4-(3-méthyl-butyrylamino)-*N*-thiazol-2-yl-benzamide,
3-bromo-4-(3-méthyl-butyrylamino)-*N*-thiazol-2-yl-benzamide,
4-(2-cyclopentyl-acétylamino)-*N*-thiazol-2-yl-benzamide,
3-méthyl-4-(3-méthyl-butyrylamino)-*N*-thiazol-2-yl-benzamide,
3-chloro-4-(cyclopentanecarbonyl-amino)-*N*-thiazol-2-yl-benzamide,
3-chloro-4-(2-méthyl-benzoylamino)-*N*-thiazol-2-yl-benzamide,
3-bromo-4-(cyclopentanecarbonyl-amino)-*N*-thiazol-2-yl-benzamide,
4-(cyclopentanecarbonyl-amino)-*N*-thiazol-2-yl-benzamide,
4-(cyclopentanecarbonyl-amino)-3-méthyl-*N*-thiazol-2-yl-benzamide,
[2-bromo-4-(thiazol-2-ylcarbamoyl)-phényl]-amide d'acide cycloheptanecarboxylique,
4-isobutyrylamino-2-méthoxy-*N*-thiazol-2-yl-benzamide,
thiazol-2-ylamide de l'acide 8-(3,3-diméthyl-butyrylamino)-2,3-dihydrobenzo[1,4]dioxine-5-carboxylique,
3-bromo-4-butyrylamino-*N*-thiazol-2-yl-benzamide,
2-méthoxy-4-(3-méthyl-butyrylamino)-*N*-thiazol-2-yl-benzamide,
[4-(thiazol-2-ylcarbamoyl)-phényll]-amide d'acide cycloheptanecarboxylique,
*rac*-2-méthoxy-4-(2-méthyl-butyrylamino)-*N*-thiazol-2-yl-benzamide,
4-(cyclopentanecarbonyl-amino)-2-méthoxy-*N*-thiazol-2-yl-benzamide,
3-bromo-4-(2-méthyl-benzoylamino)-*N*-thiazol-2-yl-benzamide,
3-chloro-4-(3,3-diméthyl-butyrylamino)-*N*-thiazol-2-yl-benzamide,
4-(2-cyclopentyl-acétylamino)-2-propoxy-*N*-thiazol- 2-yl-benzamide,
4-(3,3-diméthyl-butyrylamino)-2-propoxy-*N*-thiazol-2-yl-benzamide,
4-(2-cyclopentyl-acétylamino)-3-fluoro-*N*-thiazol-2-yl-benzamide,
4-(3-méthyl-butyrylamino)-2-propoxy-*N*-thiazol-2-yl-benzamide,
3-fluoro-4-(3-méthyl-butyrylamino)-*N*-thiazol-2-yl-benzamide,
4-butyrylamino-3-fluoro-*N*-thiazol-2-yl-benzamide,
4-butyrylamino-2-propoxy-*N*-thiazol-2-yl-benzamide,
3-fluoro-4-(2-méthyl-benzoylamino)-*N*-thiazol-2-yl-benzamide,
[2-fluoro-4-(thiazol-2-ylcarbamoyl)-phényl]-amide d'acide cycloheptanecarboxylique,
4-(cyclopentanecarbonyl-amino)-3-fluoro-*N*-thiazol-2-yl-benzamide,
4-(3,3-diméthyl-butyrylamino)-2-(2-méthoxy-éthoxy)-*N*-thiazol-2-yl-benzamide,
3-fluoro-4-(3-méthyl-benzoylamino)-*N*-thiazol-2-yl-benzamide,
*rac*-3-fluoro-*N*-thiazol-2-yl-4-(3,5,5-triméthyl-hexanoylamino)-benzamide,
4-(2-cyclopentyl-acétylamino)-2-(2-méthoxy-éthoxy)-*N*-thiazol-2-yl-benzamide,
thiazol-2-ylamide de l'acide 4-(2-méthyl-benzoyl)-3,4-dihydro-2H-benzo[1,4]oxazine-7-carboxylique,
4-(3,3-diméthyl-butyrylamino)-3-fluoro-*N*-thiazol-2-yl-benzamide,

4-(3,3-diméthyl-butyrylamino)-2-méthyl-*N*-thiazol-2-yl-benzamide,
5-chloro-4-(3,3-diméthyl-butyrylamino)-2-méthoxy-*N*-thiazol-2-yl-benzamide,
4-(3,3-diméthyl-butyrylamino)-*N*-(5-méthyl-thiazol-2-yl)-benzamide,
5-chloro-2-méthoxy-4-(3-méthyl-butyrylamino)-*N*-thiazol-2-yl-benzamide,
4-(2-méthyl-benzoylamino)-*N*-(5-méthyl-thiazol-2-yl)-benzamide,
thiazol-2-yl-amide de l'acide 1-(3,3-diméthyl-butyryl)-1,2,3,4-tetrahydro-quinoline-6-carboxylique,
5-chloro-2-méthoxy-4-(2-méthyl-benzoylamino)-*N*-thiazol-Z-yl-benzamide,
thiazol-2-yl-amide de l'acide 1-(3-méthyl-butyryl)-1,2,3,4-tetrahydro-quinoline-6-carboxylique,
thiazol-2-yl-amide de l'acide 1-(3,3-diméthyl-butyryl)-2,3-dihydro-1*H*-indole-5-carboxylique,
4-[(3,3-diméthyl-butyryl)-méthyl-amino]- *N*-thiazol-2-yl-benzamide.
4-[(2-cyclopentyl-acétyl)-propyl-amino]-*N*-thiazol-2-yl-benzamide,
2-(2-méthoxy-éthoxy)-4-(3-méthyl-butyrylamino)-*N*-thiazol-2-yl-benzamide,
*rac*-2-propoxy-*N*-thiazol-2-yl-4-(3,5,5-triméthyl-hexanoylamino)-benzamide.
*rac*- N-thiazol-2-yl-4-(3,5,5-triméthyl-hexanoylamino)-benzamide,
4-(3-cyclopentyl-propionylamino)-*N*-thiazol-2-yl-benzamide,
4-(2-cyclopentyl-acétylamino)-3-méthoxy-*N*-thiazol-2-yl-benzamide,
[2-méthyl-4-(thiazol-2-ylcarbamoyl)-phényl]-amide d'acide cycloheptanecarboxylique,
3-méthoxy-4-(3-phényl-propionylamino)-*N*-thiazol-2-yl-benzamide,
[2-chloro-4-(thiazol-2-ylcarbamoyl)-phényl]-amide d'acide cycloheptanecarboxylique,
4-[2-(3-méthoxy-phényl)-acétylamino)-3-méthyl-*N*-thiazol-2-yl-benzamide,
3-bromo-4-(2-cyclopentyl-acétylamino)-*N*-thiazol-2-yl-benzamide,
4-butyrylamino-3-chloro-*N*-thiazol-2-yl-benzamide,
5-chloro-4-(2-cyclopentyl-acétylamino)-2-méthoxy-*N*-thiazol-2-yl-benzamide,
5-chloro-4-(cyclopentanecarbonyl-amino)-2-méthoxy-*N*-thiazol-2-yl-benzamide,
4-(cyclohexanecarbonyl-amino)-2-méthoxy-*N*-thiazol-2-yl-benzamide,
2-méthoxy-4-(4-méthoxy-benzoylamino)-*N*-thiazol-2-yl-benzamide,
3-méthoxy-4-phénylacétylamino-*N*-thiazol-2-yl-benzamide,
3-méthyl-*N*-thiazol-2-yl-4-(2-thiophen-2-yl-acétylamino)-benzamide,
3-chloro-4-(2-cyclopentyl-acétylamino)-*N*-thiazol-2-yl-benzamide,
4-(4-méthoxy-benzoylamino)-3-méthyl-*N*-thiazol-2-yl-benzamide,
4-butyrylamino-3-méthyl-*N*-thiazol-2-yl-benzamide,
4-(2-chloro-benzoylamino)-3-méthyl-*N*-thiazol-2-yl-benzamide,
4-(2,5 di-chloro-benzoylamino)-3-méthyl-*N*-thiazol-2-yl-benzamide,
4-(2-chloro-benzoylamino)-2-méthoxy-*N*-thiazol-2-yl-benzamide,
4-(2-ethyl-butyrylamino)-2-méthoxy-*N*-thiazol-2-yl-benzamide,
2-méthoxy-4-(2-méthyl-benzoylamino)-*N*-thiazol-2-yl-benzamide,
3-méthyl-4-(3-phényl-propionylamino)-*N*-thiazol-2-yl-benzamide,
4-(3,3-diméthyl-butyrylamino)-3-méthoxy-*N*-thiazol-2-yl-benzamide,
*rac*-3-méthyl-*N*-thiazol-2-yl-4-(3,5,5-triméthyl-hexanoylamino)-benzamide,
[2-méthoxy-4-(thiazol-2-yl-carbamoyl)- phényl]-amide de l'acide *rac*- 2,3-dihydrobenzo[1,4]dioxine-2-carboxylique,
4-(2,2-diméthyl-propionylamino)-3-méthoxy-*N*-thiazol-2-yl-benzamide,
2-méthoxy-4-(4-méthyl-benzoylamino)-*N*-thiazol-2-yl-benzamide,
[3-méthoxy-4-(thiazol-2-ylcarbamoyl)-phényl]-amide d'acide thiophène-2-carboxylique,
4-(3-méthoxy-benzoylamino)-*N*-thiazol-2-yl-benzamide, thiazol-2-ylamide d'acide 8-(2-cyclopentyl-acétylamino)-2,3-dihydrobenzo[1,4]dioxine-5-carboxylique,
thiazol-2-ylamide d'acide 6-(2-cyclopentyl-acétylamino)-biphényl-3-carboxylique,
4-(2-cyclopentyl-acétylamino)-3-(2-méthoxy-éthoxy)-*N*-thiazol-2-yl-benzamide
4-(3,3-diméthyl-butyrylamino)-2-fluoro-*N*-thiazol-2-yl-benzamide,
2-chloro-4-(2-méthyl-benzoylamino)-*N*-thiazol-2-yl-benzamide,
4-(2-fluoro-benzoylamino)-*N*-thiazol-2-yl-benzamide,
4-(2-méthoxy-benzoylamino)-*N*-thiazol-2-yl-benzamide,
[2-méthyl-4-(thiazol-2-ylcarbamoyl)-phényl]-amide d'acide benzo[*b*]thiophène-2-carboxylique,
thiazol-2-ylamide d'acide 5-(3, 3-diméthyl-butyrylamino)-biphényl-2-carboxylique,
N-(5-chloro-thiazol-2-yl)-4-(3,3-diméthyl-butyrylamino)-benzamide,
N-(5-chloro-thiazol-2-yl)-4-(3-méthyl-butyrylamino)-benzamide,
N-(5-chloro-thiazol-2-yl)-4-(2-cyclopropyl-acétylamino)-benzamide.
4-butyrylamino-*N*-(5-chloro-thiazol-2-yl)-benzamide,

4-benzoylamino-*N*-(5-chloro-thiazol-2-yl)-benzamide,
3-fluoro-*N*-thiazol-2-yl-4-(4,4,4-trifluoro-3-méthyl-butyrylamino)-benzamide,
4-(3,3-diméthyl-butyrylamino)-*N*-thiazol-2-yl-3-trifluorométhoxy-benzamide,
4-(3,3-diméthyl-butyrylamino)-3-méthoxyméthyl-*N*-thiazol-2-yl-benzamide,
4-(3,3-diméthyl-butyrylamino)-3-propoxy-*N*-thiazol-2-yl-benzamide,
3-chloro-4-(3,3-diméthyl-butyrylamino)-5-méthyl-*N*-thiazol-2-yl-benzamide,
4-(3,3-diméthyl-butyrylamino)-3,5-difluoro-*N*- thiazol-2-yl-benzamide,
4-(3,3-diméthyl-butyrylamino)-3,5-diméthyl-*N*-thiazol-2-yl-benzamide,
4-(3,3-diméthyl-butyrylamino)-N-thiazol-2-yl-3-trifluorométhyl-benzamide,
3-chloro-4-(3,3-diméthyl-butyrylamino)-*N*-thiazol-2-yl-5-trifluorométhyl-benzamide,
*N*-(2,2-diméthyl-propyl)-*N'*-thiazol-2-yl-téréphtalamide,
3,5-dichloro-4-(3,3-diméthyl-butyrylamino)-*N*-thiazol-2-yl-benzamide,
4-(3-*tert*-butyl-uréido)-*N*-thiazol-2-yl-benzamide, et
[4-(thiazol-2-ylcarbamoyl)-phényl]-carbamate de 2,2-diméthylpropyle.

**21.** Composé selon l'une quelconque des revendications 17 - 20 pour une utilisation en médecine.

**22.** Utilisation d'un composé selon l'une quelconque des revendications 17 - 20 pour la fabrication d'un médicament destiné au traitement d'une maladie choisie dans le groupe formé par la maladie de Parkinson, la maladie d'Alzheimer, la maladie de Huntington, l'épilepsie, l'ischémie cérébrale, l'accident cérébro-vasculaire hémorragique, l'ischémie et l'hypoxie néonatales, l'hémorragie méningée, les lésions traumatiques du cerveau, les lésions du cerveau à la suite d'un arrêt cardiaque, et pour le traitement de la dépression et des troubles psychotiques.

**23.** Utilisation selon la revendication 22, dans laquelle la maladie est la maladie de Parkinson.